# EUROPEAN PATENT SPECIFICATION

(11) **EP 1 859 044 B1**
(45) Date of publication and mention of the grant of the patent: **02.07.2014**
(21) Application number: 06716692.6
(22) Date of filing: 20.03.2006
(51) Int. Cl.: C12N 15/82, A01H 5/00, A01H 17/00

(54) **RESISTANCE AGAINST PARASITIC WEEDS**
RESISTENZ GEGENÜBER PARASITÄREN UNKRÄUTERN
RÉSISTENCE CONTRE LES MAUVAISES HERBES

(30) Priority: 18.03.2005 EP 05102164
(43) Date of publication of application: 28.11.2007
(73) Proprietor: Wageningen Universiteit, 6708 PB Wageningen (NL)
(72) Inventor: RANI, Kumkum, Meerut 250 001 (IN); BEALE, Michael H., 949 01 Nitra (SK); MATUSOVA, Radoslava, 949 01 Nitra (SK); BOUWMEESTER, Hendrik Jan, Winterbourne Bristol BS36 1DH (GB); SUN, Zhongkui, 300192 Tianjin (IN)
(74) Representative: Nederlandsch Octrooibureau
(86) International application number: PCT/NL2006/050059
(87) International publication number: WO 2006/098626

(56) References cited:
- EP-A- 1 156 117
- WO-A-01/88169
- WO-A-99/55888
- WO-A-02/094008
- WO-A-03/008534
- WO-A-2004/007691
- WO-A1-01/73089
- WO-A2-03/020015
- DENEV I ET AL.: "Biosynthesis of germination stimulants for Orobanche ramosa (L.) in tobacco" COST ACTION 849, PARASITIC PLANT MANAGEMENT IN SUSTAINABLE AGRICULTURE, WORKSHOP ON "STATE OF THE ART IN OROBANCHE CONTROL", [Online] 18 October 2001 (2001-10-18), - 20 October 2001 (2001-10-20) page 6, XP002331381 BARI, ITALY Retrieved from the Internet: URL:http://cost849.ba.cnr.it/BARI%20ABSTRA CTS.pdf>
- HABLE W E ET AL: "Viviparous-5 encodes phytoene desaturase, an enzyme essential for abscisic acid (ABA) accumulation and seed development in maize" MOLECULAR AND GENERAL GENETICS, vol. 257, no. 2, January 1998 (1998-01), pages 167-176, XP002331380 ISSN: 0026-8925 cited in the application
- BOUWMEESTER HARRO J ET AL: "Secondary metabolite signalling in host-parasitic plant interactions." CURRENT OPINION IN PLANT BIOLOGY, vol. 6, no. 4, August 2003 (2003-08), pages 358-364, XP002331379 ISSN: 1369-5266
- BOUWMEESTER, H., ET AL: "Biosynthesis of broomrape germination stimulants" COST ACTION 849, PARASITIC PLANT MANAGEMENT IN SUSTAINABLE AGRICULTURE, JOINT WORKING GROUP AND MC MEETING WORKSHOP, WORKSHOP " BROOMRAPE MANAGEMENT", [Online] 15 July 2004 (2004-07-15), - 17 July 2004 (2004-07-17) pages 1-2, XP002331382 NITRA, SLOVAKIA Retrieved from the Internet: URL:http://cost849.ba.cnr.it/proceedings%2 0Nitra.pdf>
- BOUWMEESTER, H., ET AL.: "Secondary metabolites in the signalling between parasite weeds and host plants" COST ACTION 849, PARASITIC PLANT MANAGEMENT IN SUSTAINABLE AGRICULTURE, JOINT MEETING OF WG 1+3, [Online] 14 March 2002 (2002-03-14), - 18 March 2002 (2002-03-18) page 6, XP002331383 SOFIA, BULGARIA Retrieved from the Internet: URL:http://cost849.ba.cnr.it/Proceedings-S ofia.PDF>
- BOUWMEESTER, H., ET AL: "Manipulation of germination stimulants for control" COST ACTION 849, PARASITIC PLANT MANAGEMENT IN SUSTAINABLE AGRICULTURE, USE OF NATURAL COMPOUNDS FOR PARASITIC PLANT MANAGEMENT., [Online] 29 October 2004 (2004-10-29), - 31 October 2004 (2004-10-31) page 3, XP002331384 NAPLES, ITALY Retrieved from the Internet: URL:http://cost849.ba.cnr.it/Proceedings%2 0Naples%202004.pdf>
- SUN Z. ET AL.: "Biosynthesis of Orobanche germination stimulants in Arabidopsis" COST ACTION 849, PARASITIC PLANT MANAGEMENT IN SUSTAINABLE AGRICULTURE, MEETING ON MECHANISMS OF SUSCEPTIBILITY AND RESISTANCE IN PARASITIC ANGIOSPERM-HOST SYMBIOSES: A COMPARATIVE APPROACH, [Online] 13 October 2004 (2004-10-13), - 15 October 2004 (2004-10-15) page 16, XP002331385 WAGENINGEN, THE NETHERLANDS Retrieved from the Internet: URL:http://cost849.ba.cnr.it/Proceedings%2 0Wageningen.pdf>
- DATABASE BIOSIS [Online] BIOSCIENCES INFORMATION SERVICE, PHILADELPHIA, PA, US; October 2003 (2003-10), GWORGWOR NUHU ADAMU ET AL: "Arbuscular mycorrhizal fungi-parasite-host interaction for the control of Striga hermonthica (Del.) Benth. in sorghum (Sorghum bicolor (L.) Moench)." XP002331386 Database accession no. PREV200400055816 & MYCORRHIZA, vol. 13, no. 5, October 2003 (2003-10), pages 277-281, ISSN: 0940-6360
- MATUSOVA RADOSLAVA ET AL: "The strigolactone germination stimulants of the plant-parasitic Striga and Orobanche spp. are derived from the carotenoid pathway" PLANT PHYSIOLOGY (ROCKVILLE), vol. 139, no. 2, October 2005 (2005-10), pages 920-934, XP002396513 ISSN: 0032-0889
- LÓPEZ-RÁEZ JUAN ANTONIO ET AL: "Fine-tuning regulation of strigolactone biosynthesis under phosphate starvation.", PLANT SIGNALING & BEHAVIOR NOV 2008, vol. 3, no. 11, November 2008 (2008-11), pages 963-965, ISSN: 1559-2316
- TAN BAO CAI ET AL: "Genetic control of abscisic acid biosynthesis in maize", PROCEEDINGS OF THE NATIONAL ACADEMY OF SCIENCES - PNAS, NATIONAL ACADEMY OF SCIENCES, US, vol. 94, no. 22, 28 October 1997 (1997-10-28), pages 12235-12240, XP002146511, ISSN: 0027-8424, DOI: 10.1073/PNAS.94.22.12235
- SUN, K.: "Biosynthesis of germination stimulants of parasitic weeds Striga and Orobanche", PHD THESIS, 26 February 2008 (2008-02-26), Wageningen
- VOGEL JONATHAN T ET AL: "SlCCD7 controls strigolactone biosynthesis, shoot branching and mycorrhiza-induced apocarotenoid formation in tomato", PLANT JOURNAL, vol. 61, no. 2, January 2010 (2010-01), pages 300-311, ISSN: 0960-7412
- SUN ZHONGKUI ET AL: "Cloning and characterisation of a maize carotenoid cleavage dioxygenase (ZmCCD1) and its involvement in the biosynthesis of apocarotenoids with various roles in mutualistic and parasitic interactions", PLANTA (BERLIN), vol. 228, no. 5, October 2008 (2008-10), pages 789-801, ISSN: 0032-0935
- BOOKER J ET AL: "MAX3/CCD7 Is a Carotenoid Cleavage Dioxygenase Required for the Synthesis of a Novel Plant Signaling Molecule", CURRENT BIOLOGY, CURRENT SCIENCE, GB, vol. 14, no. 14, 27 July 2004 (2004-07-27) , pages 1232-1238, XP025916180, ISSN: 0960-9822, DOI: 10.1016/J.CUB.2004.06.061 [retrieved on 2004-07-26]
- STEVEN H SCHWARTZ ET AL: "The Biochemical Characterization of Two Carotenoid Cleavage Enzymes from Arabidopsis Indicates That a Carotenoid-derived Compound Inhibits Lateral Branching", JOURNAL OF BIOLOGICAL CHEMISTRY, AMERICAN SOCIETY FOR BIOCHEMISTRY AND MOLECULAR BIOLOGY, vol. 279, no. 45, 5 November 2004 (2004-11-05), pages 46940-46945, XP007906444, ISSN: 0021-9258, DOI: 10.1074/JBC.M409004200
- VICTORIA GOMEZ-ROLDAN ET AL: "Strigolactone inhibition of shoot branching", NATURE: INTERNATIONAL WEEKLY JOURNAL OF SCIENCE (AND SUPPLEMENTARY INFORMATION), NATURE PUBLISHING GROUP, UNITED KINGDOM, vol. 455, 11 September 2008 (2008-09-11), pages 189-194, XP007906447, ISSN: 0028-0836, DOI: 10.1038/NATURE07271 [retrieved on 2008-08-10]
- MIKIHISA UMEHARA ET AL: "Strigolaction inhibition of shoot branching", NATURE: INTERNATIONAL WEEKLY JOURNAL OF SCIENCE (AND SUPPLEMENTARY INFORMATION), NATURE PUBLISHING GROUP, UNITED KINGDOM, vol. 455, 11 September 2008 (2008-09-11), pages 195-200, XP002629960, ISSN: 0028-0836, DOI: 10.1038/NATURE07272 [retrieved on 2008-08-10]
- ZOU JUNHUANG ET AL: "Characterizations and fine mapping of a mutant gene for high tillering and dwarf in rice (Oryza sativa L.)", PLANTA (BERLIN), vol. 222, no. 4, November 2005 (2005-11), pages 604-612, ISSN: 0032-0935
- ZOU JUNHUANG ET AL: "Characterizations and fine mapping of a mutant gene for high tillering and dwarf in rice (Oryza sativa L.)", PLANTA (BERLIN), vol. 222, no. 4, November 2005 (2005-11), pages 604-612, ISSN: 0032-0935
- ISHIKAWA SHINJI ET AL: "Suppression of tiller bud activity in tillering dwarf mutants of rice", PLANT AND CELL PHYSIOLOGY, vol. 46, no. 1, January 2005 (2005-01), pages 79-86, ISSN: 0032-0781
- SOREFAN KARIM ET AL: "MAX4 and RMS1 are orthologous dioxygenase-like genes that regulate shoot branching in Arabidopsis and pea.", GENES AND DEVELOPMENT, vol. 17, no. 12, 15 June 2003 (2003-06-15) , pages 1469-1474, ISSN: 0890-9369
- SNOWDEN KIMBERLEY C ET AL: "The Decreased apical dominance 1/petunia hybrida carotenoid cleavage dioxygenase8 gene affects branch production and plays a role in leaf senescence, root growth, and flower development", PLANT CELL, vol. 17, no. 3, March 2005 (2005-03), pages 746-759, ISSN: 1040-4651

## Description

### FIELD OF THE INVENTION

The present invention relates to the field of plant biotechnology and plant breeding. In particular methods for making plants having enhanced resistance to parasitic weeds are provided. Further provided is a method to use specific herbicides and/or mycorrhiza to control parasitic plants through their effect on the host plant. Also provided are methods for making trap plants and catch plants for parasitic weed control, as well as chimeric genes, overexpression vectors and gene silencing vectors for use in any of these methods. Also, recombinant plants and plant cells, tissues and organs are provided.

### BACKGROUND OF THE INVENTION

Parasitic weeds cause enormous yield losses in agriculture. Broomrapes (Orobanche spp., Orobanchaceae) and witchweeds (Striga spp., Scrophulariaceae) are serious pests in many countries. Infected crops can be heavily damaged even before Orobanche or Striga emerge above the soil. Orobanche spp. are holoparasites that lack chlorophyll and for their development they obtain water and nutrients through the roots of their host. Orobanche cumana Wallr. parasitises sunflower in Spain as well as in Eastern Europe around the Black Sea (Akhtouch et al., 2002, Plant Breeding, 121, 266-268) *Orobanche cernua* is closely related to *O*. cumana but parasites a wider range of hosts, mainly Solanaceous species. Orobanche ramosa is widely spread in Southern Europe and the Mediterranean region and was introduced to regions of South Africa, USA and Central America (Musselman, 1994, Biology and management of Orobanche. Procceedings of the Third International Workshop on Orobanche and related Striga research, 27-35). Together with Orobanche *aegyptiaca* from which it is difficult to distinguish, *O*. ramosa parasitises a wide range of hosts such as tomato, potato, eggplant, tobacco, cucurbits, crucifers, sunflower and some other vegetables such as carrot, celery, parsnip and lettuce (Press et al., 2001, The World's worst weeds, 71-90) Orobanche crenata is a widespread parasite of legumes all around the Mediterranean (Press et al., 2001, supra). Striga spp. belong to the hemiparasites with lower photosynthetic activity and basically behave as holoparasites (Parker and Riches, 1993, 111-163). Striga spp. are a problem particularly in the African continent, but also extend into Asia (Press et al., 2001, supra). Hosts of the most important agriculturally important Striga spp, *S. hermonthica, Striga asiatica, Striga aspera, Striga forbesii* include grain cereals such as maize, sorghum, millet and upland rice (Press et al., 2001, supra). *Striga gesneroides* is a parasite of cowpea, and causes extensive damage in dry areas of Sub-Saharan, particularly West- Africa (Press et al., 2001, supra).

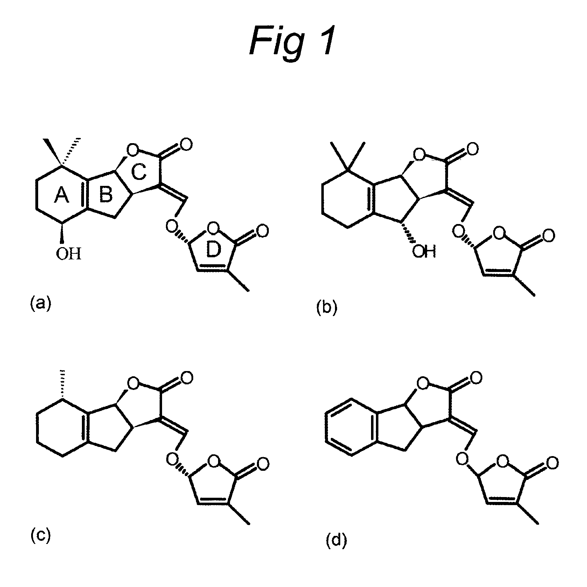

The first critical step in the life cycle of these parasites - germination of their seeds, is regulated by specific chemical signals exuded by the roots of host plants. For *Striga* spp. several germination stimulants were identified from host and non-host plants. Most of them are known as strigolactones (Figure 1). Germination stimulants in maize and sorghum were identified as strigol (Siame et al., 1993, J Agr Food Chem, 41, 1486-1491) and sorgolactone (Hauck et al., 1992, J Plant Physiol, 139, 474-478). Alectrol was identified in the root exudate of cowpea (Muller et al., 1992, J Plant Growth Regul, 11, 77-84). Alectrol and orobanchol, germination stimulants for *O*. *minor* were isolated and identified from the root exudate of red clover (Yokota et al., 1998, Phytochemistry, 49, 1967-1973). The same group recently reported on the isolation of four novel strigolactones from the root exudate of tomato, and the presence of a novel strigol-isomer in the root exudate of sorghum (Yoneyama et al., 2004, 8th International Parasitic Weed Symposium, 9).

Although *Striga* and *Orobanche* spp parasitise different hosts in different parts of the world, their lifecycles are principally similar, and hence we will discuss the two genera together. The important steps in the lifecycle are germination, radicle growth to the host root, haustorium formation and attachment to the host root, the establishment of a xylem connection and compatible interaction, and seed production. In many of the steps there is extensive signalling between the host plant and the parasite. This begins with the secretion of secondary metabolites from the roots of hosts [and some false (non) hosts] that induce the germination of the parasite seeds. For the seeds of *Orobanche* and *Striga* spp. to become responsive to these germination stimulants they require a moist environment for a certain period of time at a suitable temperature. This period is described as preconditioning or conditioning and is comparable to what is called (warm) stratification in seeds of non-parasitic plants or release of dormancy (Matusova et al., 2004, Seed Sci Res, 14, 335-344). During preconditioning of parasitic plant seeds the dormancy of the seeds is broken and they become progressively more responsive to germination stimulants. After reaching the maximum sensitivity, the seeds start entering into secondary dormancy and their sensitivity to the germination stimulants gradually decreases. Hence, the length of the preconditioning period has a great effect on the sensitivity of the parasitic weed seeds to germination stimulants, similar to the sensitivity of non-parasitic plant seeds to other external stimuli such as light and nitrate (Matusova et al., 2004, supra).

The adaptation of the parasitic weeds to these germination stimulants is of evolutionary significance as the tiny seeds contain minimal reserves and thus cannot survive for more than a few days after germination unless a host root is invaded (Butler, 1995, Allelopathy: organism, processes and application, 158-168). After germination, further host-derived secondary metabolites are involved in the plant-parasite interaction. For example, sunflower-derived allelochemical coumarins induce necrosis in the germinated seeds of *O*. cumana (Serghini et al., 2001, J Exp Bot, 52, 2227-2234). Also, the radicle of the parasite must grow towards the host root and this process may be directed by host-root-derived compounds, perhaps by the concentration gradient of germination stimulant (Dube and Olivier, 2001, Can J Bot, 79, 1225-1240). On encountering a host root, attachment to the root and the host xylem vessels is realised by formation of a haustorium, which is also initiated and guided by host-derived secondary metabolites (Hirsch et al., 2003, Ecology, 84, 858-868;Keyes et al., 2001, Plant Physiol., 127, 1508-1512;Yoder, 2001, Curr Opin Plant Biol, 4, 359-365). Finally, after haustorium formation a connection to the host root xylem is established, probably with involvement of hydrolytic enzymes produced by the penetrating parasite (Labrousse et al., 2001, Ann Bot-London, 88, 859-868). Several authors have shown that the success of this process, i.e, the establishment of a xylem connection, is also dependent on the host and can be negated by host produced toxins (Goldwasser et al., 1999, Physiol Mol Plant P, 54, 3-4; Labrousse et al., 2001, supra; Serghini et al., 2001, supra).

### Germination stimulants

As mentioned above, the strigolactones have been identified in the root exudates of a variety of plant species (Figure 1). Strigol was first identified in the false host cotton (Cook et al., 1972, J Am Chem Soc, 94, 6198-6199) and later also in the true Striga hosts maize, sorghum and millet (Butler, 1995, supra; Hauck et al., 1992, supra; Siame et al., 1993, supra). The structurally related alectrol was identified in cowpea, a host of S. *gesneroides* (Muller et al., 1992, supra). The first Orobanche germination stimulants alectrol, orobanchol and a third unidentified germination stimulant have been isolated from root exudate of red clover (Yokota et al., 1998, supra). The same group recently reported on the isolation of four novel strigolactones from the root exudate of tomato, and the presence of a novel strigol-isomer in the root exudate of sorghum (Yoneyama et al., 2004, supra).

The chemical structures of the four strigolactones identified so far are small variations on one molecular backbone (Figure 1) and it is tempting to speculate that the small variations in structure of these compounds play a role in the host specificity of the parasitic weeds. Recognition of germination stimulants may ensure that seeds of parasites only germinate in the presence of a true host. However, a number of examples show that the specificity may not be very high. Wigchert et al. (Wigchert and Zwanenburg, 1999, J Agr Food Chem, 47, 1320-1325) induced germination of the seeds of *O*. crenata - that normally parasitises legumes - with sorgolactone, one of the germination stimulants identified in sorghum. Alectrol was identified in cowpea as a germination stimulant for S. gesneroides (Muller et al., 1992, supra), but was also identified in red clover as a germination stimulant for *O*. minor (Yokota et al., 1998, supra). Finally, the synthetic strigolactone analogue GR 24 (Figure 1) induces germination of many parasitic weed seeds regardless of parasite or host plant species. On the other hand, there is some degree of host specificity. For example, not all host plant species induce germination of all parasitic weed seeds and not all synthetic germination stimulants induce germination of all parasites to the same extent (Mwakaboko, 2003). Furthermore, different races amongst parasitic weed populations appear to have evolved to recognise germination stimulants from crops parasitised by the parent weed (Gurney et al., 2002, Weed Res, 42, 299-306).

Although there is not a single determinant in a successful interaction between parasite and host plant, the production of a germination stimulant is a prerequisite. Other factors are the presence of 'conditioned' parasite seed (determined by suitable environmental conditions such as temperature and moisture) and compatibility between parasite and host. The first step in the interaction between host and parasite - induction of germination - is an important target for improved control measures. In sorghum a selection program for low-germination stimulant formation has resulted in low-stimulant sorghum varieties with improved resistance (or decreased sensitivity) (Mohamed et al., 2001, 7th International Parasitic Weed Symposium, 96-100). Work on synthetic germination stimulants in the group of B Zwanenburg has led to the development of molecules that have potential as parasitic weed control agents through the induction of suicidal germination (Mwakaboko, 2003, supra; Wigchert and Zwanenburg, 1999, supra). Another control strategy, based on the production of germination stimulants by non-hosts, is the use of trap and catch crops in monoculture or in intercropping. Usually, these non-host crops produce germination stimulants, sometimes in high amounts, and hence induce massive germination of the parasite, but they are resistant in a later stage of the parasite's lifecycle (trap crops) or harvested before the seeds of the parasite are shed (catch crops) (Chittapur et al., 2001, Allelopathy J, 8, 147-160).

### Biosynthetic origin of germination stimulants

Surprisingly, little is known about the biochemical pathway and regulation of the biosynthesis of germination stimulants in the roots of the host species. This is without doubt due to the extremely low concentrations of highly active compounds that are produced by and secreted from the host roots. The strigolactones have been described as sesquiterpene lactones by many authors. Thus, control methods have so far focused on modulating the sequiterpene pathway, for example using chemical inhibitors. The present inventors surprisingly found that germination stimulants are synthesized via the carotenoid pathway, which allows for the first time to devise methods for reducing or increasing the production of germination stimulants.

Therefore, in one embodiment, the present invention provides methods of making low-stimulant producing plants or plant varieties, which have enhanced resistance to one or more species of parasitic weeds. In addition, the present invention shows that sub-lethal concentrations of carotenoid biosynthesis inhibitors can reduce the formation of germination stimulants. The present disclosure also provides a method to use herbicides (carotenoid biosynthesis inhibitors, such as fluridone, norflurazone, isoxaflutole, flurtamone, clomazone, flurochloridone, pyridazinone, nicotinanilide, amitrol, naproxen and/or abamine, or others) to reduce parasitic weed infestation.

The parasitic weed resistance mechanisms of the invention (modulating levels of germination stimulants) may be combined with other resistance mechanisms, such as incompatibility or the presence of phytoalexins, for more durable resistance. Also provided are high stimulant producing plants or varieties that are more efficient trap or catch crops and/or can induce suicidal germination at greater distances from the plant root.

Most agricultural plants form arbuscular mycorrhizas, a beneficial relationship between plant roots and certain root-inhabiting fungi. Interestingly, two groups have reported that mycorrhiza can reduce Striga infection of sorghum and maize (Gworgwor and Weber, 2003, Mycorrhiza, 13, 277-281;Lendzemo, 2004, PhD thesis, ;Lendzemo and Kuyper, 2001, Agr Ecosyst Environ, 87, 29-35). The mechanism of this reduction was so far unknown, and therefore the possibilities to optimise and explore this phenomenon were limited. In the present invention, we show that this reduction is due to a decrease in germination stimulant formation after mycorrhizal colonisation. Therefore, the present disclosure provides a method to optimise the use of mycorrhizae for controling parasitic plants by using a germination bioassay to analyse the effect of mycorrhizae on germination stimulant formation.

### GENERAL DEFINITIONS

"Germination stimulants" or "parasitic weed seed germination stimulants" refer to strigolactones, which are capable of stimulating the seed germination of parasitic weed species, especially Striga and Orobanche species, but are also known to be the "branching factor" that mycorrhizae need to recognise and colonise their host (Akiyama et al., 2005, Nature 435, 824-827).

The strigolactones have the chemical formula as depicted in Figure 1. Individual members of the strigolactones are strigol, sorgholactone, alectrol and orobanchol. The (tentative) identification of other strigolactones such as 1 dehydro- and 3 tetradehydrostrigol isomers and unknown strigolactones in the root exudates of sorghum and red clover demonstrate that probably (many) more members of this class exist that have not been discovered yet, and which have similar activity as the already described members.

"Isoprenoids" are molecules having a carbon skeleton derived from isoprene [CH2=C(CH3)CH=CH2], and are subdivided into groups based on their carbon number, e.g. C10 monoterpenes, C15 sesquiterpenes, C20 diterpenes, C25 sesterterpenes, C30 triterpenes, C40 tetraterpenes and C5n polyterpenes.

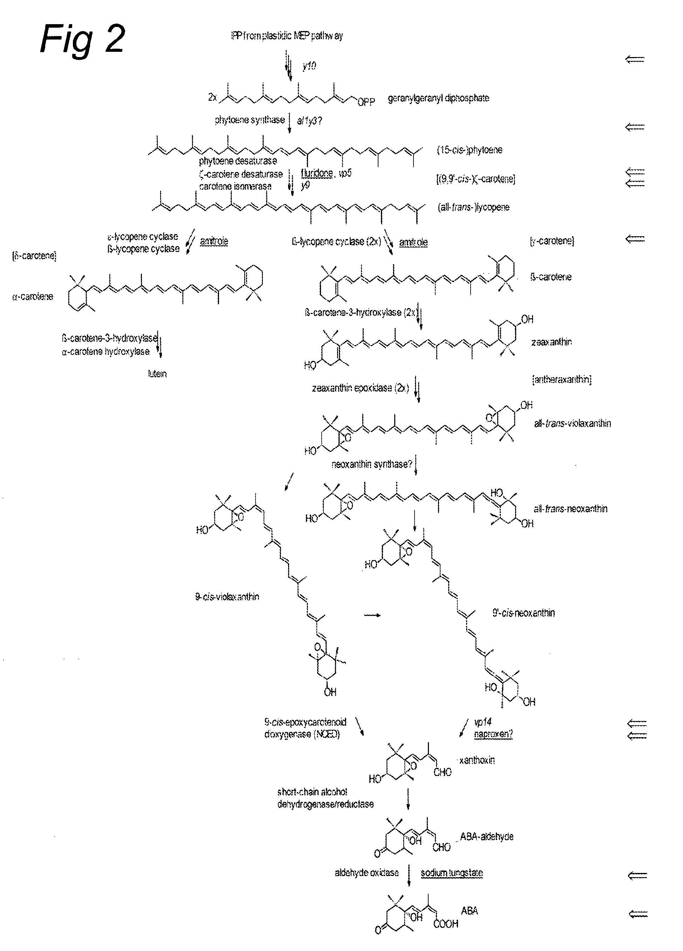

"Carotenoids" are C40 isoprenoids, derived from eight isoprene units whose order is inverted at the molecular center (Figure 2). Carotenoids are classified by their chemical structure, with carotenes being hydrocarbons and oxycarotenoids or xanthophylls having additional oxygen. In addition, carotenoids can be classified as primary or secondary, where primary carotenoids are required in photosynthesis (ß-carotene, violaxanthin and neoxanthin) whereas secondary carotenoids are localised in fruits and flowers (Delgado-Vargas et al., 2000, Crit Rev Food Sci, 40, 173-289). "Apocarotenoids" are carotenoid degradation products, such as for example the fragrance volatiles α- and ß-ionone, the pigment bixin (annatto), the mycorrhiza-induced compounds mycorradicin and blumenin and the plant hormone abscisic acid.

"Carotenoid pathway" is the biosynthetic pathway of the carotenoids. This pathway starts from the condensation of two molecules of geranylgeranyl diphosphate to form the first C40 carotenoid molecule phytoene and ends at any enzymatic or non-enzymatic carotenoid cleavage step which leads to the formation of apocarotenoids (see above).

Whenever "Striga" is used we mean one or more of the *Striga* spp. Whenever "Orobanche" is used we mean one or more of the Orobanche spp. "Parasitic plants" or "parasitic weeds" refers thus to plant species of the genus Striga and/or Orobanche. The term "nucleic acid sequence" (or nucleic acid molecule) refers to a DNA or RNA molecule in single or double stranded form, particularly a DNA encoding a protein or protein fragment according to the invention. An "isolated nucleic acid sequence" refers to a nucleic acid sequence which is no longer in the natural environment from which it was isolated, e.g. the nucleic acid sequence in a bacterial host cell or in the plant nuclear or plastid genome.

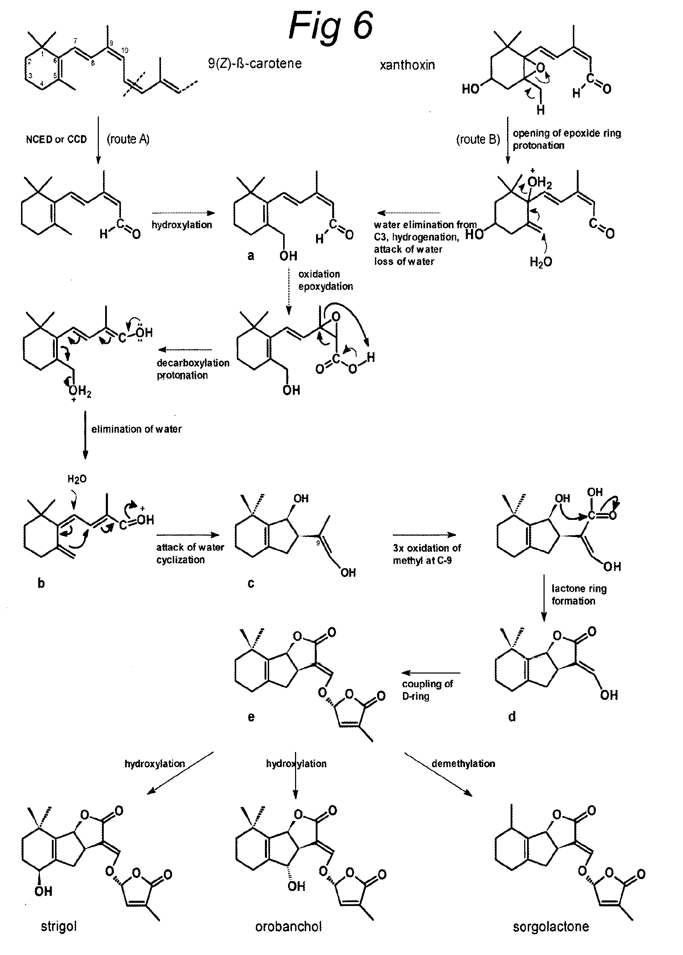

"Target enzyme(s)" or "carotenoid pathway enzymes" are enzymes involved in carotenoid biosynthesis or catabolism (e.g. carotenoid catabolism to strigolactone germination stimulants) and wherein the genes encoding these enzymes are suitable targets for changing (e.g. enhancing or reducing) strigolactone germination stimulant formation in a plant cell, tissue or plant, e.g. by modulating their expression using e.g. gene silencing or overexpression approaches. Herein two categories of target enzymes are referred to: (i) "primary carotenoid pathway enzymes", which are enzymes from the primary carotenoid/ABA biosynthetic pathway (see also Figure 2), such as, but not limited to, phytoene synthase (EC **2.5.1**.-), phytoene desaturase (EC 1.14.99.-), ξ-carotene desaturase (1.14.99.30), carotene isomerase, lycopene cyclase (EC 1.14.-.-), ß-carotene hydroxylase (EC 1.14.13.-), zeaxanthin epoxidase (EC 1.14.-.-), neoxanthin synthase and (ii) "strigolactone biosynthetic enzymes" or "secondary carotenoid pathway enzymes" or "enzymes involved in carotenoid catabolism to strigolactone germination stimulants", which are enzymes from and downstream of the branch point in the carotenoid pathway where germination stimulant formation branches away from the the primary carotenoid/ABA biosynthetic pathway (see also Figure 6), such as, but not limited to, carotenoid cleavage dioxygenases (CCD), 9-*cis*-epoxycarotenoid dioxygenase (NCED), cytochrome P450 hydroxylases, epoxidases, dehydrogenases, demethylase and the D-ring coupling enzyme. Similarely, the genes encoding these enzymes are referred to as "target genes", "carotenoid pathway genes", etc.

The terms "protein" or "polypeptide" are used interchangeably and refer to molecules consisting of a chain of amino acids, without reference to a specific mode of action, size, 3-dimensional structure or origin. A "fragment" or "portion" of a carotenoid cleavage dioxygenase (CCD) protein may thus still be referred to as a "protein". An "isolated protein" is used to refer to a protein which is no longer in its natural environment, for example in vitro or in a recombinant bacterial or plant host cell. An enzyme is a protein comprising enzymatic activity.

The term "gene" means a DNA sequence comprising a region (transcribed region), which is transcribed into an RNA molecule (e.g. an mRNA) in a cell, operably linked to suitable regulatory regions (e.g. a promoter). A gene may thus comprise several operably linked sequences, such as a promoter, a 5' leader sequence comprising e.g. sequences involved in translation initiation, a (protein) coding region (cDNA or genomic DNA) and a 3'non-translated sequence comprising e.g. transcription termination sites.

A "chimeric gene" (or recombinant gene) refers to any gene, which is not normally found in nature in a species, in particular a gene in which one or more parts of the nucleic acid sequence are present that are not associated with each other in nature. For example the promoter is not associated in nature with part or all of the transcribed region or with another regulatory region. The term "chimeric gene" is understood to include expression constructs in which a promoter or transcription regulatory sequence is operably linked to one or more coding sequences or to an antisense (reverse complement of the sense strand) or inverted repeat sequence (sense and antisense, whereby the RNA transcript forms double stranded RNA upon transcription).

"Expression of a gene" refers to the process wherein a DNA region, which is operably linked to appropriate regulatory regions, particularly a promoter, is transcribed into an RNA, which is biologically active, i.e. which is capable of being translated into a biologically active protein or peptide (or active peptide fragment) or which is active itself (e.g. in posttranscriptional gene silencing or RNAi). The coding sequence is preferably in sense-orientation and encodes a desired, biologically active protein or peptide, or an active peptide fragment. In gene silencing approaches, the DNA sequence is preferably present in the form of an antisense DNA or an inverted repeat DNA, comprising a short sequence of the target gene in antisense or in sense and antisense orientation. "Ectopic expression" refers to expression in a tissue in which the gene is normally not expressed. A "transcription regulatory sequence" is herein defined as a nucleic acid sequence that is capable of regulating the rate of transcription of a (coding) sequence operably linked to the transcription regulatory sequence. A transcription regulatory sequence as herein defined will thus comprise all of the sequence elements necessary for initiation of transcription (promoter elements), for maintaining and for regulating transcription, including e.g. attenuators or enhancers. Although mostly the upstream (5') transcription regulatory sequences of a coding sequence are referred to, regulatory sequences found downstream (3') of a coding sequence are also encompassed by this definition.

As used herein, the term "promoter" refers to a nucleic acid fragment that functions to control the transcription of one or more genes, located upstream with respect to the direction of transcription of the transcription initiation site of the gene, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active in most tissues under most physiological and developmental conditions. An "inducible" promoter is a promoter that is physiologically (e.g. by external application of certain compounds) or developmentally regulated. A "tissue specific" promoter is preferrentially (not necessarily exsclusively) active in specific types of tissues or cells. For example, a root specific promoter is a promoter which is mainly (and preferably exclusively) active in the root tissue.

As used herein, the term "operably linked" refers to a linkage of polynucleotide elements in a functional relationship. A nucleic acid is "operably linked" when it is placed into a functional relationship with another nucleic acid sequence. For instance, a promoter, or rather a transcription regulatory sequence, is operably linked to a coding sequence if it affects the transcription of the coding sequence. Operably linked means that the DNA sequences being linked are typically contiguous and, where necessary to join two protein encoding regions, contiguous and in reading frame so as to produce a "chimeric protein". A "chimeric protein" or "hybrid protein" is a protein composed of various protein "domains" (or motifs) which is not found as such in nature but which a joined to form a functional protein, which displays the functionality of the joined domains (for example DNA binding or repression leading to a dominant negative function). A chimeric protein may also be a fusion protein of two or more proteins occurring in nature. The term "domain" as used herein means any part(s) or domain(s) of the protein with a specific structure or function that can be transferred to another protein for providing a new hybrid protein with at least the functional characteristic of the domain.

The terms "target peptide" refers to amino acid sequences which target a protein to intracellular organelles such as vacuoles, plastids, preferably chloroplasts, mitochondria, leucoplasts or chromoplasts, the endoplasmic reticulum, or to the extracellular space (secretion signal peptide). A nucleic acid sequence encoding a target peptide may be fused (in frame) to the nucleic acid sequence encoding the amino terminal end (N-terminal end) of the protein or may replace part of the amino terminal end of the protein.

A "nucleic acid construct" or "vector" is herein understood to mean a man-made nucleic acid molecule resulting from the use of recombinant DNA technology and which is used to deliver exogenous DNA into a host cell. The vector backbone may for example be a binary or superbinary vector (see e.g. US5591616, US2002138879 and WO9506722), a co-integrate vector or a T-DNA vector, as known in the art and as described elsewhere herein, into which a chimeric gene is integrated or, if a suitable transcription regulatory sequence is already present, only a desired nucleic acid sequence (e.g. a coding sequence, an antisense or an inverted repeat sequence) is integrated downstream of the transcription regulatory sequence. Vectors usually comprise further genetic elements to facilitate their use in molecular cloning, such as e.g. selectable markers, multiple cloning sites and the like (see below).

A "host cell" or a "recombinant host cell" or "transformed cell" are terms referring to a new individual cell (or organism) arising as a result of at least one nucleic acid molecule, especially comprising a chimeric gene encoding a desired protein or a nucleic acid sequence which upon transcription yields an antisense RNA or an inverted repeat RNA (or hairpin RNA) for silencing of a target gene/gene family, having been introduced into said cell. The host cell may be any eukaryotic or prokaryotic cell e.g. a plant cell, microbial, insect or mammal (including human) cell. The host cell may contain the nucleic acid construct as an extra-chromosomally (episomal) replicating molecule, or more preferably, comprises the chimeric gene integrated in the nuclear or plastid genome of the host cell. Included are any derivatives of the host cell, such as tissues, whole organism, cell cultures, explants, protoplasts, further generations, etc. derived from the cell which retain the introduced gene.

In some embodiments the term "host" is used in the pathological sense and refers to the plant species which is infected by parasitic weed species, but this use of the term will be clear from the context.

The term "selectable marker" is a term familiar to one of ordinary skill in the art and is used herein to describe any genetic entity which, when expressed, can be used to select for a cell or cells containing the selectable marker. Selectable marker gene products confer for example antibiotic resistance, or herbicide resistance or another selectable trait such as a phenotypic trait (e.g. a change in pigmentation) or a nutritional requirement. The term "reporter" is mainly used to refer to visible markers, such as green fluorescent protein (GFP), eGFP, luciferase, GUS and the like.

The term "ortholog" of a gene or protein refers herein to the homologous gene or protein found in another species, which has the same function as the gene or protein, but is (usually) diverged in sequence from the time point on when the species harbouring the genes diverged (i.e. the genes evolved from a common ancestor by speciation). Orthologs of the genes of interest may thus be identified in other plant, animal, bacterial or fungal species based on both sequence comparisons (e.g. based on percentages sequence identity over the entire sequence or over specific domains) and/or functional analysis.

The terms "homologous" and "heterologous" refer to the relationship between a nucleic acid or amino acid sequence and its host cell or host organism, especially in the context of transgenic cells / organisms. A homologous sequence is thus naturally found in the host species (e.g. a tomato plant transformed with a tomato gene), while a heterologous sequence is not naturally found in the host cell (e.g. a tomato plant transformed with a sequence from potato plants). Depending on the context, the term "homolog" or "homologous" may alternatively refer to sequences which are descendent from a common ancestral sequence (e.g. they may be orthologs).

"Stringent hybridisation conditions" can be used to identify nucleotide sequences, which are substantially identical to a given nucleotide sequence. Stringent conditions are sequence dependent and will be different in different circumstances. Generally, stringent conditions are selected to be about 5°C lower than the thermal melting point (Tm) for the specific sequences at a defined ionic strength and pH. The Tm is the temperature (under defined ionic strength and pH) at which 50% of the target sequence hybridises to a perfectly matched probe. Typically stringent conditions will be chosen in which the salt concentration is about 0.02 molar at pH 7 and the temperature is at least 60°C. Lowering the salt concentration and/or increasing the temperature increases stringency. Stringent conditions for RNA-DNA hybridisations (Northern blots using a probe of e.g. 100nt) are for example those which include at least one wash in 0.2X SSC at 63 ° C for 20min, or equivalent conditions. Stringent conditions for DNA-DNA hybridisation (Southern blots using a probe of e.g. 100nt) are for example those which include at least one wash (usually 2) in 0.2X SSC at a temperature of at least 50° C, usually about 55°C, for 20 min, or equivalent conditions. See also Sambrook et al. (1989) Molecular Cloning: A Laboratory Manual, Second Edition, Cold Spring Harbor Laboratory Press, and Sambrook and Russell (2001) Molecular Cloning: A Laboratory Manual, Third Edition, Cold Spring Harbor Laboratory Press, NY.

"Sequence identity" and "sequence similarity" can be determined by alignment of two peptide or two nucleotide sequences using global or local alignment algorithms. Sequences may then be referred to as "substantially identical" or "essentially similar" when they (when optimally aligned by for example the programs GAP or BESTFIT using default parameters) share at least a certain minimal percentage of sequence identity (as defined below). GAP uses the Needleman and Wunsch global alignment algorithm to align two sequences over their entire length, maximizing the number of matches and minimises the number of gaps. Generally, the GAP default parameters are used, with a gap creation penalty = 50 (nucleotides) / 8 (proteins) and gap extension penalty = 3 (nucleotides) / 2 (proteins). For nucleotides the default scoring matrix used is nwsgapdna and for proteins the default scoring matrix is Blosum62 (Henikoff & Henikoff, 1992, PNAS 89, 915-919). Sequence alignments and scores for percentage sequence identity may be determined using computer programs, such as the GCG Wisconsin Package, Version 10.3, available from Accelrys Inc., 9685 Scranton Road, San Diego, CA 92121-3752 USA or the open-source software Emboss for Windows (current version 2.7.1-07). Alternatively percent similarity or identity may be determined by searching against databases such as FASTA, BLAST, etc.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one", e.g. "a cell" refers also to several cells in the form of cell cultures, tissues, whole organism, etc. It is further understood that, when referring to "sequences" herein, generally the actual physical molecules with a certain sequence of subunits (e.g. amino acids) are referred to.

### DETAILED DESCRIPTION OF THE INVENTION

In a series of experiments with pathway inhibitors and maize mutants the present inventors surpisingly found that the germination stimulants of S. hermonthica present in the root exudates of maize, cowpea and sorghum are derived from the carotenoid biosynthetic pathway. This is unexpected, since the strigolactones have always been classified as sesquiterpenes, which is a principally different chemical class (see e.g. Akiyama et *al.*, 2005, supra; Cook et *al.,* 1972, supra; Butler, 1995, supra; Hauck et *al.,* 1992, supra; Siame et *al.,* 1993, supra; Muller et *al.,* 1992, supra; Yokota et *al.,* 1998, supra; Yoneyama et *al.,* 2004, supra). Further, it was found that this also holds for the germination stimulant(s) of *O*. crenata in the root exudate of cowpea. For these three host species - maize, cowpea and sorghum - the germination stimulants have been isolated and identified to be strigolactones, *viz.* strigol, alectrol and sorgolactone (Figure 1) (Hauck et al., 1992, supra; Muller et al., 1992, supra; Siame et al., 1993, supra). The present results with *O*. crenata suggest that this species also responds to a strigolactone germination stimulant. *O*. crenata is not a parasite of cowpea but parasitizes legumes in more-temperate climates such as North-Africa and Spain. The germination stimulant(s) of *O*. crenata has not been identified yet. Root exudates of cowpea readily induced germination of *O*. crenata and fluridone treatment decreased this germination by about the same percentage as for S. hermonthica. This makes it likely that the germination stimulants of Orobanche spp. that parasitise legumes in temperate regions of the world are also strigolactones and are hence also derived from the carotenoid pathway. This hypothesis is supported by the detection of orobanchol, alectrol and a third unidentified strigolactone in the legume red clover (Yokota et al., 1998, supra) and alectrol in cowpea (Muller et al., 1992, supra).

The fact that the present inventors demonstrate the carotenoid-origin of the germination stimulants for two parasitic plant species and three mono- and dicotyledonous hosts, and the (tentative) identification of strigolactones in the root exudates of other plant species such as red clover and tomato (Yokota et al., 1998, supra; Yoneyama et al., 2004, supra) suggest that carotenoid-derived germination stimulant formation occurs throughout the plant kingdom. This finding enables for the first time to effectively modify the levels of one or more germination stimulants produced by a plant cell, tissue, organ or whole plant, thereby modifying the resistance to one or more parasitic weed species (see further below).

The finding of the inventors that sorghum root exudate induced S. *hermonthica* germination is completely blocked by fluridone sheds an interesting light on the discussion about the "true" nature of the sorghum germination stimulant. Lynn and coworkers have claimed that sorgoleone is the natural sorghum germination stimulant but this was disputed by Zwanenburg and coworkers based a.o. on the low water solubility of dihydrosorgoleone (Butler, 1995, supra; Keyes et al., 2001, supra; Wigchert and Zwanenburg, 1999, supra). The finding herein makes it very likely that indeed the natural sorghum germination stimulant is a strigolactone.

The germination stimulants are produced in extremely low concentrations, which causes large difficulties for an analytical approach to discover the biosynthetic origin of the germination stimulants. The present inventors used a germination bioassay-guided approach herein to unravel the true biosynthetic pathway of germination stimulants. For these bioassays, the seeds of *Orobanche* and *Striga* spp. were preconditioned for a certain period of time at a suitable temperature such that the seeds become responsive to germination stimulants (Matusova et al., 2004, Seed Sci Res, 14, 335-344) (see Example 1). Seedlings of plant species to be investigated were grown in perlite, in sand or on filter paper, conditions under which the effect of inhibitors and transgenes can be investigated. After 1-2 weeks, seedlings were taken from the growing medium, carefully cleaned if necessary and then put into tap water in glass tubes for 6-24 hrs. After root exudate collection, root weight was determined and root exudates were diluted to equal root weight per volume concentrations. 50 µl of the test solutions were added to duplicate or triplicate disks containing preconditioned seeds and germination evaluated under a disecting microscope. The synthetic germination stimulant GR24 (0.01 and 0.1 mg.L⁻¹ GR24) as a positive and water as a negative control were included in each bioassay. Alternative bioassays used are the *in situ* assays described for rice (Example 3) and tomato (Example 7). In these bioassays, the germination of Striga and Orobanche seeds was evaluated by placing the seeds close to the roots of plants growing in sand (rice) or growing on filter paper (tomato).

The above bioassay, and its use for various purposes, such as testing and identifying or selecting plants (especially recombinant or mutant plants) for the production of germination stimulants, is provided. The assay may also be used to identify or select target genes and to verify the function of target genes, as described elsewhere herein or the use of mycorrhizae or chemicals to reduce germination stimulant production as described elsewhere herein. The bioassay preferably involves testing the germination of Orobanche and/or Striga seeds in the presence of roots, root exudates or root extracts and identifying a plant (or a transgene) which results in lower or higher germination compared to a control. Preferably, the roots, root exudates or root extracts are from a recombinant plant overexpressing a target gene or wherein a target gene is down-regulated.

For downregulated target genes (especially under control of a root-specific promoter), it is preferred that those plants are identified and/or selected of which the roots, root exudates or root extracts result in a significantly lower seed germination percentage of Orobanche and/or Striga seeds compared to a suitable control (e.g. a non-recombinant control or an empty-vectro control). A significantly lower germination is a reduced percentage of germination of Orobanche and/or Striga seeds by at least 20% compared to the control, preferably at least 30%, 40%, 50%, or more.

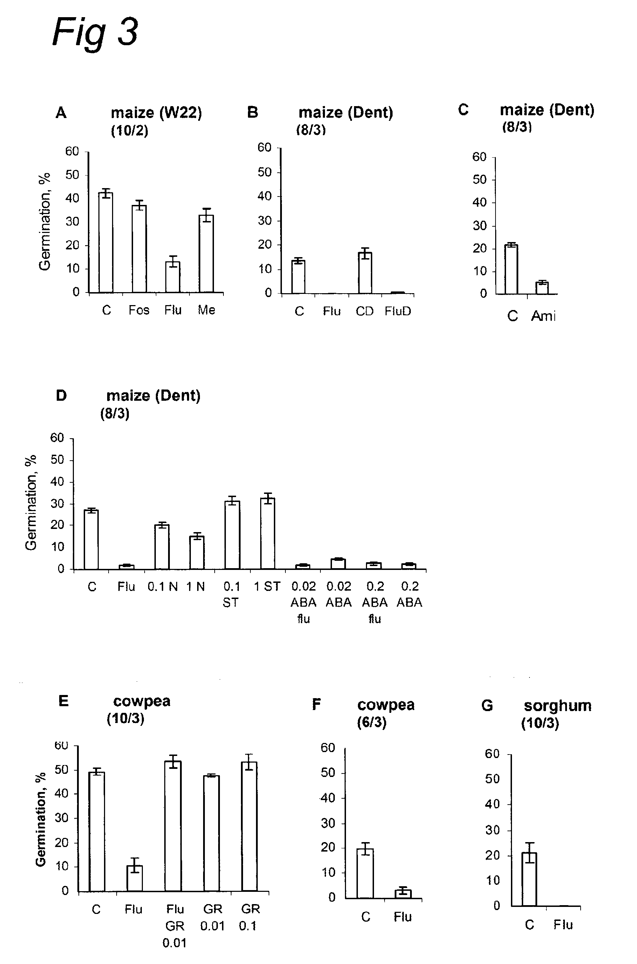

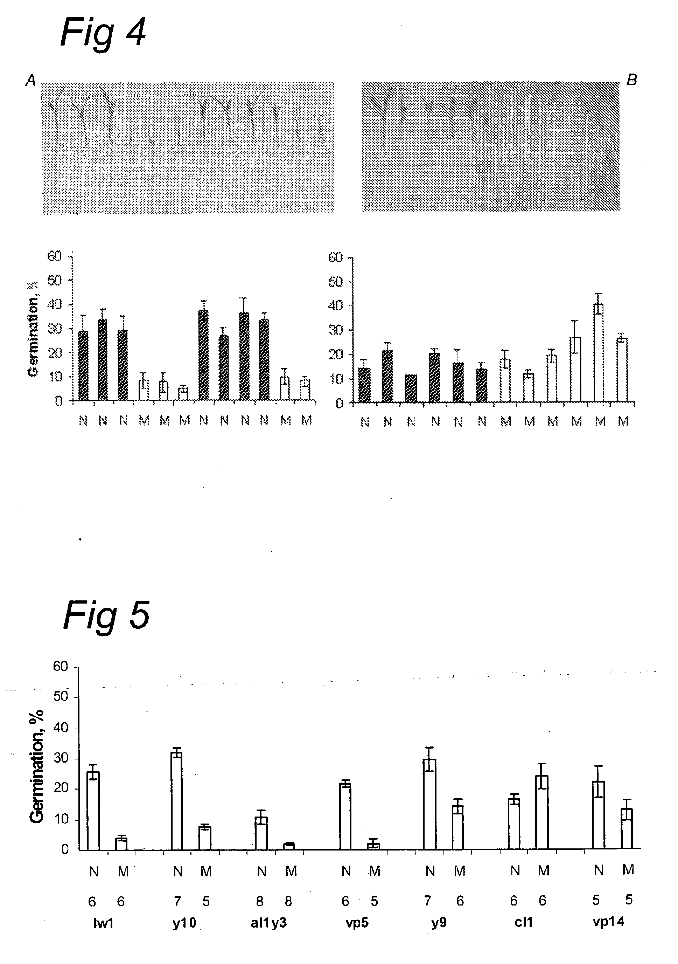

The carotenoid biosynthesis inhibitor fluridone effectively blocks the activity of phytoene desaturase which catalyses two desaturation steps converting phytoene to ζ-carotene and corresponds to the *vp*5 locus (Hable et al., 1998, Mol Gen Genet, 257, 167-176; Li et al., 1996, Plant Mol. Biol., 30, 269-279). The vp5 mutant of maize has a lesion in the phytoene desaturase gene (Hable et al., 1998, supra) and reduced Pds transcript in maize endosperm was detected in this mutant (Li et al., 1996, supra). The vp5 mutant results in lethality of the plant at the seedling stage. Surprisingly, both fluridone-treated maize and the *vp*5 mutant root exudates induced significantly lower germination of S. *hermonthica* (Figures 3, 5).

Both mevastatin and fosmidomycin also slightly, but not significantly, reduced root exudate induced germination (Figure 3A). Both compounds have been demonstrated to be quite efficiently inhibiting the cytosolic or plastidic isoprenoid biosynthetic pathway, respectively (Laule et al., 2003, Proc Natl Acad Sci USA, 100, 6866-6871) and hence one might expect a clear effect of fosmidomycin, which in principle should also block carotenoid biosynthesis. However, fosmidomycin and mevastatin have also been used to prove exchange of isoprenoid intermediates between cytosol and cytoplasm, likely in the form of IPP (Botella-Pavia et al., 2004, Plant J, 40, 188-199; Hemmerlin et al., 2003, J Biol Chem, 278, 26666-26676). Recently, it was also shown that the exchange from cytosol to plastids occurs particularly in the dark (Botella-Pavia et al., 2004, supra). All this suggests that the inhibition by fosmidomycin of carotenoid formation was less effective than by fluridone, perhaps as a consequence of isoprenoid precursor supply from the cytosol. This assumption is supported by the phenotype of the treated seedlings. Fosmidomycin-treated seedlings did not exhibit the bleached phenotype that fluridone-treated seedlings showed. The modest inhibition of root exudate induced germination by mevastatin is less easy to explain. Possibly, inhibition of the cytosolic IPP formation also diminishes plastidic IPP concentrations either because there is no IPP export from the cytosol to the plastids anymore or because IPP is now exported more readily from the plastids into the cytosol. Alternatively, it could be that part of the germination stimulant biosynthesis occurs in the cytosol. In abscisic acid biosynthesis, xanthoxin is transported out of the plastids into the cytosol, where it is further processed to form ABA. Possibly, the strigolactone precursor is also exported to the cytosol after carotenoid cleavage. There, several additional enzymatic reactions would still be required (see below) and the D-ring would have to be coupled. The origin of the D-ring is as yet completely unknown, but it seems to be isoprenoid. It could arise from coupling of the tricyclic A-B-C skeleton to an isoprenoid unit such as dimethylallyl diphosphate or 4-hydroxydimethylallyl diphosphate by the action of a prenyl transferase. If this occurs in the cytosol then mevastatin treatment could also lead to a lower formation of germination stimulants, as the D-ring is required for biological activity (Wigchert and Zwanenburg, 1999, supra).

In addition to fluridone-treated maize seedlings, the root exudates of maize carotenoid mutants *lw1, y10, al1y3,* vp5 and y9 also induced lower germination of S. *hermonthica* seeds than their corresponding wild-type phenotype siblings. These results indicate that the biosynthetic pathway leading to the production of the strigolactone germination stimulant branches from the main carotenoid pathway below phytoene (*vp*5 mutant and fluridone) and ζ-carotene (y9 mutant). In maize, only four genes encoding early steps in carotenoid biosynthesis have been cloned and characterized so far. The maize *y1* (*yellow* 1) gene encoding phytoene synthase, the enzyme that catalyse the first biosynthetic step specific to carotenoid production (Buckner et al., 1990, Plant Cell, 2, 867-876; Buckner et al., 1996, Genetics, 143, 479-488), the maize phytoene desaturase corresponding to the *vp*5 locus in maize (Hable et al., 1998, supra; Li et al., 1996, supra), the maize ζ-carotene desaturase corresponding to the vp9 (*viviparous 9*) locus (Luo and Wurtzel, 1999, Plant Physiol., 120, 1206; Matthews et al., 2003, J Exp Bot, 54, 2215-2230) and the *Ps1* (*Pink scutellum* 1) gene corresponding to the *vp7* locus encoding lycopene-β-cyclase (Singh et al., 2003, Plant Cell, 15, 874-884).

The inventors' results in germination bioassays with root exudates of amitrole treated plants (Figures 2, 3C) confirmed that the branch point is below lycopene. Accumulation of lycopene in maize plants treated with amitrole and grown at 20°C was observed (Dalla Vecchia et al., 2001, supra). In plants, the linear carotene lycopene is the precursor for cyclic carotenoids (reviewed by (Hirschberg, 2001, Curr Opin Plant Biol, 4, 210-218) at the branch point of two pathways. In one branch, the formation of a ß-ionone ring at both ends of lycopene through the action of lycopene ß-cyclase leads to the formation of ß-carotene and its derivatives, including abscisic acid. In the other branch, the co-action of a lycopene ε-cyclase and lycopene ß-cyclase leads to the formation of α-carotene and its derivatives, including lutein.

### Carotenoid cleavage enzymes

In a next step the present inventors tested the effect of naproxen, a putative inhibitor of epoxy-carotenoid cleavage (Lee and Milborrow, 1997, Aust J Plant Physiol, 24, 715-726) (Figure 2). The formation of the germination stimulant of maize was indeed reduced by the action of naproxen (Figure 3D). This suggests that carotenoid cleavage is involved in its biosynthesis, which is logical as the C40-carotenoids need to be cleaved in order to lead to the C14 (excluding the D-ring) strigolactones. Naproxen has been used to inhibit the 9-*cis*-epoxycarotenoid dioxygenase (NCED), a sub-category of the carotenoid cleavage dioxygenases (CCDs), that catalyses the oxidative cleavage of 9-*cis*-epoxy-carotenoids to apocarotenoids (C25) and xanthoxin (C15), the precursor of abscisic acid in higher plants (Schwartz, S. H. et al., 1997, Science, 276, 1872-1874). After cleavage, xanthoxin is exported to the cytosol where further conversion to abscisic acid takes place (Figure 2) (Seo and Koshiba, 2002, Trends Plant Sci, 7, 41-48). The herein used bioassay with *vp14*, a mutant of NCED, confirmed the result obtained with naproxen. Also *vp14* induced lower germination (Figure 5). Both naproxen and *vp14* were not fully effective in inhibiting root exudate induced germination but only gave about 44% inhibition compared with the corresponding control/wildtype seedlings (Figures 3D,5). This agrees quite well with results obtained by others. Lee and Milborrow reported that in a cell-free system of avocado, naproxen reduced radiolabel accumulation from [¹⁴C]-MVA in abscisic acid by 43% (Lee and Milborrow, 1997, supra). In the maize *vp14* mutant, the mutation in NCED reduced abscisic acid accumulation in embryos by 40-60%, depending on embryo-developmental stage, and abscisic acid accumulation in water-stressed leaves by 45% (Tan et al., 1997, Proc Natl Acad Sci USA, 94, 12235-12240).

From the maize mutant *vp14-*2274, NCED was cloned and the substrate specificity characterized (Schwartz, S. H. et al., 2003, Biochim Biophys Acta-Gen Subjects, 1619, 9-14). The enzyme requires a 9-cis double bond adjacent to the site of cleavage (the 11-12 bond) and cleaves 9-cis-violaxanthin, 9-*cis*-neoxanthin and 9-*cis-*zeaxanthin (Schwartz et al., 2003, supra). However, the structural requirements in the carotenoid ring for cleavage have not been investigated and thus it is conceivable that NCED is nonspecific and can cleave other 9-cis carotenoids such as 9-*cis*-β-carotene (Figure 6). Other 9-*cis-*carotenoids, such as 9-*cis*-ß-carotene and 9-*cis*-zeaxanthin have been reported to occur in a range of plant species (Ben-Amotz and Fishler, 1998, Food Chem, 62, 515-520). How these cis-isomers arise is unknown. However, recently it has been established that cis-isomers of early intermediates such as phytoene, phytofluene, ξ-carotene, neurosporene and lycopene are naturally occurring isomers in carotenoid biosynthesis (Isaacson et al., 2004, Plant Physiol., 136, 4246-4255; Park et al., 2002, Plant Cell, 14, 321-332). A carotene isomerase has been discovered that is responsible for the cis to *trans* isomerisation during the process of all-*trans*-lycopene formation. It is unclear whether *cis-*isomers of for example β-carotene arise from these early *cis-*intermediates or from trans to cis isomerisation in a later stage.

*Vp14* is expressed constitutively in maize embryos and roots and in Southern blots at least 9 bands were detected when hybridizing with *vp14* cDNA (Tan et al., 1997, Proc Natl Acad Sci USA, 94, 12235-12240). This may also explain why the mutation in *vp14* is not 100% effective in inhibiting abscisic acid (Tan et al., 1997, supra) nor germination stimulant formation (Figure 5), as *NCED* family members may partly compensate for the NCED activity lost in *vp14. NCED* cDNAs have been cloned from several other plant species, such as *Phaseolus vulgaris* (Qin and Zeevaart, 1999, Proc Natl Acad Sci USA, 96, 15354-15361), cowpea (luchi et al., 2000, Plant Physiol., 123, 553-562), avocado (Chernys and Zeevaart, 2000, Plant Physiol., 124, 343-353) and Arabidopsis (Neill et al., 1998, J Exp Bot, 49, 1893-1894). Just like in maize, in all these species, *NCEDs* were found to belong to a gene family. Luchi et al. (Luchi et al., 2000, supra) characterized *VuNCED1* in cowpea which was strongly induced by drought stress in leaves and stems but not in roots. Under stress conditions abscisic acid accumulated in a similar pattern, i.e. mainly in leaves and stems.

Several authors have also cloned CCDs from a range of plant species. For example, several CCDs were cloned from Arabidopsis (Schwartz, Steven H. et al., 2001, J Biol Chem, 276, 25208-25211;Schwartz, S. H. et al., 2004, J Biol Chem, 279, 46940-46945). *AtCCD1* cleaves carotenoids at the 9-10 and 9'-10' position. A homologue of *At*CCD1 was postulated to catalyse the cleavage of a carotenoid precursor to a C14 dialdehyde which is probably the precursor of mycorradicin, the major component of yellow pigment observed in roots colonized by arbuscular mycorrhiza (Fester et al., 2002b,Planta, 216, 148-154; Schwartz et al., 2001, supra) that was first identified in maize roots (Klingner et al., 1995, Phytochemistry, 38, 53-55). The function of these apocarotenoids is unknown but they have been shown to be produced upon mycorrhizal colonization by a multitude of plant species such as maize and other cereals, Medicago *truncatula,* tomato and tobacco (Fester et al., 2002a, Plant Cell Physiol, 43, 256-265;Strack et al., 2003, J Chem Ecol, 29, 1955-1979;Walter et al., 2000, Plant J, 21, 571-578).

### Biosynthetic fate after carotenoid cleavage

The inhibitor of abscisic acid-aldehyde oxidation, sodium tungstate did not have an effect, but abscisic acid strongly reduced root exudate induced *S*. *hermonthica* germination (Figure 3). This showed that the germination stimulants are not derived from intermediates below abscisic acid-aldehyde nor from abscisic acid itself. The reduction of root exudate induced germination by abscisic acid is most probably due to feed back inhibition by the exogenously applied abscisic acid on the carotenoid pathway. The levels of violaxanthin and ß-carotene were indeed reduced by about 35 and 65%, respectively, in abscisic acid-treated roots and in the shoot a similar trend was observed (Table 1). Consequently the formation of germination stimulant branching from the carotenoid biosynthetic pathway is also decreased. Very little is known about the effects of exogenously applied abscisic acid on carotenoid and abscisic acid biosynthesis in roots and other non photosynthetic tissues. Feedback regulation of phytoene desaturase in green tissue by accumulation of abscisic acid, the end-product of the carotenoid pathway, was proposed (Corona et al., 1996, Plant J, 9, 505-512). In Arabidopsis seedlings, exogenously applied abscisic acid did not affect the transcript level of *NCED* (Xiong et al., 2001b, Plant Cell, 13, 2063-2083). In contrast, positive feedback regulation by abscisic acid at the transcriptional level was observed for *AAO3* (abscisic acid aldehyde oxidase) (Seo et al., 2000, Proc Natl Acad Sci USA, 97, 12908-12913) and *abscisic acid3* (a molybdenum cofactor sulfurase) (Xiong et al., 2001a, Dev Cell, 1, 771-781).

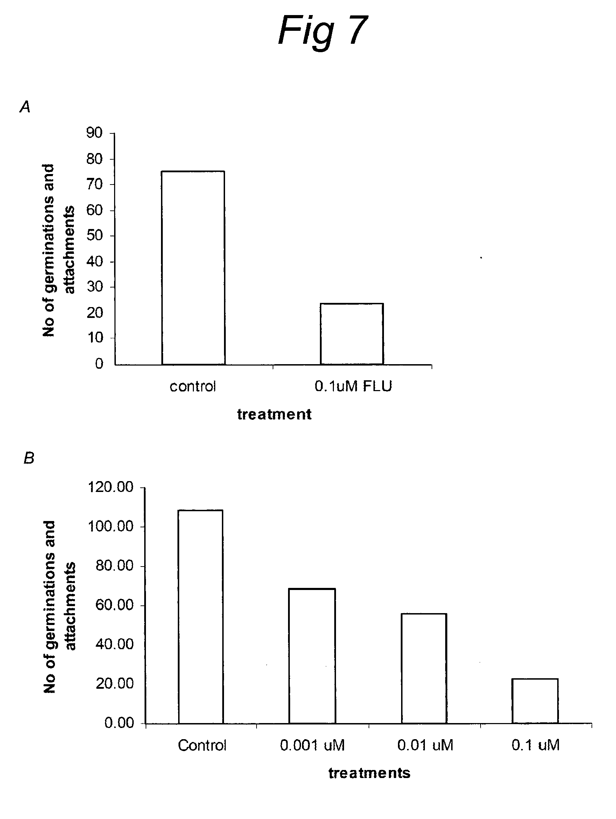

In rice, the inventors used *in situ* assays to assess the effect of carotenoid biosynthesis inhibitors and also the possibility to apply the inhibitors, in low, sublethal, concentrations, to the root or shoot and hence influence Striga germination. Irrigation with a low concentration of fluridone, significantly reduced the number of germinated/attached Striga seeds. Control experiments, where the synthetic germination stimulant GR24 was used to induce full germination, showed that fluridone was not affecting the attachment phase (but just germination) (data not shown). In a subsequent experiment fluridone was applied by spraying, and also here a strong, significant reduction in the number of germinated/attached seeds/tubercles of Striga was obtained even with very low concentrations. In both experiments, the concentrations of fluridone used were so low that bleaching of the leaves did not occur. Although the rice germination stimulant has sofar not been identified these results clearly demonstrate that they must also be strigolactones, and that hence rice - because the genome is sequenced, it can be easily transformed and because the inventors have herein developed a suitable bioassay to evaluate strigolactone formation on individual plants - is a suitable model to look for strigolactone biosynthetic pathway gene candidates. These experiments show, that herbicides which inhibit carotenoid biosynthesis can be used for significantly reducing the germination of parasitic seeds and that spraying or irrigating plants or soil with such herbicides at one or more time intervals is an effective way for reducing parasitic-weed induced yield losses of crop plants. Especially, the use of low amounts of inhibitors is preferred, so that no bleaching of the leaves occurs but still a significant reduction in the percentage of parasitic weed germination and/or attachment is seen compared to controls (see Fig. 7). Depending on the activity of the inhibitor, for example, amounts of less than 2 µM or less than 1µM, such as equal to or less than 0.1µM, 0.01µM, 0.001µM may be used.

The present results demonstrate that the strigolactone germination stimulants of S. *hermonthica* and *O*. *crenata* in the exudates of maize, sorghum and cowpea - strigol, sorgolactone and alectrol - and the unknown strigolactone germination stimulant of rice are derived from the carotenoid biosynthetic pathway. Considering the high structural similarity of orobanchol with these other strigolactones, also orobanchol must be derived from this pathway and this will also hold for the tentative strigol-analogues, recently reported for tomato (Yoneyama et al., 2004, supra). The results with the present bioassays with inhibitors and mutants suggest that the biosynthesis of germination stimulants branches off from the carotenoid pathway at an intermediate that is a product of NCED or CCD action. This may be xanthoxin but could more likely be an analogue derived from cleavage of other substrates such as 9-*cis-*β*-*carotene. A biogenetic scheme can be postulated starting from such a 9-*cis*-β-carotene cleavage products, but also from xanthoxin (Figure 6). However, downstream derivatives of xanthoxin or alternative carotenoids could also serve as substrate in this biogenetic scheme.

Starting from 9-*cis*-ß-carotene, C11-12 cleavage by a dioxygenase leads to a C15-aldehyde, which upon hydroxylation yields intermediate a (Figure 6). Alternatively, xanthoxin, upon opening of the epoxide ring followed by protonation, elimination of water at C3 followed by hydrogenation, attack of water and loss of water could also lead to intermediate a. Oxidation followed by epoxidation and decarboxylation, protonation and elimination of water leads to intermediate b. This intermediate, upon attack of water at C7, cyclizes to intermediate c or its tautomeric aldehyde. After oxidation of the methyl at C9 to an acid function, a lactone ring will be formed with the C7 hydroxyl group to produce intermediate d. Alternatively, the methyl at C9 could already be oxidized to form a carboxyl group in compound a (not shown in Figure 6). Instead of attack of water at C7, that carboxyl group could then attack the carbocation at C7 leading directly to intermediate d. As an alternative to lactone ring formation as described above, intermediate c could undergo keto-enol tautomerization, followed by oxidation to form a carboxyl group at C10, which can then form a lactone ring with the hydroxy at C7 (not shown in Figure 6). Oxidation of the methyl at C9 to an aldehyde, would then also lead to intermediate d. From intermediate d, allylic hydroxylation in ring A or B or demethylation in ring A and coupling of the D-ring will lead to strigol, orobanchol and sorgolactone, respectively (Figure 6). The structure of alectrol is still under debate. It is not unlikely that our biogenetic scheme may help to postulate a new structure for alectrol which should subsequently be proven using chemical synthesis. Of course the order of the reactions as proposed herein may in vivo be different. In addition, it is not unlikely that the D-ring is coupled to for example intermediate d before hydroxylation/demethylation to form the three different - and other - germination stimulants.

In conclusion, the present results with mutants and inhibitors, and the postulated biogenetic scheme lead to the conclusion that a carotenoid cleaving enzyme such as CCD or NCED, is involved in the biosynthesis of the strigolactone germination stimulants in hosts of Orobanche and *Striga* spp. After this cleavage step, a number of other enzymatic reactions, such as hydroxylation, epoxydation, oxidation, demethylation, D-ring coupling, etc are involved in the further modification of the primary apocarotenoid skeleton to the different strigolactones.

### Target genes according to the invention

A method for modulating (i.e. reducing or enhancing) the amount of germination stimulant produced by a plant cell, tissue or plant is provided (see further below). This method involves either expression vectors or gene silencing vectors, comprising a suitable promoter operably linked to one or more nucleic acid sequences, whereby the introduction into a host cell results in either overexpression of the recombinant gene or silencing of the endogenous target gene or gene family. In turn, the recombinant cell, tissue or plant produces either significantly enhanced amounts of one or more germination stimulants (overexpression) or significantly reduced amounts of one or more germination stimulants (downregulation), compared to the non-recombinant control (or control plants transformed with a control vector, e.g. an empty vector). Cell, tissues or plants with modified formation of germination stimulants can be easily selected using a germination bioassay, as described above and in the Examples.

As it was found that germination stimulants are synthesized via the carotenoid pathway, all nucleic acid sequences encoding carotenoid pathway enzymes (either primary carotenoid pathway enzymes or secondary carotenoid pathway enzymes, as defined) are target DNAs for genetic engineering, breeding or selection of plant cell, tissues or whole plants with reduced or increased germination stimulant formation. Moreover, it has been suggested that direction of radicle growth after germination of parasitic plant seeds is along the germination stimulant gradient (Dube and Olivier, 2001, supra). Therefore, in addition to a direct negative effect on germination, a reduced formation of germination stimulants may also contribute in reduced success of radicle growth in the right direction.

Target enzymes of the primary carotenoid pathway are, but are not limited to, the enzymes indicated in Figure 2, such as phytoene synthase, phytoene desaturase, ξ-carotene desaturase, carotene isomerase, lycopene cyclase, ß-carotene hydroxylase, zeaxanthin epoxidase and neoxanthin synthase. The nucleic acid sequences may be derived from various sources, such as monocotyledonous or dicotyledonous plants, bacteria, fungi, etc. Nucleic acid sequences encoding such enzymes are widely available in the art and may be synthesized or may be easily cloned using existing DNA and protein information. For example, sequence information either from maize and other plant species is available in Genbank and other databases. Examples are herein provided (nucleic acid sequence ID 1, 3, 5, 7, 9, 11, 13 and 15 and amino acid sequences 2, 4, 6, 8, 10, 12, 14 and 16; see sequence listing). Sequences essentially similar to these (variants), such as for example orthologs from various species, and fragments of any of these sequences are also discussed.

The use of these cDNAs or fragments thereof (e.g. sense and/or antisense fragments) is disclosed in antisense or RNAi constructs (or vectors) to knock-down/downregulate the endogenous target gene(s) and thereby to reduce the formation of carotenoid intermediates supplying the substrate for germination stimulant formation. Preferably, these constructs are expressed in an organ-specific and/or development-specific way, such that the inhibition of carotenoid/ABA biosynthesis is restricted to time and place necessary to obtain resistance against parasitic plants and to avoid too much side-effects on mycorrhizal colonisation. Thus, in one embodiment an gene silencing vector is provided comprising a suitable promoter which is active in the host cell (preferably a tissue specific, e.g. root specific promoter) operably linked to a sense and/or antisense nucleic acid fragment of a carotenoid pathway gene. Hosts of parasitic plants (e.g. maize, tomato, sunflower, rice, etc) can be transformed with these constructs and primary, rooted, transformants easily assayed for their induction of germination of seeds of the corresponding parasitic plants using the bioassay described in this invention (Example 1). Successful inhibition of germination stimulant formation can thus be easily identified.

Also apocarotenoid pathway enzymes, i.e. the enzymes involved in cleavage and the postulated enzymes after cleavage, such as cytochrome P450 hydroxylases and epoxidases, dehydrogenaes, demethylase, a D-ring transferase etc are suitable targets for engineering, breeding or selection for reduced or increased germination stimulant formation. For genetic engineering a skilled person can clone the corresponding genes from parasitic plant hosts, such as maize, sunflower, tomato, tobacco, sorghum, millet, cowpea and rice (see below). Alternatively, the nucleic acid sequences provided herein e.g. the nucleic acid sequences encoding CCDs and *NCEDs* (sequence ID 17 and 19), and nucleic acid sequences encoding the proteins of sequence ID 18 and 20 from maize and the nucleic acid sequences encoding CCDs and **NCEDs** (sequence ID 21, 23, 25, 27, 29, 31, 33, 35, 37, 39, 41, 43, 45, 47 and 49) and nucleic acid sequences encoding the proteins of sequence ID 22, 24, 26, 28, 30, 32, 34, 36, 38, 40, 42, 44, 46, 48 and 50 from rice or sequences essentially similar thereto or encoding proteins with similar function or fragments thereof, may be used to make overexpression or gene silencing vectors. Several groups are investigating these enzymes usually in relation to the formation of flavour and fragrance compounds or pigments (Bouvier et al., 2003, Plant Cell, 15, 47-62) or in ABA biosynthesis (Schwartz et al., 2003, supra). Many CCDs and **NCEDs** have already been cloned or were identified from genome sequencing projects, eg on Arabidopsis and rice (Tan et al., 2003). A method for cloning of all rice CCDs and **NCEDs** is provided, which are used to knock- out their activity and to study the consequences of the knock-out for the induction of parasitic seed germination. In this way the rice gene that is involved in strigolactone formation is identified (see Example 7). Also, rice transposon and tDNA mutants in the CCDs and **NCEDs** can be used to this goal. From maize the present inventors have cloned two root-expressed target gene cDNAs (Sequence ID 17-20), as well as various CCD and NCED sequences from rice. A preferred embodiment is the use of these cDNAs in antisense or RNAi constructs to knock-down the cleavage of carotenoid intermediates supplying the apocarotenoid substrate for germination stimulant formation. Preferably, these constructs are expressed in an organ-specific and/or development-specific way, such that the inhibition of cleavage is restricted to time and place necessary to obtain resistance against parasitic plants.

In addition to the CCDs and NCEDs, there are several other enzyme classes that can be used as targets (Figure 6). This concerns the genes encoding enzymes that are probably more specific only for germination stimulant formation such as the, but not limited to, as yet unidentified hydroxylases, epoxidases, of which some likely belong to the cytochrome P450s, dehydrogenases, demethylase and D-ring transferase. The genes encoding these enzymes can be readily cloned using molecular and biochemical methods known to persons skilled in the art and can then be used to modify germination stimulant formation as described herein. Strategies to clone these genes include subtractive techniques or transcriptomics approaches and thus identifying genes that are downregulated by mycorrhizal colonisation, as in the present invention we show that mycorrhizal colonisation reduces strigolactone formation (Example 3). Alternatively, the negative effect of phosphate on orobanchol production as reported by Yoneyama (personal communication) may be exploited in subtractive and/or transcriptomics approaches to pinpoint genes that are down-regulated by high phosphate and/or up-regulated by low phosphate and that fit into the herein presented biosynthetic scheme. The first cytochrome P450 encoding gene from this pathway may be cloned based on the homology expected to exist with *MAX1*, a cytochrome P450 from Arabidopsis and oxidising the carotenoid cleavage product of a CCD (CCD7/MAX3) (Booker et al., 2005, Developmental Cell 8, 443-449; Booker et al., 2004, Current Biology 14, 1232-1238). Cloning can also be achieved using enzyme purification and de novo sequencing, nucleic acid hybridization methods, degenerate primer PCR approaches or using genomics approaches such as cDNA micro-array analysis metabolomics and proteomics. Successful inhibition after transformation could be easily identified using the germination assays described in Examples 1, 3 and 7.

Thus, a method for identifying an enzyme involved in carotenoid catabolism to strigolactones (and cloning the gene encoding it and verifying its function) is disclosed, comprising the steps of
a) identifying a gene which is downregulated by mycorrhizal colonization and /or by high phosphate and/or which is up-regulated by low phosphate (phosphate limitation); and
b) transforming a plant, plant cell or plant tissue with the nucleotide sequence operably linked to a promoter active in plants (i.e. with an expression or silencing construct) and obtaining a transformed plant thereform; and optionally
c) testing the germination of Orobanche and/or Striga seeds in the presence of roots, root exudates or root extracts of the recombinant plant (e.g. using the bioassay described); and optionally
d) selecting a plant which results in lower or higher seed germination compared to a control plant or selecting the gene whose downregulation/silencing in the recombinant plant results in significantly lower or whose overexpression results in significantly higher seed germination compared to the control plant for further use, e.g. for generating a transgenic plant or for identifying herbicides or compounds which specifically inhibit the activity of the enzyme encoded by the gene.

The above mentioned known or still to be obtained target gene sequences can also be used in TILLING approaches (Targeting Induced Local Lesions IN Genomics; McCallum et al., 2000, Nat Biotech 18:455, and McCallum et al. 2000, Plant Physiol. 123, 439-442, both incorporated herein by reference), where in induced-mutant collections of plants or plant tissues (e.g. seeds), are screened for mutations in target genes using PCR-based methods. Such an induced-mutant collection can also be used to screen more untargeted for mutants affected in any of the genes described above, and the genes can then be cloned. Also transposon-knock out mutant collections provide a suitable source to look for mutants affected in any of the target genes. Because of the transposon, the affected genes can easily be cloned. Plants can be grown in a high-throughput manner, and root exudates collected after 10 days and tested for induction of germination (see Example 1) or under greenhouse or field conditions on soil infested with parasitic plant seeds. These mutants can then be characterised and the mutated genes cloned and used to knock out germination stimulant formation in crop species. The selection of natural (spontaneous) mutants in crop species affected in carotenoid formation or cleavage in their roots, whereby the plant roots produce either enhanced or reduced amounts of one or more germination stimulants is disclosed. Such plants can be obtained by screening a plurality of plants, e.g. germbank collection, for modified expression of target genes and/or modified germination stimulant levels using e.g. the germination bioassays described in the present invention (Examples 1, 3 and 7).

As an alternative to knocking out enzymes from the germination stimulant pathway, overexpression of key-enzymes of competing pathways to channel away substrate can be considered as a strategy to reduce germination stimulant formation. Also, the strong inhibiting effect of externally applied ABA can be used as a control strategy. Alternative production pathways for ABA exist in micro-organisms (Siewers et al., 2004, Appl. Environ. Microbiol, 70, 3868-3876). There, ABA biosynthesis proceeds from farnesyl diphosphate and not geranylgeranyl diphosphate. Such microbial ABA biosynthesis is introduced into parasitic plant hosts, preferably with root-specific expression and preferably under the control of gene promoters involved in normal ABA-biosynthesis. This will lead to the formation of ABA and subsequent feedback inhibition of the endogenous carotenoid/ABA biosynthetic pathway as demonstrated in Figure 3 and Table 1, and hence to a reduction in germination stimulant formation. This could be combined with knocking out carotenoid/ABA biosynthesis in roots, and hence to an even more efficient reduction in germination stimulant formation.

Alternatively, ABA catabolism in roots could be blocked, also leading to feed-back inhibition of the carotenoid/ABA pathway. Enzymes responsible for catabolism of ABA are known, for example the cytochrome P450 enzyme ABA 8'-hydroxylase, but also other catabolic pathways exist, such as 4'-reduction, 7'-hydroxylation and conjugation (Cutler and Krochko, 1999). These catabolic enzymes are knocked-out preferably with root-specific expression. This will inhibit the catabolism of ABA and hence will lead to feedback inhibition of the endogenous carotenoid/ABA biosynthetic pathway as demonstrated in Figure 3 and Table 1, and hence to a reduction in germination stimulant formation.

Chemicals that inhibit the activity of any of the target enzymes are used to block germination stimulant formation. Examples of this are - but are not limited to - fluridone, norflurazone, isoxaflutole, flurtamone, clomazone, flurochloridone, pyridazinone, nicotinanilide, amitrole, naproxen or abamine, or cheap analogs of this, inhibitors of P450s or cheap analogs of ABA that very effectively blocks germination stimulant formation (Figure 3). We show that sublethal concentrations of fluridone - applied to the soil or sprayed on the leafs of the host plant, can be used to reduce germination stimulant formation and hence infection with parasitic weeds (Example 3).

To make the resistance mechanism based on reduced germination stimulant formation durable it can be combined with other resistance mechanisms. For example, a low production of germination stimulants is combined with a parasite-inducible toxin such as the parasite-inducible promoter-sarcotoxin IA (WO 02/094008). The transgenic tobacco plants described in that invention are protected against Orobanche infection but only to a limited level. In combination with the present invention a more solid and durable resistance may be obtained, not only in tobacco but in all plant species that are parasitised by Orobanche and Striga spp.

Resistance levels of recombinant plants compared to controls can be tested in *in vitro* assays, green-house bioassays or preferably in field trials with a high parasitic plant pressure. "Resistance" refers herein to both a significant reduction in damage caused when exposed to the parasitic plant, as well as a complete absence of damage. Resistant plants have the advantage that less or no chemicals need to be used to protect the crop plants. Recombinant or mutant plants according to the invention produce at least less germination stimulants and have enhanced resistance to one or more parasite species compared to control plants. Especially, enhanced resistance against S. *hermonthica*, S. gesneroides, S. *asiatica,* S. aspera, S. *forbesii, O*. *ramosa, O*. *crenata, O*. cernua, *O*. cumana and *O*. *aegyptiaca* are provided.

The fact that plants respond upon mycorrhization with reduced germination stimulant formation can be used to control *Orobanche* and Striga spp.. Most agricultural plants form arbuscular mycorrhizas, a beneficial relationship between plant roots and certain root-inhabiting fungi. Interestingly, two groups have reported that mycorrhiza can reduce Striga infection of sorghum and maize (Gworgwor and Weber, 2003, supra; Lendzemo, 2004, PhD thesis, supra; Lendzemo and Kuyper, 2001, supra). However, it was not known that mycorrhiza reduce the formation of germination stimulants. Also, it has been shown that micro-organisms may produce ABA and this has also been hypothesised to be true for mycorrhiza (Cutler and Krochko, 1999, Trends Plant Sci, 4, 472-478; Hartung and Gimmler, 1994, Prog Bot, 55, 157-173). In the present invention it was found that mycorrhizal colonisation reduces the production of germination stimulants (Example 4). This relationship now enables the inventors to explain and therefore also exploit the positive effect of mycorrhiza on parasitic plant infection. For example, much simpler and more efficient bioassays are provided herein, based on the finding that mycorrhiza reduce the formation of germination stimulants in the roots. Such bioassays were not possible before.

Without limiting the scope of the invention, a possible explanation is that due to the formation of mycorrhiza-specific apocarotenoids or ABA the formation of the *Striga* germination stimulant is reduced. Apocarotenoid formation (probably from ß-carotene) may be competing for substrate with germination stimulant formation. Alternatively, mycorrhizal colonisation may be down-regulating the strigolactone production pathway. Interestingly, the apocarotenoids have been shown to be produced upon mycorrhization in a number of *Orobanche* hosts as well, such as Medicago *truncatula,* tomato and tobacco (Fester et al., 2002a, supra; Fester et al., 2002b, supra; Strack et al., 2003, supra; Walter et al., 2000, supra). In the present invention evidence is provided that also the germination stimulants for *Orobanche* spp. are derived from the carotenoid/ABA pathway. This is for example clear from the identification of strigol-analogs in tomato (Yoneyama et al., 2004, supra) and the present results with *O*. *crenata* (see above and Figure 3). In one embodiment of the invention this knowledge is used to optimise this positive interaction between mycorrhizae and parasitic weed hosts by screening for successful host-mycorrhizal species combinations in which maximal reduction in germination stimulant formation is obtained using the bioassays described in the present invention (e.g. Example 4).

The fact that in for example tomato the germination stimulants are carotenoid-derived and that tomato is a host for mycorrhizae can now be used to investigate whether selective mycorrhization of tomato could be used as a control measure and to select suitable mycorrhizae-host combinations. This will also hold for many other crop species that can be colonised by mycorrhizae and are hosts of *Orobanche* spp., such as sunflower, and crop species from the Fabaceae and Solanaceae (such as tobacco and potato) (Fester et al., 2002b, supra).

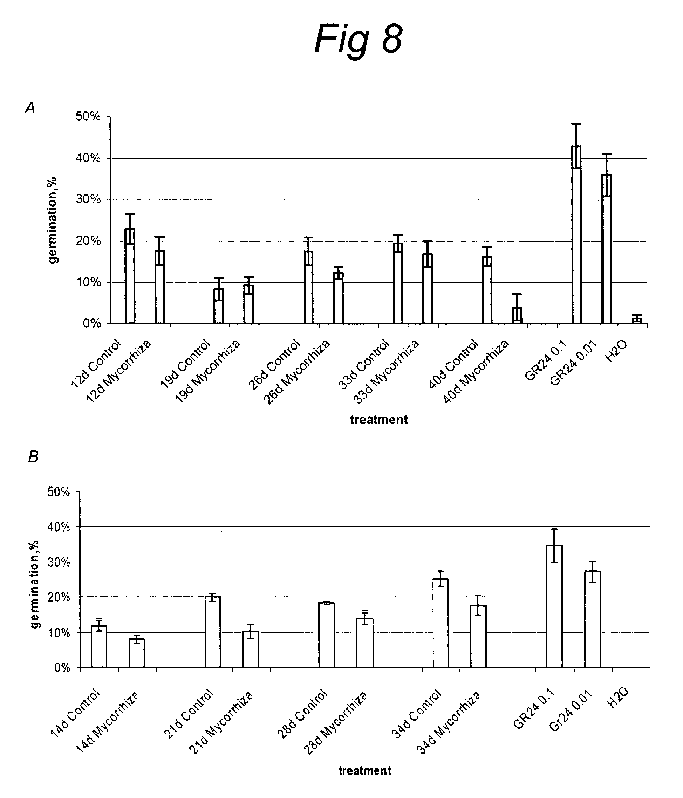

Thus, a method for identifying and selecting a mycorrhiza - parasitic weed host plant combination, which results in a significant reduction of strigolactone germination stimulants in the mycorrhiza colonized plant is provided, comprising the steps of a) inoculating a plurality of parasitic weed host species and/or varieties with one or more mycorrhizal species and b) testing the germination of Orobanche and/or Striga seeds in the presence of roots, root exudates, or root extracts from the mycorrhiza-colonized plants (i.e. using a germination bioassay as described elsewhere herein) and c) identifying and optionally selecting the mycorrhiza - parasitic weed host plant combination which results in significantly lower seed germination compared to a suitable control, such as the plant lacking mycorrhiza or a plant-mycorrhiza combination inducing a specific percentage of parasitic seed germination. The method above is, thus, also a method for selecting plant host-mycorrhizae species combinations having enhanced resistance to one or more Orobanche and/or Striga species. Also provided is the use of mycorrhizae species for reducing germination stimulant production of an Orobanche and/or Striga host species and, thus, for enhancing resistance of the host plant to one or more Orobanche and/or Striga species. The host species or varieties may be any species which is a host of one or more parasitic weed species. In the bioassay, a reduction in the percentage of seed germination of at least 5%, 10%, 20%, 30%, 40%, 50%, or more compared to the control is a significant reduction. See Figure 8 and Example 4.

Herein, recombinant plant cells, tissues and organs overexpression one or more target genes are disclosed (see further below). This can be done by transforming so-called catch and trap crop species, i.e. crops that are removed after they have induced a lot of parasitic plant germination (catch crops) or crops that do induce germination but are no host to the parasite that is present. However, also in host crops, a very high germination stimulant production may be beneficial, if this induces germination (only) at sufficient distance from the root, so that the germinating radicle can not reach the host root. In addition, overexpression of strigolactone formation may improve mycorrhizal colonisation efficiency.

Also provided are nucleic acid sequences (genomic DNA, cDNA, RNA) encoding target genes (including any chimeric or hybrid genes). Due to the degeneracy of the genetic code various nucleic acid sequences may encode the same amino acid sequence, e.g. various nucleic acid sequences encode the proteins depicted in Seq Id No's 2, 4, 6, 8, 10, 12, 14, 16, 18, 20, 22, 24, 26, 28, 30, 32, 34, 36, 38, 40, 42, 44, 46, 48 and 50. SEQ ID NO 1 depicts the Zea *mays* phytoene synthase 1 cDNA. SEQ ID NO 2 depicts the Zea *mays* phytoene synthase 1 protein. SEQ ID NO 3 depicts the Zea *mays* phytoene desaturase cDNA. SEQ ID NO 4 depicts the Zea *mays* phytoene desaturase protein. SEQ ID NO 5 depicts the Zea *mays* ζ-carotene desaturase cDNA. SEQ ID NO 6 depicts the Zea *mays* ζ-carotene desaturase protein. SEQ ID NO 7 depicts the Lycopersicon *esculentum* carotene isomerase cDNA. SEQ ID NO 8 depicts the Lycopersicon *esculentum* carotene isomerase protein. SEQ ID NO 9 depicts the *Zea mays* lycopene-β-cyclase cDNA. SEQ ID NO 10 depicts the Zea *mays* lycopene-β-cyclase protein. SEQ ID NO 11 depicts the Oryza sativa β-carotene hydroxylase cDNA. SEQ ID NO 12 depicts the *Oryza* sativa β-carotene hydroxylase protein. SEQ ID NO 13 depicts the *Oryza* sativa zeaxanthin epoxidase cDNA. SEQ ID NO 14 depicts the *Oryza sativa* zeaxanthin epoxidase protein. SEQ ID NO 15 depicts the Solanum *tuberosum* neoxanthin synthase cDNA. SEQ ID NO 16 depicts the Solanum tuberosum neoxanthin synthase protein. SEQ ID NO 17 depicts the Zea *mays* vp14 = 9-cis-epoxycarotenoid dioxygenase (NCED) cDNA. SEQ ID NO 18 depicts the Zea *mays* vp14 = 9-cis-epoxycarotenoid dioxygenase (NCED) protein. SEQ ID NO 19 depicts the Zea *mays* carotenoid cleavage dioxygenase 1 cDNA (CCD1). SEQ ID NO 20 depicts the Zea *mays* carotenoid cleavage dioxygenase 1 protein (CCD1). SEQ ID NO 21: *Oryza* sativa 9-cis-epoxycarotenoid dioxygenase (NCED-3) cDNA. SEQ ID NO 22 depicts the *Oryza* sativa 9-cis-epoxycarotenoid dioxygenase (NCED-3) protein. SEQ ID NO 23 depicts the *Oryza* sativa 9-cis-epoxycarotenoid dioxygenase (NCED-5) cDNA. SEQ ID NO 24 depicts the Oryza sativa 9-cis-epoxycarotenoid dioxygenase (NCED-5) protein. SEQ ID NO 25 depicts the *Oryza sativa* 9-cis-epoxycarotenoid dioxygenase (NCED-4) cDNA. SEQ ID NO 26 depicts the Oryza *saliva* 9-cis-epoxycarotenoid dioxygenase (NCED-4) protein. SEQ ID NO 27 depicts the *Oryza* sativa 9-cis-epoxycarotenoid dioxygenase (NCED-1) cDNA. SEQ ID NO 28 depicts the Oryza sativa 9-cis-epoxycarotenoid dioxygenase (NCED-1) protein. SEQ ID NO 29 depicts the Oryza sativa 9-cis-epoxycarotenoid dioxygenase (NCED-2) cDNA. SEQ ID NO 30 depicts the *Oryza* sativa 9-cis-epoxycarotenoid dioxygenase (NCED-2) protein. SEQ ID NO 31 depicts the *Oryza sativa* 9-cis-epoxycarotenoid dioxygenase (NCED-like) cDNA. SEQ ID NO 32 depicts the *Oryza* sativa 9-cis-epoxycarotenoid dioxygenase (NCED-like) protein. SEQ ID NO 33 depicts the *Oryza* sativa lignostilbene 1 cDNA. SEQ ID NO 34 depicts the *Oryza* sativa lignostilbene 1 protein. SEQ ID NO 35 depicts the Oryza sativa carotenoid cleavage dioxygenase 8 (CCD8) cDNA. SEQ ID NO 36 depicts the Oryza sativa carotenoid cleavage dioxygenase 8 (CCD8) protein. SEQ ID NO 37 depicts the Oryza sativa neoxanthin 1 cleavage enzyme cDNA. SEQ ID NO 38 depicts the *Oryza* sativa neoxanthin 1 cleavage enzyme protein. SEQ ID NO 39 depicts the *Oryza* sativa neoxanthin 2 cleavage enzyme cDNA. SEQ ID NO 40 depicts the *Oryza* sativa neoxanthin 2 cleavage enzyme protein. SEQ ID NO 41 depicts the *Oryza* sativa lignostilbene 2 cDNA. SEQ ID NO 42 depicts the *Oryza* sativa lignostilbene 2 protein. SEQ ID NO 43 depicts the *Oryza* sativa crocetin cDNA. SEQ ID NO 44 depicts the *Oryza* sativa crocetin protein. SEQ ID NO 45 depicts the *Oryza* sativa dioxygenase cDNA. SEQ ID NO 46 depicts the Oryza sativa dioxygenase protein. SEQ ID NO 47 depicts the *Oryza* sativa carotenoid cleavage dioxygenase 7 (CCD7) cDNA. SEQ ID NO 48 depicts the *Oryza* sativa carotenoid cleavage dioxygenase 7 (CCD7) protein. SEQ ID NO 49 depicts the *Oryza* sativa carotenoid cleavage dioxygenase 1 (CCD1) cDNA. SEQ ID NO 50 depicts the *Oryza* sativa carotenoid cleavage dioxygenase 1 (CCD1) protein.

The nucleic acid sequences provided herein include naturally occurring, artificial or synthetic nucleic acid sequences. Included are also sequences generated from the provided sequences by e.g. gene shuffling methods as described in US 5,811,238, WO97/20078, US 6,180,406 and US 6,117,679, which encode target proteins comprising higher or modified catalytic activity and methods of using the nucleic acid sequences of the invention for generating such "evolved" sequences. It is understood that when sequences are depicted as DNA sequences while RNA is referred to, the actual base sequence of the RNA molecule is identical with the difference that thymine (T) is replace by uracil (U).

Also included are variants and fragments of any of the target gene nucleic acid sequences, such as nucleic acid sequences hybridizing to target gene nucleic acid sequences under stringent hybridization conditions as defined, and fragments of these. Variants of target gene nucleic acid sequences also include essentially similar nucleic acid sequences, which have a nucleotide sequence identity (see definitions) to any target nucleic acid sequence, especially to any one of SEQ ID NO: 1, 3, 5, 7, 9, 11, 13, 15, 17, 19, 21, 23, 25, 27, 29, 31, 33, 35, 37, 39, 41, 43, 45, 47 and 49, of at least 30%, 40%, 50%, 60%, 70%, 80%, 85%, 90%, 95%, 98%, 99% or more. Further, variants of target nucleic acid sequences also include nucleic acid sequence encoding any target protein according to the invention, especially any one of the proteins of SEQ ID NO: 2, 4, 6, 8, 10, 12, 14, 16, 18, 20, 22, 24, 26, 28, 30, 32, 34, 36, 38, 40, 42, 44, 46, 48 and 50, and variants of any of these proteins. Variants of target proteins (i.e. proteins essentially similar to any of the target proteins) are proteins having an amino acid sequence identity (see definitions) of at least 30%, 40%, 50%, 60%, 70%, 80%, 85%, 90%, 95%, 98%, 99% or more to the target protein, especially to any one of the proteins of SEQ ID NO: 2, 4, 6, 8, 10, 12, 14, 16, 18, 20, 22, 24, 26, 28, 30, 32, 34, 36, 38, 40, 42, 44, 46, 48 and 50.

It is clear that many methods can be used to identify, synthesise or isolate variants or fragments of target gene nucleic acid sequences, such as nucleic acid hybridization, PCR technology, *in silico* analysis and nucleic acid synthesis, and the like.

The codon usage may be adapted to plant genes native to the plant genus or species of interest (Bennetzen & Hall, 1982, J. Biol. Chem. 257, 3026-3031; Itakura et al., 1977 Science 198, 1056-1063) using available codon usage tables (e. g. more adapted towards expression in cotton, soybean corn or rice). Codon usage tables for various plant species are published for example by Ikemura (1993, In "Plant Molecular Biology Labfax", Croy, ed., Bios Scientific Publishers Ltd.) and Nakamura et al. (2000, Nucl. Acids Res. 28, 292.) and for various organisms in the major DNA sequence databases (e.g. EMBL at Heidelberg, Germany). Accordingly, synthetic DNA sequences can be constructed so that the same or substantially the same proteins are produced. Several techniques for modifying the codon usage to that preferred by the host cells can be found in patent and scientific literature. The exact method of codon usage modification is not critical for this invention. Likewise, codon usage of a monocot derived target gene may be (partially) adapted to a dicot preferred codon usage for expression in dicots, and vice versa (see Batard et al. 2000, Arch Biochem Biophys 379, 161-169).

Small modifications to a DNA sequence such as described above can be routinely made, i.e., by PCR-mediated mutagenesis (Ho et al., 1989, Gene 77, 51-59., White et al., 1989, Trends in Genet. 5, 185-189). More profound modifications to a DNA sequence can be routinely done by de novo DNA synthesis of a desired coding region using available techniques.

Also, the target gene nucleic acid sequences can be modified so that the N-terminus of the proteins has an optimum translation initiation context, by adding or deleting one or more amino acids at the N-terminal end of the protein. Often it is preferred that the proteins of the invention to be expressed in plants cells start with a Met-Asp or Met-Ala dipeptide for optimal translation initiation. An Asp or Ala codon may thus be inserted following the existing Met, or the second codon can be replaced by a codon for Asp (GAT or GAC) or Ala (GCT, GCC, GCA or GCG). The DNA sequences may also be modified to remove illegitimate splice sites.

### Target gene silencing methods and recombinant plants

It is an object of the invention to provide a method for making a recombinant plant having one or more tissues (especially roots) which produce reduced amounts of one or more strigolactone germination stimulants compared to controls. This method may comprise the steps of:
(a) generating a gene silencing vector comprising a promoter sequence operably linked to a sense and/or antisense nucleic acid sequence of a carotenoid pathway gene (target gene),
(b) transforming a plant or plant cell with the vector of step a), and
(c) regenerating a plant comprising at least one tissue which produces (significantly) reduced amounts of strigolactone germination stimulants compared to control plants.

Preferably, the root tissue or part of the root tissue produces (significantly) reduced amounts of the target enzyme (or family) and thus also of one or more strigolactone germination stimulants.

The present invention provides methods and use according to claims 1-6.

Also disclosed is a recombinant plant, plant cell or tissue comprising, integrated in its genome, a sense and/or antisense fragment of a nucleic acid sequence encoding a a carotenoid pathway enzyme operably linked to a promoter and optionally a 3' non-translated nucleic acid sequence comprising a polyadenylation signal, whereby the recombinant plant, plant cell or tissue produces (significantly) reduced amounts of one or more strigolactone germination stimulants compared to a control plant, plant cell or tissue.

Preferably, if the target gene is a gene family, the whole gene family is silenced. Effective silencing can be analysed by for example determining the mRNA and/or protein levels of the target gene being produced. Silencing results in plants and/or tissues which produce significantly reduced amounts of the target-gene-derived product, the strigolactones. Any plant that is parasitised by *Orobanche* or *Striga* spp. may be a suitable host for transformation in order to silence germination stimulant formation. Suitable host plants are for example monocotyledonous plants or dicotyledonous plants, for example maize/corn (Zea species), pearl millet *(Pennisetum* spp. e.g. P. *glaucum),* rice *(Oryza* species, e.g. *O*. *sativa indica* cultivar-group or *japonica* cultivar-group), sorghum *(Sorghum bicolor),* soybean *(Glycine* spp, e.g. G. *max),* sunflower *(Helianthus annuus), Brassica* spp. (e.g. *B. napus, B. juncea, B. oleracea, B. rapa,* etc), tobacco (*Nicotiana* species), alfalfa *(Medicago sativa),* forage grasses, vegetable species, such as tomato *(Lycopersicon* ssp e.g. *Lycopersicon esculentum),* potato *(Solanum tuberosum,* other *Solanum* species), eggplant *(Solanum melongena),* peppers *(Capsicum annuum, Capsicum frutescens),* beans (e.g. *Phaseolus* species), zucchini, cucumber, melon, squash, artichoke, asparagus, broccoli, garlic, leek, lettuce, onion, radish, turnip, Brussels sprouts, carrot, cauliflower, chicory, celery, spinach, endive, fennel, beet, parsnip, herbs (mint, parsley, basil, thyme, etc.), other legumes such as pea, vetch, broadbean, faba bean, lentil, chickpea, cowpea, groundnut, bambara groundnut, fibre species e.g. flax *(Linum usitatissimum)* and hemp *(Cannabis sativa).*

Plant species transformed with a gene silencing construct under control of a suitable promoter, such as constitutive or a root specific promoter induce significantly lower germination of parasitic weed seeds due to a significant reduction (or complete absence) of strigolactones. A "significant reduction" (or "significantly reduced") refers herein to a reduction of at least 5%, 10%, 20%, 30%, 40%, 50%, 70%, 80%, 90%, 95% or 100% less strigolactone(s) being produced, than produced by the control plant/cell/tissue. It is understood that endogenous expression of other enzymes involved in strigolactone biosynthesis or catabolism may additionally be silenced using an appropriate gene silencing construct, for example endogenous P450s may be silenced. The reduction in strigolactone production is easily assayed by, for example, using a germination bioassay, as described in the Examples. This assay is an indirect assay which however, due to its sensitivity, provides a good indication of the amounts of germination inhibitors being produced. Thus, the percentage of germination of the parasitic seeds is preferably at least 5%, 10%, 20%, 30%, 40%, 50%, 70%, 80%, 90%, 95% or 100% less in recombinant cells, tissues or plants, than in control plant/cell/tissue. Alternatively, biochemical or molecular assays may be used.

"Gene silencing" refers to the down-regulation or complete inhibition of gene expression of one or more target genes. The use of inhibitory RNA to reduce or abolish gene expression is well established in the art and is the subject of several reviews (e.g Baulcombe, 1996, Plant Cell 8: 1833-1844, Stam et al. , 1997, Ann. Botan. 79:3-12, Depicker and Van Montagu, 1997, Curr. Opin. Cell. Biol. 9: 373-382). There are a number of technologies available to achieve gene silencing in plants, such as chimeric genes which produce antisense RNA of all or part of the target gene (see e.g. EP 0140308 B1, EP 0240208 B1 and EP 0223399 B1), or which produce sense RNA (also referred to as co-suppression), see EP 0465572 B1.

The most successful approach so far has however been the production of both sense and antisense RNA of the target gene ("inverted repeats"), which forms double stranded RNA (dsRNA) in the cell and silences the target gene. Methods and vectors for dsRNA production and gene silencing have been described in EP 1068311, EP 983370 A1, EP 1042462 A1, EP 1071762 A1 and EP 1080208 A1. A vector may therefore comprise a transcription regulatory region which is active in plant cells operably linked to a sense and/or antisense DNA fragment of a target gene according to the invention. Generally short (sense and antisense) stretches of the target gene sequence, such as 17, 18, 19, 20, 21, 22 or 23 nucleotides of coding or non-coding sequence are sufficient. Longer sequences can also be used, such as about 100, 200, 250, 300, 500 nucleotides or more. Thus, sense and/or antisense fragments of these lengths of any of the target nucleic acids are provided. Preferably, the short sense and antisense fragments are separated by a spacer sequence, such as an intron, which forms a loop (or hairpin) upon dsRNA formation. Any short stretch of a target gene, especially of SEQ ID NO: 1, 3, 5, 7, 9, 11,13, 15, 17, 19, 21, 23, 25, 27, 29, 31, 33, 35, 37, 39, 41, 43, 45, 47 and 49 or of any nucleic acid sequence encoding SEQ ID NO: 2, 4, 6, 8, 10, 12, 14, 16, 18, 20, 22, 24, 26, 28, 30, 32, 34, 36, 38, 40, 42, 44, 46, 48 and 50 (or any sequence essentially similar to these sequences) may be used to make a target gene silencing vector and a transgenic plant in which one or more target genes are silenced in all or some tissues or organs. A convenient way of generating hairpin constructs is to use generic vectors such as pHANNIBAL and pHELLSGATE, vectors based on the Gateway® technology (see Wesley et al. 2004, Methods Mol Biol. 265:117-30; Wesley et al. 2003, Methods Mol Biol. 236:273-86 and Helliwell & Waterhouse 2003, Methods 30(4):289-95.).

By choosing conserved nucleic acid parts of the target genes, target gene family members in a host plant can be silenced. Encompassed herein are also transgenic plants comprising a transcription regulatory element operably linked to a sense and/or antisense DNA fragment of a target gene and exhibiting a target gene silencing phenotype (i.e. reduced germination stimulant(s) being produced). Gene silencing constructs may also be used in reverse genetic approaches, to elucidate or confirm the function of a target gene or gene family in a host species.

The construction of chimeric genes and vectors for, preferably stable, introduction of the nucleic acid sequences into the genome of host cells is generally known in the art. To generate a chimeric gene the sense and/or antisense nucleic acid sequence (or, in overexpression approaches, the nucleic acid sequence encoding a target protein) according to the invention is operably linked to a promoter sequence, suitable for expression in the host cells, using standard molecular biology techniques. The promoter sequence may already be present in a vector so that the target gene nucleic acid sequence is simply inserted into the vector downstream of the promoter sequence. The vector is then used to transform the host cells and the chimeric gene is inserted in the nuclear genome or into the plastid, mitochondrial or chloroplast genome and expressed there using a suitable promoter (e. g., Mc Bride et al., 1995 Bio/Technology 13, 362; US 5,693, 507). In one embodiment a chimeric gene comprises a suitable promoter for expression in plant cells, operably linked thereto a nucleic acid sequence suitable for silencing the target gene(s) or encoding a functional target protein according to the invention (overexpression, see below), optionally followed by a 3'nontranslated nucleic acid sequence.

The target gene nucleic acid sequence, preferably the target chimeric gene, comprising the sense and/or antisense nucleic acid sequence (or encoding a functional target protein in overexpression approaches as described below), can be stably inserted in a conventional manner into the nuclear genome of a single plant cell, and the so-transformed plant cell can be used in a conventional manner to produce a transformed plant that has an altered phenotype due to the presence of the gene silencing nucleic acid sequence (or target protein) in certain cells at a certain time. In this regard, a T-DNA vector, comprising a nucleic acid sequence encoding a target protein, in *Agrobacterium tumefaciens* can be used to transform the plant cell, and thereafter, a transformed plant can be regenerated from the transformed plant cell using the procedures described, for example, in EP 0 116 718, EP 0 270 822, PCT publication WO84/02913 and published European Patent application EP 0 242 246 and in Gould et al. (1991, Plant Physiol. 95,426-434). The construction of a T-DNA vector for Agrobacterium mediated plant transformation is well known in the art. The T-DNA vector may be either a binary vector as described in EP 0 120 561 and EP 0 120 515 or a co-integrate vector which can integrate into the Agrobacterium Ti-plasmid by homologous recombination, as described in EP 0 116 718.

Preferred T-DNA vectors each contain a promoter operably linked to the target gene (sense and/or antisense) nucleic acid sequence between T-DNA border sequences, or at least located to the left of the right border sequence. Border sequences are described in Gielen et al. (1984, EMBO J 3,835-845). Of course, other types of vectors can be used to transform the plant cell, using procedures such as direct gene transfer (as described, for example in EP 0 223 247), pollen mediated transformation (as described, for example in EP 0 270 356 and WO85/01856), protoplast transformation as, for example, described in US 4,684, 611, plant RNA virus- mediated transformation (as described, for example in EP 0 067 553 and US 4,407, 956), liposome-mediated transformation (as described, for example in US 4,536, 475), and other methods such as those described methods for transforming certain lines of corn (e. g., US 6,140, 553; Fromm et al., 1990, Bio/Technology 8, 833-839; Gordon-Kamm et al., 1990, The Plant Cell 2, 603-618) and rice (Shimamoto et al., 1989, Nature 338, 274-276; Datta et al. 1990, Bio/Technology 8, 736-740) and the method for transforming monocots generally (PCT publication WO92/09696). The most widely used transformation method for dicot species is *Agrobacterium* mediated transformation. For cotton transformation see also WO 00/71733. *Brassica* species (e.g. cabbage species, broccoli, cauliflower, rapeseed etc.) can for example be transformed as described in US5750871 and legume species as described in US 5565346. Musa species (e.g. banana) may be transformed as described in US5792935. *Agrobacterium*-mediated transformation of strawberry is described in Plant Science, 69, 79-94 (1990). Likewise, selection and regeneration of transformed plants from transformed cells is well known in the art. Obviously, for different species and even for different varieties or cultivars of a single species, protocols are specifically adapted for regenerating transformants at high frequency.

Besides transformation of the nuclear genome, also transformation of the plastid genome, preferably chloroplast genome, is included in the invention. One advantage of plastid genome transformation is that the risk of spread of the transgene(s) can be reduced. Plastid genome transformation can be carried out as known in the art, see e.g. Sidorov VA et al. 1999, Plant J.19: 209-216 or Lutz KA et al. 2004, Plant J. 37(6):906-13, US 6541682, US6515206, US6512162 or US6492578.

The target gene nucleic acid sequence is inserted in a plant cell genome so that the inserted sense and/or antisense sequence is downstream (i.e. 3') of, and under the control of, a promoter which can direct the expression in the plant cell. This is preferably accomplished by inserting the chimeric gene in the plant cell genome, particularly in the nuclear or plastid (e. g. chloroplast) genome.

Preferred promoters include: the strong constitutive 35S promoters or (double) enhanced 35S promoters (the "35S promoters") of the cauliflower mosaic virus (CaMV) of isolates CM **1841** (Gardner et al., 1981, Nucleic Acids Research 9, 2871-2887), CabbB-S (Franck et al., 1980, Cell 21, 285-294) and CabbB-JI (Hull and Howell, 1987, Virology 86,482-493); the 35S promoter described by Odell et al. (1985, Nature 313, 810-812) or in US5164316, promoters from the ubiquitin family (e.g. the maize ubiquitin promoter of Christensen et al., 1992, Plant Mol. Biol. 18,675-689, EP 0 342 926, see also Cornejo et al. 1993, Plant Mol.Biol. 23, 567-581), the gos2 promoter (de Pater et al., 1992 Plant J. 2, 834-844), the emu promoter (Last et al., 1990, Theor. Appl. Genet. 81,581-588), Arabidopsis actin promoters such as the promoter described by An et al. (1996, Plant J. 10, 107.), rice actin promoters such as the promoter described by Zhang et al.(1991, The Plant Cell 3, 1155-1165) and the promoter described in US 5,641,876 or the rice actin 2 promoter as described in WO070067; promoters of the Cassava vein mosaic virus (WO 97/48819, Verdaguer et al. 1998, Plant Mol. Biol. 37,1055-1067), the pPLEX series of promoters from Subterranean Clover Stunt Virus (WO 96/06932, particularly the S7 promoter), a alcohol dehydrogenase promoter, e.g., pAdh1S (GenBank accession numbers X04049, X00581), and the TR1' promoter and the TR2' promoter (the "TR1'promoter" and "TR2'promoter", respectively) which drive the expression of the 1' and 2' genes, respectively, of the T-DNA (Velten et al., 1984, EMBO J 3, 2723-2730), the Figwort Mosaic Virus promoter described in US6051753 and in EP426641, histone gene promoters, such as the Ph4a748 promoter from Arabidopsis (PMB 8: 179-191), or others.

Alternatively, a promoter can be utilized which is not constitutive but rather is specific for one or more tissues or organs of the plant (tissue preferred / tissue specific, including developmentally regulated promoters), for example root preferred whereby the CCD or NCED gene is expressed only in cells of the specific tissue(s) or organ(s) and/or only during a certain developmental stage, for example during the early stages of plant development when the plant is most vulnerable to parasite attack.

For root specific expression a promoter preferentially active in roots is described in WO00/29566. Another promoter for root preferential expression is the ZRP promoter (and modifications thereof) as described in US 5,633, 363. Other suitable root-specific promoters are MtPT1, expressed in roots and root hairs in red clover (pers. comm. Zengyu Wang, The Samuel Roberts Noble Foundation, Ardmore, USA), pHM62, pHM58, pHM72, pHM78 (pers. comm. Hiromi Higo, Plant Functional genomics Co., Ibaraki, Japan) and Catb delt9 (pers. comm. Masao Iwamoto, National Institute of Agrobiological Sciences, Ibaraki, Japan).

Another alternative is to use a promoter whose expression is inducible. The reduction of carotenoid and/or germination stimulant formation may thus only develop after induction of target gene expression, for example upon a change in temperature, wounding, chemical treatment (e.g. substrate-inducible) etc. Examples of inducible promoters are wound-inducible promoters, such as the MPI promoter described by Cordera et al. (1994, The Plant Journal 6, 141), which is induced by wounding (such as caused by insect or physical wounding), or the COMPTII promoter (WO0056897) or the promoter described in US6031151. Alternatively the promoter may be inducible by a chemical, such as dexamethasone as described by Aoyama and Chua (1997, Plant Journal 11: 605-612) and in US6063985 or by tetracycline (TOPFREE or TOP 10 promoter, see Gatz, 1997, Annu Rev Plant Physiol Plant Mol Biol. 48: 89-108 and Love et al. 2000, Plant J. 21: 579-88). Other inducible promoters are for example inducible by a change in temperature, such as the heat shock promoter described in US 5,447, 858, by anaerobic conditions (e.g. the maize ADH1S promoter), by light (US6455760), by pathogens (e.g. EP759085 or EP309862) or by senescence (SAG12 and SAG13, see US5689042). Obviously, there are a range of other promoters available.

The target gene sense and/or antisense sequence is inserted into the plant genome so that the sense and/or antisense sequence is upstream (i.e. 5') of suitable 3'end transcription regulation signals ("3'end") (i.e. transcript formation and polyadenylation signals). Polyadenylation and transcript formation signals include those of the CaMV 35S gene ("3' 35S"), the nopaline synthase gene ("3' nos") (Depicker et al., 1982 J. Molec. Appl. Genetics 1, 561-573.), the octopine synthase gene ("3'ocs") (Gielen et al., 1984, EMBO J 3, 835-845) and the T-DNA gene 7 ("3' gene 7") (Velten and Schell, 1985, Nucleic Acids Research 13, 6981-6998), which act as 3'-untranslated DNA sequences in transformed plant cells, and others.

Introduction of the T-DNA vector into Agrobacterium can be carried out using known methods, such as electroporation or triparental mating.

A target gene nucleic acid sequence can optionally be inserted in the plant genome as a hybrid gene sequence whereby the target gene sequence is linked in-frame to a (US 5,254, 799; Vaeck et al., 1987, Nature 328, 33-37) gene encoding a selectable or scorable marker, such as for example the neo (or nptII) gene (EP 0 242 236) encoding kanamycin resistance, so that the plant expresses a fusion protein which is easily detectable.

Preferably, for selection purposes but also for weed control options, the transgenic plants of the invention are also transformed with a DNA encoding a protein conferring resistance to herbicide, such as a broad-spectrum herbicide, for example herbicides based on glufosinate ammonium as active ingredient (e.g. Liberty^{®} or BASTA; resistance is conferred by the PAT or bar gene; see EP 0 242 236 and EP 0 242 246) or glyphosate (e.g. RoundUp^{®}; resistance is conferred by EPSPS genes, see e.g. EP0 508 909 and EP 0 507 698). Using herbicide resistance genes (or other genes conferring a desired phenotype) as selectable marker further has the advantage that the introduction of antibiotic resistance genes can be avoided. Alternatively, other selectable marker genes may be used, such as antibiotic resistance genes. As it is generally not accepted to retain antibiotic resistance genes in the transformed host plants, these genes can be removed again following selection of the transformants. Different technologies exist for removal of transgenes. One method to achieve removal is by flanking the chimeric gene with lox sites and, following selection, crossing the transformed plant with a CRE recombinase-expressing plant (see e.g. EP506763B1). Site specific recombination results in excision of the marker gene. Another site specific recombination systems is the FLP/FRT system described in EP686191 and US5527695. Site specific recombination systems such as CRE/LOX and FLP/FRT may also be used for gene stacking purposes. Further, one-component excision systems have been described, see e.g. WO9737012 or WO9500555).

When reference to "a transgenic plant cell" or "a recombinant plant cell" is made anywhere herein, this refers to a plant cell (or also a plant protoplast) as such in isolation or in tissue/cell culture, or to a plant cell (or protoplast) contained in a plant or in a differentiated organ or tissue, and these possibilities are specifically included herein. Hence, a reference to a plant cell in the description or claims is not meant to refer only to isolated cells in culture, but refers to any plant cell, wherever it may be located or in whatever type of plant tissue or organ it may be present. Also, parts removed from the recombinant plant, such as harvested fruit, seeds, cut flowers, pollen, etc. as well as cells derived from the recombinant cells, such as seeds derived from traditional breeding (crossing, selfing, etc.) which retain the chimeric target gene are specifically included.

### Overexpression of target genes and methods for making catch and trap crops

In principle, any plant that is parasitised by Orobanche or Striga spp. or could be used as a trap or catch crop may be a suitable host for overexpression. Target genes as defined above can be overexpressed using methods as described above, with the difference that the whole coding sequence (cDNA or genomic) of one or more target genes is used to make an expression vector, which is then used in the transformation method. Thus, in the above description reference to sense and/or antisense sequences is simply replaced by sense sequences encoding the target enzyme(s). A higher production of germination stimulants can be easily assessed using for example the germination bioassay provided (Example 1, 3 and 7). A higher production of the strigolactones may also improve mycorrhizal colonisation. Overexpression leads to (significantly) enhanced amounts of one or more germination stimulants being produced by the recombinant cells, which in turn leads to (significantly) enhanced germination of the parasitic seeds. "Significantly enhanced amount(s)" refers herein to an increase of at least 5%, 10%, 20%, 30%, 40%, 50%, 70%, 80%, 90%, 95% or 100% of one or more strigolactones being produced, than produced by the control plant/cell/tissue. The enhanced amount of strigolactone production is easily assayed by, for example, using a germination bioassay, as described in the Examples. This assay is an indirect assay which however, due to its sensitivity, provides a good indication of the amounts of germination inhibitors being produced. Thus, the percentage of germination of the parasitic seeds is preferably at least 5%, 10%, 20%, 30%, 40%, 50%, 70%, 80%, 90%, 95% or 100% higher in recombinant cells, tissues or plants, than in control plant/cell/tissue. Alternatively, biochemical or molecular assays may be used.

### Uses of the recombinant plants and plant host cells

The resulting transformed plant can be used in a conventional plant breeding scheme to produce more transformed plants with the same characteristics or to introduce the transgene into other varieties of the same or related plant species. Seeds, which are obtained from the transformed plants, preferably contain the chimeric target gene (resulting in overexpression or silencing) as a stable genomic insert, and optionally one or more other chimeric genes as described above. Preferred host plants are crop plants, i.e. plants cultivated by humans for food, feed or ornamental purposes.

### Spontaneous and induced mutant plants

As described herein above, it is also an object of the invention to provide a method for making non-recombinant plants which produce significantly reduced or significantly enhanced amounts of one or more germination stimulants. The method comprises selecting plants having one or more mutations in at least one carotenoid pathway gene (target gene) and producing significantly reduced levels or significantly enhanced levels of one or more germination stimulants. It is noted that when referring to a mutation in a gene, the mutation need not be in the coding sequence of the gene, but may for example be in the promoter region. Also, "mutation" refers to insertion, deletion and/or replacement of one or more nucleotides. The mutation may be in any gene coding for an enzyme involved in carotenoid biosynthesis or carotenoid catabolism to the strigolactone germination stimulants as defined in claim 5.

The selection may be carried out using various known methods, such as molecular or biochemical methods, followed by e.g. a germination bioassay. Optionally, selection of cells, tissues or plants may be preceded by mutagenesis, such as chemical mutagenesis (e.g. as done in TILLING), transposon mutagenesis, UV- or gamma- radiation treatment, etc.

### SEQUENCES

SEQ ID NO 1: Zea *mays* phytoene synthase 1 cDNA
SEQ ID NO 2: Zea *mays* phytoene synthase 1 protein
SEQ ID NO 3: Zea *mays* phytoene desaturase cDNA
SEQ ID NO 4: Zea *mays* phytoene desaturase protein
SEQ ID NO 5: Zea *mays* ζ-carotene desaturase cDNA
SEQ ID NO 6: Zea *mays* ζ-carotene desaturase protein
SEQ ID NO 7: *Lycopersicon esculentum* carotene isomerase cDNA
SEQ ID NO 8: *Lycopersicon esculentum* carotene isomerase protein
SEQ ID NO 9: Zea *mays* lycopene-β-cyclase cDNA
SEQ ID NO 10: Zea *mays* lycopene-β-cyclase protein
SEQ ID NO 11: *Oryza sativa* β-carotene hydroxylase cDNA
SEQ ID NO 12: *Oryza sativa* β-carotene hydroxylase protein
SEQ ID NO 13: *Oryza sativa* zeaxanthin epoxidase cDNA
SEQ ID NO 14: *Oryza sativa* zeaxanthin epoxidase protein
SEQ ID NO 15: *Solanum tuberosum* neoxanthin synthase cDNA
SEQ ID NO 16: *Solanum tuberosum* neoxanthin synthase protein
SEQ ID NO 17: Zea *mays* vp14 = 9-cis-epoxycarotenoid dioxygenase (NCED) cDNA
SEQ ID NO 18: Zea *mays* vp14 = 9-cis-epoxycarotenoid dioxygenase (NCED) protein
SEQ ID NO 19: Zea *mays* carotenoid cleavage dioxygenase 1 cDNA (CCD1)
SEQ ID NO 20: Zea *mays* carotenoid cleavage dioxygenase 1 protein (CCD1)
SEQ ID NO 21: *Oryza sativa* 9-cis-epoxycarotenoid dioxygenase (NCED-3) cDNA
SEQ ID NO 22: *Oryza sativa* 9-cis-epoxycarotenoid dioxygenase (NCED-3) protein
SEQ ID NO 23: *Oryza sativa* 9-cis-epoxycarotenoid dioxygenase (NCED-5) cDNA
SEQ ID NO 24: *Oryza sativa* 9-cis-epoxycarotenoid dioxygenase (NCED-5) protein
SEQ ID NO 25: *Oryza sativa* 9-cis-epoxycarotenoid dioxygenase (NCED-4) cDNA
SEQ ID NO 26: *Oryza sativa* 9-cis-epoxycarotenoid dioxygenase (NCED-4) protein
SEQ ID NO 27: *Oryza sativa* 9-cis-epoxycarotenoid dioxygenase (NCED-1) cDNA
SEQ ID NO 28: *Oryza sativa* 9-cis-epoxycarotenoid dioxygenase (NCED-1) protein
SEQ ID NO 29: *Oryza sativa* 9-cis-epoxycarotenoid dioxygenase (NCED-2) cDNA
SEQ ID NO 30: *Oryza sativa* 9-cis-epoxycarotenoid dioxygenase (NCED-2) protein
SEQ ID NO 31: *Oryza sativa* 9-cis-epoxycarotenoid dioxygenase (NCED-like) cDNA
SEQ ID NO 32: *Oryza sativa* 9-cis-epoxycarotenoid dioxygenase (NCED-like) protein
SEQ ID NO 33: *Oryza sativa* lignostilbene 1 cDNA
SEQ ID NO 34: *Oryza sativa* lignostilbene 1 protein
SEQ ID NO 35: *Oryza sativa* carotenoid cleavage dioxygenase 8 (CCD8) cDNA
SEQ ID NO 36: *Oryza sativa* carotenoid cleavage dioxygenase 8 (CCD8) protein
SEQ ID NO 37: *Oryza sativa* neoxanthin 1 cleavage enzyme cDNA
SEQ ID NO 38: *Oryza sativa* neoxanthin 1 cleavage enzyme protein
SEQ ID NO 39: *Oryza sativa* neoxanthin 2 cleavage enzyme cDNA
SEQ ID NO 40: *Oryza sativa* neoxanthin 2 cleavage enzyme protein
SEQ ID NO 41: *Oryza sativa* lignostilbene 2 cDNA
SEQ ID NO 42: *Oryza sativa* lignostilbene 2 protein
SEQ ID NO 43: *Oryza sativa* crocetin cDNA
SEQ ID NO 44: *Oryza sativa* crocetin protein
SEQ ID NO 45: *Oryza sativa* dioxygenase cDNA
SEQ ID NO 46: *Oryza sativa* dioxygenase protein
SEQ ID NO 47: *Oryza sativa* carotenoid cleavage dioxygenase 7 (CCD7) cDNA
SEQ ID NO 48: *Oryza sativa* carotenoid cleavage dioxygenase 7 (CCD7) protein
SEQ ID NO 49: *Oryza sativa* carotenoid cleavage dioxygenase 1 (CCD1) cDNA
SEQ ID NO 50: *Oryza sativa* carotenoid cleavage dioxygenase 1 (CCD1) protein

### FIGURE LEGENDS

Figure 1
   Structure of strigolactone germination stimulants. (a) (+)-strigol, (b) orobanchol, (c) sorgolactone, (d) synthetic germination stimulant GR24. A, B, C and D indicate the A-, B-, C- and D-ring.
Figure 2
   Carotenoid and abscisic acid biosynthetic pathway. Intermediates that are not shown are in square brackets. The 15-cis and 9,9'-cis between brackets for phytoene and ξ-carotene refer to new insights in carotene isomerisation during early steps of the pathway (Park et al., 2002; Isaacson et al., 2004). Carotenoid maize mutants (italics) and inhibitors (underlined) at different steps in the pathway that are described in the present invention are indicated with ⇐. Redrawn from (Cunningham and Gantt, 1998, Annu Rev Plant Physiol Plant Mol Biol, 49, 557-583; Hirschberg, 2001, supra) and (Seo and Koshiba, 2002, supra).
Figure 3
   Germination of S. *hermonthica* seeds induced by root exudates of maize (W22 or Dent, as indicated in the graphs) (A-D), cowpea (E) and sorghum (G) and germination of *O*. *crenata* induced by the root exudates of cowpea (F) as affected by treatment of the host seedlings.
   A, maize: 100 µM fosmidomycin (Fos), 25 µM fluridone (Flu), 10 µM mevastatin (Me), untreated control (C). B, maize: 10 µM fluridone (Flu) applied to plants growing under normal light (Flu) and dim light (FluD), untreated controls (C and CD) C, maize: 200 µM amitrole (Ami), untreated control (C). Seedlings were grown at 21°C. D, maize: untreated control (C), 10 µM fluridone (Flu), 0.1 mM naproxen (0.1 N), 1 mM naproxen (1 N), 0.1 mM sodium tungstate (0.1 ST), 1 mM sodium tungstate (1 mM ST), 0.02 mM abscisic acid plus 10 µM fluridone (0.02 ABA Flu), 0.02 mM abscisic acid (0.02 ABA), 0.2 mM abscisic acid plus 10 µM fluridone (0.2 ABA Flu), 0.2 mM abscisic acid (0.2 ABA). E, cowpea: 10 µM fluridone, nontreated control (C), 10 µM fluridone (Flu), root exudate of fluridone treated seedlings plus 0.01 mg.L⁻¹ GR24 (Flu GR 0.01), 0.01 and 0.1 mg.L⁻¹ GR24 alone (GR 0.01 and GR 0.1). F, cowpea (with *O*. *crenata):* 10 µM fluridone (Flu), nontreated control (C). G, sorghum: 4 µM fluridone (Flu), nontreated control (C).
   Within each experiment, the concentrations of root exudates were equalized, by dilution, for differences in root fresh weight. Data represent average germination ± standard error of 8 to 10 individual plants and 2 or 3 disks per plant (indicated in each diagram by 8/3 etc), with about 50-100 S. *hermonthica* or *O*. crenata seeds each. Statistical analysis showed significant differences (P<0.05) for all treatments, except the treatments with 100 µM fosmidomycin, 10 µM mevastatin and 0.1 mM naproxen.
Figure 4
   A, Phenotypes of mutant *y10* seedlings (white) and corresponding wild type siblings (grey) and germination of S. hermonthica induced by the root exudates of these seedlings. B, Phenotypes of mutant *cl1* 311AA seedlings (white) and corresponding wild type siblings (grey) and germination of S. hermonthica induced by root exudates of these seedlings. N=non-mutant phenotype, M=mutant phenotype. Bars indicate average germination ± standard error of 3 disks per plant with about 50-100 S. hermonthica seeds each.
Figure 5
   Average germination of S. hermonthica induced by root exudates of maize mutants *lw1, y10, al1y3, vp5, y9, cl1* **311AA** and *vp14* and their corresponding wild type siblings (the number of seedlings used for each bioassay are indicated between brackets). Statistical analysis showed that *lw1, y10, al1y3, vp5*, *y9* mutant phenotype induced significantly lower (P<0.05) germination than the corresponding non-mutant phenotype seedlings. The difference in germination induced by the *vp14* mutant was not significant (P=0.09). N=non-mutant phenotype, M=mutant phenotype. Bars indicate average germination ± standard error of 5 to 8 individual plants (indicated below the X-axis) and 3 disks per plant with about 50-100 S. *hermonthica* seeds each.
Figure 6
   Postulated biogenetic scheme for the formation of strigolactones.
Figure 7
   Effect of application of the inhibitor fluridone to rice roots (A) or leafs (B) on the germination/attachment of *Striga hermonthica* seeds. Data presented are the mean of six independent replicates.
Figure 8
   Effect of maize root exudates of roots colonized by *G. intraradices* on germination of *Striga hermonthica* seeds as induced by root exudates collected from these plants in two independent experiments (A and B). The concentrations of all root exudates were adjusted to refelect the same weight of roots per mL of exudates. Germination percentages are averages of exudates of 3 individual plants. Average colonization percentages of roots by mycorrhiza were: A: 10% (12d); 10% (19d); 52% (26d); 63% (33d); 57% (40d) and B: 11% (14d); 30% (21d); 28% (28d); 47% (34d). GR24 (0.1/0.01 mg.L⁻¹) was used as positive control, H₂O as negative control. Error bars indicate standard error.
Figure 9
   Diagram describing the RNAi constructs that are used for transformation of maize, rice and tomato.

**Table 1**

| Carotenoid composition of shoots and roots of 10-day old maize seedlings treated with inhibitors of abscisic acid biosynthesis or abscisic acid itself and carotenoid mutants *al1y3* and *cl1* 311AA. Carotenoid content in µg.g⁻¹ fwt; n.d. - below detection limit. | | | | |
|---|---|---|---|---|
| | neoxanthin | violaxanthin | lutein | ß-carotene |
| Shoots | | | | |
| control | 153.40 | 72.65 | 88.56 | 65.51 |
| 10 µM fluridone | n.d. | n.d. | n.d. | n.d. |
| 1 mM naproxen | 111.81 | 52.08 | 68.58 | 47.56 |
| 0.1 mM sodium tungstate | 147.58 | 72.19 | 83.94 | 64.29 |
| 0.02 mM abscisic acid | 115.29 | 50.84 | 73.01 | 47.35 |

| Roots | | | | |
|---|---|---|---|---|
| control | n.d. | 0.11 | n.d. | 0.02 |
| 10 µM fluridone | n.d. | n.d. | n.d. | n.d. |
| 1 mM naproxen | n.d. | 0.29 | n.d. | 0.04 |
| 0.1 mM sodium tungstate | n.d. | 0.22 | n.d. | 0.03 |
| 0.02 mM abscisic acid | n.d. | 0.07 | n.d. | 0.01 |

| Carotenoid mutants | | | | |
|---|---|---|---|---|
| Shoots | | | | |
| *Al1y3* | 32.43 | 31.06 | 55.72 | 46.47 |
| Mutant *al1y3* | 2.64 | 3.15 | 6.17 | 4.68 |
| *Cl1* 311AA | 28.06 | 26.77 | 50.95 | 36.44 |
| Mutant *cl1* 311AA | n.d. | 1.14 | 1.08 | n.d. |

| Roots | | | | |
|---|---|---|---|---|
| *Al1y3* | n.d. | n.d. | n.d. | n.d. |
| Mutant *al1y3* | n.d. | n.d. | n.d. | n.d. |
| *Cl1* 311AA | n.d. | 0.07 | n.d. | 0.01 |
| Mutant *cl1* 311AA | n.d. | 0.08 | n.d. | 0.01 |

The following non-limiting Examples illustrate the invention. Unless stated otherwise in the Examples, all recombinant DNA techniques are carried out according to standard protocols as described in Sambrook et al. (1989) Molecular Cloning: A Laboratory Manual, Second Edition, Cold Spring Harbor Laboratory Press, and Sambrook and Russell (2001) Molecular Cloning: A Laboratory Manual, Third Edition, Cold Spring Harbor Laboratory Press, NY; and in Volumes 1 and 2 of Ausubel et al. (1994) Current Protocols in Molecular Biology, Current Protocols, USA. Standard materials and methods for plant molecular work are described in Plant Molecular Biology Labfax (1993) by R.D.D. Croy, jointly published by BIOS Scientific Publications Ltd (UK) and Blackwell Scientific Publications, UK.

### EXAMPLES

### Example 1. General Material and Methods

### 1.1 Plant material and chemicals

Maize inbred W22 was obtained from Vicky Child, IACR, UK and inbred line Dent was obtained from JC Robinson Seeds, The Netherlands. Maize seeds deficient in chlorophyll and carotenoid biosynthesis were obtained from the Maize Genetics COOP Stock Center, Urbana, Illinois (mutants *lw1, y10, vp5, vp14, y9, al1-y3, cl1* 311AA). Cowpea *(Vigna* unguiculata (L.) Walp) seeds were obtained from Sériba Katilé, Institut d'Economie Rurale, Mali. Sorghum bicolor, variety CSH-1 was obtained from Bob Vasey, Sheffield University, UK. Striga *(Striga hermonthica* (Del.) Benth) seeds were collected from a maize field in **1994,** Kibos, Kenya and *Striga* hermonthica seeds used in germination bioassay with sorghum root exudates were collected from a sorghum field in Sudan in 1995 and were obtained from Bob Vasey. Orobanche crenata seeds were obtained from D. M. Joel, Newe-Ya'ar Research Center, Israel.

The following inhibitors of isoprenoid pathways were used: mevastatin (Sigma-Aldrich), fosmidomycin (Molecular Probes, Inc.), fluridone (Ducheva), amitrole (3-amino-1,2,4-triazole), naproxen (S)-6-methoxy-α-methyl-2-naphthaleneacetic acid sodium salt), sodium tungstate, dihydrate) and (±)-abscisic acid (all from Sigma-Aldrich). The synthetic germination stimulant strigolactone analogue GR24 was kindly provided by prof. B. Zwanenburg, University of Nijmegen, The Netherlands.

### 1.2 Root exudate collection

Seeds of maize were sterilized in 4% sodium hypochlorite containing 0.02% (v/v) Tween 20, rinsed thoroughly with sterile water and imbibed for 24 hrs on moistened filter paper at 25°C. Plants were grown in autoclaved perlite in separate tubes or in 1-L pots (ø 15 cm) at 25°C in a climate room with 16/8 hrs photoperiod at 28 µmol photons.m^{- 2}.sec⁻¹. To grow plants under dim light, plants were grown in a box covered by several layers of cheesecloth and aluminum foil with small holes (0.5 µmol photons. m⁻².sec⁻¹). The plants were watered with tap water (control, carotenoid mutants) or with solutions of abscisic acid or inhibitors. Depending on the vigor, mutants were grown for 8-11 days. After several days, depending on the experiment, seedlings were removed from perlite and carefully cleaned from perlite. Plants were put into about 8 mL tap water in glass tubes at 25°C to collect root exudates. Tubes were covered with aluminum foil to exclude light. After 6-24 hrs root exudates were collected and root fresh weight of each seedling was determined. Exudates were diluted to equal concentrations (mL of exudate per gram of roots) within each experiment and subsequently used in a bioassay. Seeds of cowpea were imbibed for 24 hrs on moistened filter paper at 25°C in darkness. Imbibed seeds were sown in pots containing a mixture of vermiculite and perlite (1:1). Plants were grown in a climate room with 16/8 hrs photoperiod at 28 µmol photons.m⁻².sec⁻¹ at 25°C. The plants were regularly watered for 4 days. Subsequently, seedlings were watered with water (control) or with 10 µM fluridone for 5 days. Root exudates were collected as described for maize. Seeds of sorghum were germinated for 3 days at 28°C in autoclaved vermiculate, watered with 40% modified Long Ashton nutrient solution (Gurney et al., 2002, Weed Res, 42, 317-324). Small seedlings were transferred to glass tubes to grow hydroponically in a phytotron 14/10 hrs photoperiod at 250 µmol photons.m⁻².sec⁻¹ at 28/23°C. 12 days old seedlings were transferred into clean tubes, containing 40% Long Ashton nutrient solution without/with 4 µM fluridone. 5 days later root exudates were collected during 24h as described for maize.

### 1.3 Germination bioassay

Root exudates produced by individual plants were tested by bioassay. The seeds of *Orobanche* and *Striga* spp. require preconditioning (or warm stratification) for a certain period of time at a suitable temperature before the seeds become responsive to germination stimulants (Matusova et al., 2004, Seed Sci Res, 14, 335-344). Preconditioning was performed under sterile conditions. The seeds were surface sterilized in 2% sodium hypochlorite containing 0.02% (v/v) Tween 20 for 5 min and rinsed thoroughly with sterile demineralized water. Subsequently the seeds were dried for 30 min in a laminar air flow cabinet. Approximately 50 - 100 seeds were spread on a glass fiber filter paper (GFFP) disk (Ø 9 mm) and put into sterile Petri dishes (Ø 9 cm) lined with Whatman filter paper wetted with 3 mL of demineralized water. Petri dishes were sealed with parafilm and incubated for preconditioning. S. *hermonthica* seeds were preconditioned at 30°C in darkness for 10 -12 days, *O*. crenata seeds were preconditioned at 21°C in darkness for **14** days. After the preconditioning period the GFFP disks with S. *hermonthica* or *O*. crenata seeds were removed from the Petri dish and dried for 20 min to remove surplus moisture. The disks were transferred to another Petri dish within a filter paper ring (outer Ø 9 cm, inner Ø 8 cm) wetted with 0.9 ml of H₂O, which maintained a moist environment during the germination bioassay. 50 µl of the test solutions were added to duplicate or triplicate disks, depending on the experiment. The synthetic germination stimulant GR24 (0.01 and 0.1 mg.L⁻¹ GR24) as a positive and water as a negative control were included in each bioassay. Seeds were incubated at 30°C *(S. hermonthica)* or 25°C (0. *crenata)* in darkness for 2 days (S. *hermonthica)* or 6 days (*O*. *crenata).* The germinated and non-germinated seeds were counted using a binocular. Seeds were considered germinated when the radicle protruded the seed coat. To test for an inhibitory effect of root exudates from fluridone-treated seedlings, a control combining root exudates of maize, cowpea or sorghum in combination with GR24 was included in the germination bioassays. Root exudates plus GR24 were diluted with water to the same final concentration of root exudate that was used without GR24. We used a non-saturating concentration of GR24 to ensure that any inhibitory (negative) effect of the root exudate of fluridone-treated cowpea seedlings would be visible. The Generalized Linear Mixed Model method was used for statistical analysis of all germination bioassays using Genstat, procedure IRREML (Payne and Lanne, 1993).

### Example 2. Elucidation of strigolactone formation in maize and cowpea

### 2.1 The use of inhibitors early in the pathway

W22 seedlings were grown in perlite in separate tubes for 3 days at 25°C. Tubes were covered with aluminum foil and the plants were watered with tap water. Then solutions of inhibitors were applied and seedlings were grown in the presence of inhibitors for an additional 5 days. 10 µM mevastatin and 100 µM fosmidomycin were used to inhibit isoprenoid formation in the cytosol and plastids, respectively and 25 µM fluridone was used to block carotenoid formation. After 5 days, seedlings were taken from the perlite and any perlite clinging to the roots was carefully removed. Plants were put into tap water in glass tubes for 6 hrs at 25°C. A dilution of 70 mg root fresh weight /mL root exudate was used for the bioassay. Root exudates from 10 individual plants for each treatment were tested. For amitrole experiments, germinated seeds of maize inbred line DENT were sown in perlite in pots and grown at 21°C for 11 days. Control plants were watered with tap water, amitrole-treated plants were watered with 200 µM amitrole. Root exudates were collected in tap water for 8 hrs. Naproxen and sodium tungstate treated maize inbred line DENT plants were sown in perlite in pots and grown at 25°C for 10 days. Control plants were watered with tap water, naproxen-treated plants were watered with 0.1 and 1 mM naproxen. Sodium tungstate-treated plants were watered with 0.1 and 1 mM solutions. Abscisic acid treated plants were watered with 0.02 and 0.2 mM abscisic acid without/with 10 µM fluridone. Root exudates from 8 individual seedlings/treatment were collected in tap water for 18 hrs.

Root exudates of control maize seedlings always induced germination of preconditioned S. hermonthica seeds regardless of lines and cultivars used. Water alone did not induce any germination of S. hermonthica seeds. Because the plants were grown in different experiments under different conditions, the maize-induced germination of striga seeds was analyzed within each experiment separately.

Mevastatin, an inhibitor of 3-hydroxy-3-methylglutaryl (HMG) CoA reductase, catalyzing the formation of mevalonic acid from HMG, was used to reduce cytosolic isoprenoid formation (Figure 2). Fosmidomycin, blocking 1-deoxy-D-xylulose 5-phosphate reductoisomerase was used to inhibit plastidic isoprenoid formation and fluridone was used to block carotenoid formation in the plastids (Figure 2). Inhibitors were applied from 3 days after germination when the root length was about 3-5 cm. Exudates from individual control plants of inbred line W22 induced a high and reproducible germination score (42 ± 2%) (Figure 3A). Mevastatin did not affect the morphology or appearance of the plants in contrast to fosmidomycin that induced slightly pale-yellow leaves. Mevastatin and fosmidomycin slightly but non-significantly reduced maize root exudate-induced S. *hermonthica* germination (37 ± 2% for fosmidomycin and 33 ± 3% for mevastatin-treated plants (Figure 3A). Leaves of maize seedlings grown in the presence of the carotenoid inhibitor fluridone were white with green tips showing the proportion of newly formed tissue that was affected by fluridone treatment. Germination induced by the root exudates of fluridone-treated maize was about 80% lower than for control maize (Figure 3A). Fluridone specifically inhibits the second dedicated enzyme in the carotenoid pathway, phytoene desaturase (PDS) (Li et al., 1996, supra) (Figure 2). The highly reduced root exudate-induced germination of *S*. *hermonthica* by fluridone-treated plants strongly suggests that the germination stimulant produced by maize is derived from the carotenoid pathway.

Germination of S. *hermonthica* is also induced by cowpea root exudates and a strigolactone germination stimulant of S. gesneroides, alectrol, has been identified in cowpea root exudates (Muller et al., 1992, supra). Therefore we assayed the effect of fluridone also on cowpea. The root exudates of non-treated cowpea induced 49±2% germination of *S. hermonthica* seeds compared with 11±3% by fluridone-treated cowpea root exudates (Figure 3E). To exclude the possibility that fluridone treatment leads to the formation of inhibitors of germination (rather than a lower production of the germination stimulant), the root exudates of fluridone-treated cowpea were also assessed in combinations with suboptimal concentrations of the synthetic germination stimulant GR24. The resulting germination of *S*. *hermonthica* induced by fluridone-treated cowpea root exudates with GR24 was slightly higher 53 ± 3% than germination induced by GR24 alone (48 ± 1%) (Figure 3E). These results show that the germination stimulant exuded from the roots of cowpea is also derived from the carotenoid pathway and that lower germination induced by fluridone-treated root exudates is caused by a lower concentration of germination stimulants in the root exudate and not by the production of inhibitors in fluridone-treated plants. In addition to *S*. *hermonthica,* cowpea root exudates also induced the germination of seeds of *O*. *crenata* (Figure 3F). Just like for *S. hermonthica,* germination of *O*. *crenata* seeds with fluridone-treated cowpea root exudate was lower than that induced by the control (Figure 3F) showing that also for *O*. *crenata* the germination stimulant(s) in cowpea root exudate is (are) carotenoid-derived.

Fluridone, through the inhibition of carotenoid formation in the leaves, also caused photodestruction of the chlorophyll. Therefore, in a next experiment, maize seedlings were grown under dim light in order to prevent photodestruction of chlorophyll while carotenoid biosynthesis was inhibited. Control plants grown under normal light were green and root exudates induced germination of S. *hermonthica* seeds (13 ± 1%) (Figure 3B). Fluridone-treated plants exhibited the typical white phenotype and induced virtually no germination. Control seedlings grown under dim light were pale-green but they induced similar germination of *S. hermonthica* seeds as control seedlings grown under normal light conditions. Under dim light conditions, fluridone-treated seedlings did not show the typical bleaching visible under normal light but exhibited a pale-green phenotype similar to control seedlings under dim light. Nevertheless germination of S. *hermonthica* seeds induced by root exudates of fluridone-treated seedlings was also negligible (Figure 3B). This demonstrates that the degradation/absence of chlorophyll itself is not causing the reduced germination induced by root exudates of maize in which carotenoid formation is disturbed. An additional possibility to discriminate between the direct effect of the absence of carotenoids and the indirect effect through destruction of chlorophyll is the use of chlorophyll mutants. The maize mutant *cl1* (*chlorophyll1*) (311AA) exhibits an albino phenotype and was first described by Everett (see Robertson, 1966) to be a chlorophyll mutant. Later, Robertson et al. (1966) described a group of *cl1* mutants as mutants that are impaired in both chlorophyll as well as carotenoid formation to different levels depending on a *clm* (gene modifier) (Robertson et al., 1966, Photochem Photobiol, 5, 797-805). Therefore we analyzed both the induction of germination by the *cl1* 311AA mutant as well as its carotenoid content. For carotenoid analysis, the shoots and roots of maize seedlings were frozen in liquid nitrogen and ground to a fine powder. Carotenoids were extracted from 1.5 g of roots and 0.5 g of shoots essentially as described before (Bino et al., New Phytologist, in press). HPLC analysis was performed according to (Fraser et al., 2000, Plant J, 24, 551-558). Identification of carotenoids was based on retention time and spectral characteristics of the standards.

Mutant phenotype siblings contained strongly reduced carotenoid levels in the shoots (Table 1). In the roots, there was no difference in the concentrations of violaxanthin and ß-carotene between wildtype and mutant phenotype siblings (Table 1). The root exudate of *cl1* 311AA seedlings induced normal germination, comparable to the wild-type phenotype seedlings (Fig 4B).

An additional interesting inhibitor of carotenoid biosynthesis is the bleaching herbicide amitrole that inhibits carotenoid biosynthesis by blocking lycopene cyclase in maize seedlings (Figure 2) (Dalla Vecchia et al., 2001, supra). Amitrole is only effective at a relatively low temperature (Dalla Vecchia et al., 2001, supra). When grown at 21°C, amitrole-treated inbred Dent plants had a yellow-green phenotype. Root exudates of amitrole-treated seedlings induced lower germination of S. *hermonthica* seeds than control seedlings that were also grown at 21°C (Figure 3C).

### 2.2 Using maize mutants

To further investigate the germination stimulant biosynthetic pathway, we tested several other maize carotenoid mutants and inhibitors for their effect on induction of *S. hermonthica* seed germination (Figure 2). From the Maize Genetic COOP Stock Center we obtained a number of mutants that are characterized by a change in the accumulation of carotenoids.
- The mutation causing the reduced carotenoid level in the *lw1 (lemon white)* carotenoid mutant is not defined yet. Seedlings of *lw1* exhibit an albino phenotype. Root exudates collected from *lw1* albino seedlings induced significantly lower germination of *S. hermonthica* in comparison to corresponding seedlings with non-mutant phenotype (Figure 5).
- Carotenoid mutant *y10* is characterised by a pale-yellow endosperm color and albino seedlings. Although the exact position of the mutation in the carotenoid pathway has not been clarified, the defect in the *y10* mutant is supposed to affect a step in the isoprenoid pathway preceding the biosynthesis of geranylgeranyl diphosphate (Figure 2), which results in a reduced content of carotenoids in the endosperm and carotenoids and chlorophylls in the leaves of the mutant seedlings (Janick-Buckner et al., 1999, J Hered, 90, 507-513). Root exudates of *y10* mutant seedlings induced a significantly lower germination than wild-type sibling plants (Figures 4,5).
- The *al1-y3* mutant (variation of al1 *albescent plant* 1) seedlings have a white to pale-green phenotype. According to Singh et al (Singh et al., 2003, supra) the mutation is associated with β-carotene-3-hydroxylase, but according to (Wurtzel, Recent Advances in Phytochemistry, vol. 38, in press) it is associated with phytoene synthase (Figure 2). The root exudates of mutant *al1y3* seedlings induced lower germination of S. *hermonthica* seeds than the sibling seedlings from a segregating ear that exhibit a non-mutant phenotype (Figure 5). Carotenoid analysis showed 10-fold lower concentration of all detected carotenoids in the shoots of mutant seedlings than in seedlings with non-mutant phenotype (Table 1). Since also lutein content is strongly reduced in the mutant phenotype it is highly likely that *al1y3* is indeed a mutant associated with phytoene synthase. In roots of mutant as well as wildtype phenotype seedlings, carotenoids were all below the detection level (Table 1).
- Mutant *vp*5 (*viviparous* 5) is known to be associated with phytoene C-12,13 desaturation (Figure 2), which causes an accumulation of phytoene (Li et al., 1996, supra). *Vp*5 mutant seedlings are white and root-exudate induced S. *hermonthica* germination was much lower than for wild-type seedlings (Figure 5).
- Mutant y9 *(pale yellow9*) exhibits a slightly pale-green phenotype. Y9 is associated with the conversion of ζ-carotene to lycopene in the maize endosperm (Figure 2). Chlorophyll and carotenoids could still be detected in pale-green leaves of homozygous y9 plants, albeit in reduced amounts (Janick-Buckner et al., 2001, Maydica, 46, 41-46). Although y9 mutant seedlings also induced significantly lower germination of *S. hermonthica* seeds than seedlings with non-mutant phenotype, germination was higher than for the albino mutants, *lw1, y10* and *vp*5, described above showing that the mutation in y9 indeed is not 100% effective (Figure 5).
- Finally, maize mutant *vp14* -2274 was assessed. *Vp14* is a mutation in 9-cis-epoxycarotenoid dioxygenase (NCED), an important step in the abscisic acid biosynthetic pathway (Schwartz, S. H. et al., 1997, Plant Physiol., 114, 798-798) (Figure 2). The mutation is slightly leaky. *Vp14* does not have an albino phenotype, suggesting that carotenoid formation is not affected by the mutation. Nevertheless, the mutant induced lower germination than the control (P=0.09) (Figure 5).

### 2.3 Elucidation of later steps

To assess whether abscisic acid is a precursor of the germination stimulants, maize seedlings were grown in the absence or presence of fluridone in combination with 0.02 or 0.2 mM (±)-abscisic acid. The roots of abscisic acid treated plants were slightly thicker and more brittle than in non-treated seedlings and the shoots were green. Independent of the presence of fluridone, plants supplied with abscisic acid induced very low germination of *S. hermonthica* (Figure 3D). Subsequently, the effects of naproxen, a putative inhibitor of NCED (Lee and Milborrow, 1997, Aust J Plant Physiol, 24, 715-726) and sodium tungstate, an inhibitor blocking oxidation of the C-1 aldehyde group of xanthoxin (Lee and Milborrow, 1997, supra) were assessed (Figure 2). Plants treated with naproxen had a normal green phenotype with slight inhibition of root growth at the highest concentration. Naproxen reduced germination of *S. hermonthica* seeds to 20±1% (0.1 mM) or 15±2% (1 mM), which represents a **44** % reduction compared with non-treated plants (27±2%) (Figure 3D). Sodium tungstate used in concentrations of 0.1 mM and 1mM (Figure 4m,n) did not affect germination (Figure 3D) even though 1 mM sodium tungstate did affect maize root morphology. Roots were much shorter, thicker and more fragile than in control seedlings.

Plants treated with fluridone, naproxen, sodium tungstate and abscisic acid were analysed for carotenoid contents of roots and shoots. In fluridone treated roots phytoene accumulated but other carotenoids could not be detected (Table 1). In naproxen treated roots the concentration of violaxanthin was about 3-fold and of β-carotene 2-fold higher than in control roots (Table 1). In sodium tungstate treated roots the amount of violaxanthin was 2-fold higher than in control plants. In contrast, in abscisic acid treated roots violaxanthin and ß-carotene concentrations were 37 and 50% lower than in control roots, respectively. Also in the shoot of fluridone treated plants phytoene accumulated whereas other carotenoids were below detection level (Table 1). Sodium tungstate did not affect carotenoid accumulation in shoots. Both naproxen and abscisic acid decreased accumulation of β-carotene, violaxanthin, neoxanthin and lutein (Table 1).

### 2.4 Conclusions

The results demonstrate that the germination stimulants of S. *hermonthica* present in the root exudates of maize, cowpea and sorghum are derived from the carotenoid biosynthetic pathway. We showed that this also holds for the germination stimulant(s) of *O*. *crenata* in the root exudate of cowpea. For these three host species - maize, cowpea and sorghum - the germination stimulants have been isolated and identified to be strigolactones, viz. strigol, alectrol and sorgolactone (Figure 1) (Hauck et al., 1992, supra; Muller et al., 1992, supra; Siame et al., 1993, supra). Our results with *O*. *crenata* suggest that this species also responds to a strigolactone germination stimulant. *O*. *crenata* is not a parasite of cowpea but parasitizes legumes in more-temperate climates such as North-Africa and Spain. The germination stimulant(s) of *O*. *crenata* has not been identified yet. Root exudates of cowpea readily induced germination of *O*. *crenata* and fluridone treatment decreased this germination by about the same percentage as for S. *hermonthica.* This makes it likely that the germination stimulants of *Orobanche* spp. that parasitise legumes in temperate regions of the world are also strigolactones and are hence also derived from the carotenoid pathway. This hypothesis is supported by the detection of orobanchol, alectrol and a third unidentified strigolactone in the legume red clover (Yokota et al., 1998, supra) and alectrol in cowpea (Muller et al., 1992, supra).

The fact that we demonstrate the carotenoid-origin of the germination stimulants for two parasitic plant species and three mono- and dicotyledonous hosts, and the (tentative) identification of strigolactones in the root exudates of other plant species such as red clover and tomato (Yokota et al., 1998, supra; Yoneyama et al., 2004, supra) suggest that carotenoid-derived germination stimulant formation occurs throughout the plant kingdom. The finding that sorghum root exudate induced S. *hermonthica* germination is completely blocked by fluridone sheds an interesting light on the discussion about the "true" nature of the sorghum germination stimulant. Lynn and coworkers have claimed that sorgoleone is the natural sorghum germination stimulant but this was disputed by Zwanenburg and coworkers based a.o. on the low water solubility of dihydrosorgoleone (Butler, 1995, supra; Keyes et al., 2001, supra; Wigchert and Zwanenburg, 1999, supra). The present results make it very likely that indeed the natural sorghum germination stimulant is sorgolactone.

The results of the present bioassays with inhibitors and mutants suggest that the biosynthesis of germination stimulants branches of from the carotenoid pathway at an intermediate that is a product of NCED action. This may be xanthoxin but could more likely be an analogue derived from cleavage of other substrates such as 9-*cis*-β-carotene. A biogenetic scheme can be postulated starting from such a 9-*cis*-β-carotene cleavage products, but also from xanthoxin (Figure 6). However, downstream derivatives of xanthoxin or alternative carotenoids could also serve as substrate in this biogenetic scheme.

Starting from 9-*cis*-ß-carotene, C11-12 cleavage by a dioxygenase leads to a C15-aldehyde, which upon hydroxylation yields intermediate a (Figure 6). Alternatively, xanthoxin, upon opening of the epoxyde ring followed by protonation, elimination of water at C3 followed by hydrogenation, attack of water and loss of water could also lead to intermediate a. Oxidation followed by epoxydation and decarboxylation, protonation and elimination of water leads to intermediate b. This intermediate, upon attack of water at C7, cyclizes to intermediate c or its tautomeric aldehyde. After triple oxidation of the methyl at C9, a lactone ring will be formed with the C7 hydroxyl group to produce intermediate d. Alternatively, the methyl at C9 could already be oxidized to form a carboxyl group in compound a (not shown in Figure 6). Instead of attack of water at C7, that carboxyl group could then attack the carbocation at C7 leading directly to intermediate d. As an alternative to lactone ring formation as described above, intermediate c could undergo keto-enol tautomerization, followed by oxidation to form a carboxyl group at C10, which can then form a lactone ring with the hydroxy at C7 (not shown in Figure 6). Double oxidation of the methyl at C9, would then also lead to intermediate d. From intermediate d, allylic hydroxylation in ring A or B or demethylation in ring A and coupling of the D-ring will lead to strigol, orobanchol and sorgolactone, respectively (Figure 6). The structure of alectrol is still under debate. It is not unlikely that our biogenetic scheme may help to postulate a new structure for alectrol which should subsequently be proven using chemical synthesis. Of course the order of the reactions as we propose it may *in vivo* be different. In addition, it is not unlikely that the D-ring is coupled to for example intermediate d before hydroxylation/demethylation to form the three different germination stimulants.

In conclusion, our results with mutants and inhibitors, and the postulated biogenetic scheme lead to the conclusion that a carotenoid cleavage dioxygenase, most likely NCED, is involved in the biosynthesis of the strigolactone germination stimulants in hosts of *Orobanche* and *Striga* spp. After this cleavage step, a number of other enzymatic reactions, such as hydroxylation, epoxydation, oxidation, etc are involved in the further modification of the primary apocarotenoid skeleton to the different strigolactones.

### Example 3. Use of inhibitors to decrease parasitic weed infestation

In the above examples, inhibitors of carotenoid biosynthesis were used to proof the biosynthetic origin of the germination stimulants. In this example, we describe the use of these inhibitors to reduce the formation of germination stimulants in situ, in planta and the consequences this has for parasitic weed infestation. Hereto, rice seeds (Taichung Native 1, Tn 1) were surface sterilized with 70% ethanol for 1 minute, and then washed immediately with demi water. Subsequently, 2% sodium hypochlorite plus 0.02% Tween20 were added. After 30 minutes the seeds were washed 5 times during 10 minutes in sterile demi water. The seeds were sown in a 9 cm petridish with filter paper wetted with 4 ml sterile demi water and incubated at 28 °C for two days. Then the seeds were placed in washed silver sand in pots (24cm high x 10cm diameter). Two seeds were sown in each pot and seedlings were thinned to one per pot 7 days after sowing. Treatments with fluridone started two weeks after sowing. Fluridone was either applied directly into the sand (irrigation with 100 mL of 0.1 µM in nutrient solution) or sprayed to the leaves (sprayed until run-off with 0.001, 0.01 and 0.1 µM fluridone in water with 0.01% Tween-20). Irrigation was applied once, spraying was repeated during 3 consecutive days. Five days later, preconditioned *Striga hermonthica* seeds (35 mg) were applied to the roots of the rice plants. Hereto, the sand was carefully removed from the roots of the seedlings and then the Striga seeds (dispersed in water) were applied onto the root system using a syringe. Subsequently, the roots were covered with sand. Control plants were treated similarly but without fluridone treatments. After that, the pots were placed in a controlled environment greenhouse (12hr photoperiod, 28°C day/night, additional light and relative humidity 85%). Five replicates were used in each experiment. Throughout the experiment pots were irrigated with 40% full strength Long Ashton solution containing 20% of the normal nitrogen rate. After 6 weeks, the sand was carefully washed from the roots and the number of germinated and/or attached Striga seeds/tubercles determined using a binocular.

Irrigation with 0.1 µM of fluridone significantly reduced the number of germinated/attached Striga seeds (Figure 7A). In control experiments, where the synthetic germination stimulant GR24 was used to induce full germination, we could conclude that fluridone was not affecting the attachment phase (but just germination) (data not shown). In the subsequent experiment fluridone was applied by spraying, and also here a strong, significant reduction in the number of germinated/attached seeds/tubercles of Striga was obtained even at 0.001 and 0.01 µM (Figure 7B). In both experiments, the concentrations of fluridone used were so low that bleaching of the leaves did not occur.

### Example 4. The effect of mycorrhizae on germination stimulant formation

To investigate the effect of mycorrhizae on strigolactone production, we inoculated maize plants with *Glomus intaradices,* evaluated mycorrhizal colonisation at different time points after inoculation and collected exudates that we assayed for germination stimulant content using a bioassay with *Striga hermonthica* seeds. To ensure synchronous mycorrhization of the roots, the maize plants were grown in pots filled with 70% (v/v) expanded clay mixed with 30 % (v/v) expanded clay containing Glomus *intraradices* Schenck & Smith inoculum/propagules in which leek had been growing, obtained from Michael Walter and Thomas Fester (Fester et al., 2002a, supra). The plants were watered with 0.5 strength Hoagland's nutrient solution with reduced phosphate content (10% of phosphate in a full strength nutrient solution). Starting after **12** days of growth under controlled conditions in a greenhouse, the maize seedlings were harvested weekly. The root and shoot weight were recorded. The level of colonization of the roots was determined by tryphan blue staining. In roots, hyphae, vesicles and arbuscules were identified. After 33 days a yellow colour was seen on the roots that were colonized by mycorrhizal fungi. This colour probably indicates the presence of mycorradicin. Root exudates were collected and germination bioassays carried out as described in Example 1 to determine the induction of *Striga hermonthica* germination. In both experiments mycorrhizal root exudates consistently induced lower germination than the exudates of control roots and the effect was particularly large in the first experiment 40 days after inoculation (Figure 8).

### Example 5. Cloning of target genes involved in strigolactone formation

### 5.1 Introduction

Because the biosynthetic origin of the germination stimulants has now been elucidated herein, it is possible to design strategies to reduce or increase the production of germination stimulants, so crop varieties can be developed with resistance against *Striga* and *Orobanche* spp, or with improved trap/catch crop performance. Target genes for such a strategy are all the genes involved in carotenoid metabolism, carotenoid cleavage and modification of that cleavage product to the germination stimulant. The proof that this works comes from our inhibitor and mutant studies described in Example 2. In these studies the down-regulation of germination stimulant formation was obtained in a rather crude way: carotenoid biosynthesis was blocked relatively early in the pathway and throughout the plant which in most cases led to a letal or in any case growth-retarded phenotype. Nevertheless, the genes/enzymes affected in these mutants and/or by our inhibitors are suitable candidates to target, provided that the regulation is subtle. Possibilities to achieve this are the use of specific promoters, active only in the (root) tissues involved in germination stimulant formation and/or only during a limited developmental time span and/or promoters of which activity can be induced using the application of external signals, such as chemicals.

In addition to target genes/enzymes involved in the primary carotenoid/ABA pathway, such as phytoene synthase, phytoene desaturase, ξ-carotene desaturase, carotene isomerase, lycopene cyclase, ß-carotene hydroxylase, zeaxanthin epoxidase and neoxanthin synthase, enzymes involved in the branching and/or the further modifications leading to the germination stimulants are suitable targets for engineering, breeding or selection for reduced or increased germination stimulant formation too. These enzymes include CCDs, NCEDs, cytochrome P450 hydroxylases and epoxidases, dehydrogenaes, demethylase, a D-ring transferase etc.. To clone these genes we follow several strategies. These include subtractive techniques and transcriptomics approaches to identify genes that are downregulated by mycorrhizal colonisation, as in the present invention we show that mycorrhizal colonisation reduces strigolactone formation (Example **4).** In addition, we exploit the negative effect of phosphate on orobanchol production (Koichi Yoneyama, personal communication) in subtractive and transcriptomics approaches to pinpoint genes that are down-regulated by high phosphate and/or up-regulated by low phosphate and that fit in our postulated biosynthetic scheme. In another strategy, we are cloning the first cytochrome P450 encoding gene from this pathway for example using the expected homology expected to exist with MAX1, a cytochrome P450 from Arabidopsis and oxidising the carotenoid cleavage product of a CCD (CCD7/MAX3) (Booker et al., 2005, Developmental Cell 8, 443-449**;** Booker et al., 2004, Current Biology 14, 1232-1238).

All the target genes/enzymes involved can be used for a transgenic approach but can also be used as markers for selection of low or high producers.

As an example, below the isolation and characterisation of a CCD and NCED from maize is described and their use to make transgenic plants with decreased germination stimulant formation. In other examples below we describe how we use rice to screen all possible carotenoid cleaving enzymes and tomato for fast evaluation.

### 5.2 Cloning of target genes

### DNA isolation and cDNA synthesis

RNA from roots of maize cultivar Dent MBS847 was extracted by the SV total RNA Isolation kit from Promega, according to the manufacturer's instructions. 1 ug of total RNA was used in a volume of 3 ul, and mixed with 1 ul polyT primer (10 uM). The mixture was incubated at 70°C for 2 minutes, and immediately put on ice for 2 minutes. Then 2 ul 5x1st strand buffer (Invitrogen), 1 ul 100 mM DTT, 1 ul 10 mM dNTP, 1 ul Rnasin (Invitrogen) and 1 ul SST Reverse Transcriptase (Invitrogen) were added and the mixture was incubated at 42°C for 90 minutes. After this, the mixture was inactivated for 7 minutes at 70°, and stored on ice.

### Amplification of cDNAs

cDNAs of target genes can be amplified using the sequences available in the databases (see e.g. sequence ID 1-18) and/or using the high homology between these sequences and genes encoding the same protein in different plant species that has been shown to be present. As an example we describe the cloning of a carotenoid cleavage dioxygenase (ZmCCD1) and a 9-*cis*-epoxycarotenoid dioxygenase (ZmNCED = *vp14*) from maize. To clone the CCD, we used a degenerate primer PCR approach. Degenerate primers CCDfwd1 (5'-TGYYTNAAYGGNGARTTYGTNMGNGTNGGNCCNAAYCCNAARTTY-3') and CCDfwd2 (5'-GTNGCNGGNTAYCAYTGGTTYGAYGGNGAYGGNATGATHCAYGGN-3') and CCDrev1 (5'-ATGATGCAYGAYTTYGCNATHACNGAR-3') and CCDrev2 (5'-ACNAARAARGCNMGNTTYGGNGTNYTNCCNMGNTAYGCN-3') were used to amplify a PCR fragment using 1 ul of cDNA in an amplification reaction mix. The mix further contained 0.5 mM dNTP, 2.5 ul 10x BD Advantage 2 PCR buffer (BD Bioscience), 0.5 ul 50x Advantage 2 polymerase mix (BD Bioscience) and 0.4 uM of forward and reverse primers. The amplification reaction mix was incubated for 5 minutes at 94°, and subsequently subjected to 30 cycles of 30 seconds 94°, 30 seconds 45°C and 3 minutes 72°C. After these cycles, the mixture was incubated at 72°C for 5 minutes, after which it was cooled to 10°C. 1 ul of this reaction was used as a template for a second PCR reaction, under the same conditions, but with oligonucleotides CCDfwd2 and CCDrev2. The resulting PCR fragment was cloned, sequenced and BLASTed and found to belong to accession TC220599 in the TIGR database. The full length cDNA was amplified using specific primers ZmCCD1fwd1 (5'-CTTCGCTACAAGTCATCTCG-3') and ZmCCD1fwd2 (5'-CAAGTCATCTCGCCGCAACC-3') and ZmCCD1rev1 (5'-AGTGAAGATACGGCACCTGC-3') and ZmCCD1rev2 (5'-GCAGGACGTGTATTCGAACC-3') and using the conditions as above except that for the annealing temperature 55°C was used. The 1928 bp product was cloned into pGEMTeasy and this plasmid was sequenced. The sequence of the ZmCCD1 coding region, and the encoded protein, are provided in the Sequence information section (Sequence ID 19-20).

The ZmNCED *(vp14)* gene was amplified essentially as described above for ZmCCD1 but using sequence information from Genbank and specific and degenerate primers ZmNCEDfwd1 (5'-TTYGAYGGIGAYGGIATGRTICAYGC-3'), ZmNCEDfwd2 (5'-CCIAARSCIATHGGIGARYTICAYGGNCA-3') and ZmNCEDfwd3 (5'-GARAAYTTYGTIGTIRTICCIGAYCANCA-3') and ZmNCEDrev1 (5'-TCIGTDATIGCRAARTCRTGIATCATNGT-3'), ZmNCEDrev2 (5'-TCCCAIGCRTTCCAIARRTGRAARCARAA-3') and ZmNCEDrev3 (5'-AYRAAIGTICCRTGRAAICCRWAIGGNAC-3'). The obtained sequence is displayed as Sequence ID 17-18.

### Cloning into an expression vector

One ug of plasmid DNA from pGEMT-ZmCCD1 and from pRSETA was digested with BamHI and EcoRI in the appropriate buffer. Both digestions were loaded on a 1 % agarose gel. After electrophoresis, fragments of the expected size (about 1650 bp for the ZmCCD fragment and about 2900 bp for the vector DNA) were observed, and isolated from the gel using Qiaex II DNA isolation kit (Qiagen). Fragments were suspended into 30 ul EB buffer (50 mM Tris pH = 8.5). To clone the ZmCCDgene into pRSETA, 1 ul of BamHI-EcoRI cleaved pRSETA and 10 ul of purified and cleaved ZmCCD product were mixed with 3 ul 5xligase buffer (Invitrogen) and 1 ul of T4 ligase (Invitrogen). The ligation mixture was incubated for 3 hours at 16°C and 10 ul was used for transformation of competent *E. coli* XL-1 blue by standard procedures. The transformation mixture was plated on 25 ml petridishes containing LB medium, 1.5% technical agar and 100 ug/ml ampicillin. After overnight incubation at 37 °C, colonies were picked into 3 ml liquid LB medium with 100 ug/ml ampicillin and grown overnight at 37°C shaking at 250 rpm. Plasmid was isolated from 1.5 ml of this culture using the Qiagen plasmid isolation kit, and clones containing plasmids with inserts were identified by restriction digestion with KpnI and BamHI. Plasmid pRSETA-ZmCCD1 was identified in this way. Other genes are cloned essentially similar.

### Characterisation of cloned genes

To assess the substrate specificity of the cloned cleavage enzymes they were either cloned into a host cell producing T7 polymerase and the carotenoid substrates to be assayed or isolated from the expression host described above and assayed in vitro by applying the relevant carotenoid substrates. For cloning into a carotenoid-producing host cell, the T7 polymerase starts transcription from the T7 promoter, which is located just upstream of the ZmCCD cDNA in plasmid pRSETA-ZmCCD#1. *E. coli* strain BL21 is able to produce T7 polymerase in the absence of glucose. When this strain carried plasmid pRSETA-ZmCCD1, the ZmCCD protein was produced. ß-Carotene can be produced in *E. coli* by providing it with the plasmid pAC-BETA, which has been described by Cunningham et al. (1996; Plant Cell 8, 1613-1626). To construct bacteria that are capable of producing both ß-carotene and ZmCCD or NCED enzyme, *E. coli* BL21 CodonPlus-RIL (Stratagene) competent cells were transformed with pAC-BETA according to the manufacturer's instructions. Recombinant *E*. *coli* were selected overnight at 37°C on LB-agar plates with 1% glucose and 30 ug/ml chloramphenicol. A colony of *E. coli* BL21 with pAC-BETA was inoculated in 1 ml LB with chloramphenicol and glucose and the culture grown overnight at 250 rpm and 37°. The BL21-pAC-BETA was made competent by diluting the overnight culture 100-fold in fresh LB medium with 1% glucose, and shaking it at 37°C until an optical density at 600 nm of 0.4 was reached. 10 MI of culture was centrifuged for 5 minutes at 400xg. Supernatant was discarded and replaced by 10 ml of an ice-cold solution of 10 mM CaCl2 and 1 mM Tris-HCl pH = 7.5. Cells were resuspended and immediately centrifuged again at 400xg for 5 minutes. After discarding the supernatant, cells were resuspended in 2 ml of an ice-cold solution of 75 mM CaCl2 and 1 mM Tris-HCl pH = 7.5. After incubation on ice for at least 30 minutes, cells were used for plasmid transformation by standard procedures. Plasmids pRSETA and pRSETA-ZmCCD1 were used to transform these cells, and transformed colonies were selected on LB-agar plates supplied with 1% glucose, 20 ug/ml chloramphenicol and 50 ug/ml ampicillin. ß-carotene cleavage by ZmCCD was detected by discoloration of ß-carotene in BL21-pAC-BETA-pRSETA-ZmCCD colonies, zeaxanthin cleavage by discoloration of zeaxanthin in BL21-pAC-ZEA-pRSETA-ZmCCD colonies and lycopene cleavage by discoloration of lycopene in BL21-pAC-ZEA-pRSETA-ZmCCD colonies. Alternative substrates are tested in vitro. Hereto, in vitro enzyme assays are used as described in the literature (e.g. (Bouvier et al., 2003, supra; Schwartz et al., 1997, supra; Schwartz et al., 2003, supra).

### Example 6. Transformation of maize to decrease germination stimulant formation

### 6.1 Constructs

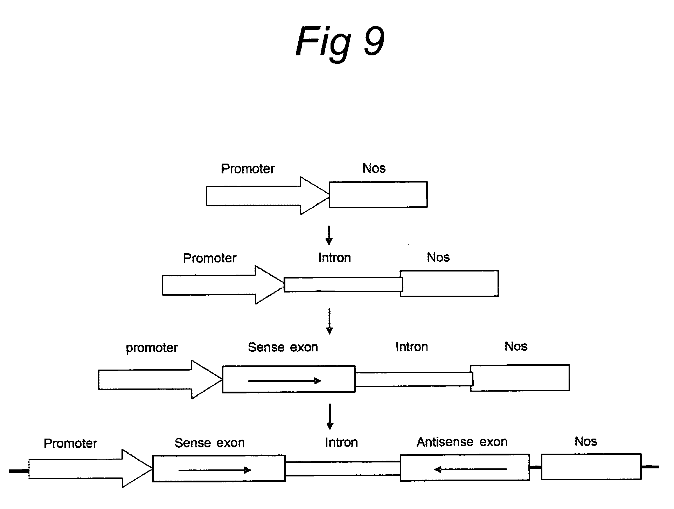

RNAi constructs were made as depicted in Figure 9. Hereto, PCR fragments of 331 nucleotides were generated using PCR. Fragments were cloned into pUBIcas (modified pUC18) as an inverted repeat separated by an intron (Figure 9). As promoters we used root-specific promoters. As an example we describe the construct for NCED.

Primer 1 forward (intron 5'-onwards oligo introducing BamH I site): 5'GGATCCCTCCTGGGTCTCTGAGAT3'; primer 2 reverse (intron 5'-onwards oligo introducing Kpn I site): 5'GGTACCCCAGCCACACCCTCCTTT3'; primer 3 forward (sense fragment 5'-onwards oligo introducing BamH I site): 5'GGATCCCCACGATGATCCACGACTTC3'; primer 4 reverse (sense fragment 5'-onwards oligo introducing Bgl II site): 5'AGATCTATCTCGGTCAGCACGCTCTC3'; primer 5 forward (antisense fragment 5'-onwards oligo introducing Sal I site): 5'GTCGACCCACGATGATCCACGACTTC3'; primer 6 reverse primer 1 (antisense fragment 5'-onwards oligo introducing Kpn I site): 5'GGTACCATCTCGGTCAGCACGCTCTC3'

The constructs were made by cloning the promoters in pUC18 (the nos terminator already inside). Three promoters were used for the constructs, ubiquitin from maize and two root specific promoters pHm62 from rice and MtPT1 from red clover. Then the intron was generated from *OsSHN1* genomic DNA using primer 1 and primer 2 and inserted into the vector. Then the 331 bp sense fragment of NCED was inserted (obtained using primer 3 and 4). Finally the 331 bp antisense fragment (obtained using primer 5 and 6) was ligated into the vector.

### 6.2 Plant transformation

Maize inbred lines A188 und H99 are transformed essentially as described by Brettschneider et al. (1997; Theor Appl Genet 94, 737-748). Particle bombardment of the scutellar tissue of immature embryos is carried out using a plasmid containing the pat (phosphinothricin acetyltransferase) gene as the selection marker and the plasmid with the construct. The co- transformation frequency is between 70-80% depending on the size of the construct. Bombardment is carried out with a PDS 1000/He gun (BioRad) using optimal parameters, such as the amount of gold particles used per bombardment, particle velocity, preculture time of the scutellum prior to bombardment and osmotic treatment of the target tissue before and after bombardment (Brettschneider et al., 1997, supra). Transgenic regenerants are selected on medium containing Basta [glufosinate]. Transgenic plant lines are verified with PCR and Southern blots, to check copy number.

Primary transformants are assayed for the induction of germination of Striga and Orobanche as described above (Example 1). The recombinant plants result in a significant reduction of germination of both Orobanche and Striga seeds, indicating that the plants have enhanced resistance to parasitic weeds.

### Example 7. Agrobacterium rhizogenes transformation of tomato for quick screening of strigolactone Pathway genes

In order to have a quick screening method for strigolactone pathway candidate genes, stable transformants are not required. Therefore we have adapted an *Agrobacterium rhizogenes* transformation protocol, developed by Limpens et al., 2004 (J Exp Botany, 55: 983-992) for use as a fast screening method for strigolactone biosynthetic pathway gene candidates. In this method RNAi constructs of candidate genes can be quickly made and transformed to create transgenic tomato roots. The effect of the gene knock/out can be efficiently evaluated using an *in situ* germination bioassay with *Orobanche ramosa.* The A. *rhizogenes* transformation, as additional advantage, usually results in transgenic and non-transgenic roots on the same seedling (that can be discriminated using a red fluorescent marker (see below), such that germination with wildtype root exudate and transgenic root exudate can be compared on the same seedling/plant.

For this method, tomato seeds were sterilised for 20 min in 2% hypochlorite, containing 0.02 % (v/v) Tween 20 and washed 3 x 20 min in sterile demineralised water. Seeds were incubated in Petri-dishes on sterile 3MM Whatmann paper wetted with Farhaeus medium without agar (Limpens et al. 2004, supra). Seeds were germinating and subsequently grown in a climate room at 21 ° C, 16/8 h light/darkness with the Petridish in vertical position. The roots of 11 days old seedlings were cut from the hypocotyl and the wound surface inoculated with *A*. *rhizogenes* containing the appropriate binary vector (see below). To increase infection efficiency, the hypocotyls were also wounded by needles immersed in a culture of the same A. *rhizogenes.* The seedlings were co-cultivated with the *A. rhizogenes* for 7 days at 21 °C, 16/8 h light/darkness. Subsequently, the seedlings were transferred into Emergence medium, containing 300 µg ml⁻¹ Cefotaxime (Duchefa), without agar (Limpens et al., 2004, supra). The plates were partly covered by aluminium foil and seedlings grown at 21 ° C, 16/8 h light/darkness in vertical position for 7-14 days. In this time new roots were formed, partly co-transformed with T-DNA of the binary vector, partly not (control roots). The co-transformed roots can be discriminated by the presence of DsRED1.

Single plants with transformed as well as non-transformed adventitious roots were transferred into a square Petri dish placed under an angle of about 45° with a small hole at the top, through which the shoot protuded out of the Petri dish while the roots grow inside on 3MM Whatmann paper wetted with Farhaeus medium. The Petri dish was sealed with parafilm and covered with aluminium foil. For germination bioassays a 40 x 15 mm sheet of sterile plastic foil covered with a 35 x 12 mm sheet of GFFP containing preconditioned *O*. *ramosa* seeds was placed on the 3MM Whatmann paper but under the tomato roots (transformed and non-transformed). In this way any contact between the *O*. *ramosa* seeds and possibly accumulated germination stimulants in the Whatmann paper is avoided. The GFFP containing *O*. *ramosa* seeds was wetted with Farhaeus medium. Two to three days later the GFFP with Orobanche seeds was transferred into a new Petri dish to allow Orobache seeds to germinate. The germination of Orobanche seeds induced by transformed/non-transformed roots was scored.

As an example of how candidate genes can be screened using this method, below we describe the cloning of RNAi constructs for LeNCED1 and a generic LeNCED RNAi construct (aimed to target all tomato NCEDs).

Genomic DNA was isolated from tomato plants, cultivar Moneymaker of 2 weeks old plants. The DNA was isolated from 2 youngest leaves using Tri Reagent (Sigma), according to the manufacturer's protocol. The LeNCED1gene was amplified from genomic DNA using nested PCR. The primer designed was based on the LeNCED1 gene sequences from the NCBI database.

| First PCR | |
|---|---|
| Forward1 | CCATAATTCCACACTCTCCC |
| Reverse1 | GGACGTATATTCTAAACCATCCC |

| Second PCR | |
|---|---|
| Forward2 | CTCTCCTCATTTCCCACCTC |
| Reverse2 | GTCACAATCATGCCTGATTTGCC |

LeNCED1 was ligated into pRSET-A (Invitrogen) and transformed to E. *coli* using restriction sites introduced by PCR using primers:

| | |
|---|---|
| Forward | GTGACGGATCCATGGCAACTACTACTTCACA |
| Reverse | GTGACGAATTCTCATGCCTGATTTGCCAAAT |

The tomato NCED fragments used for RNAi silencing were amplified by PCR using the isolated and purified LeNCED1-gene as a template. As a selection marker for co-transformed roots the gene coding for the fluorescent protein DsRED1 is used as a nondestructive selectable marker

Primer design based on conserved region (NCEDs)

| | |
|---|---|
| Forward | CACCGTCGGCTAGTTACGCTTG |
| Reversed | CTTGAGGTATGGCTTCTG |

Primer design based on non-conserved region (LeNCED1)

| | |
|---|---|
| Forward | CACCTGGCTATCGCTGAACCAT |
| Reversed | CACAGTTGCCTCCAACTT |

Amplified sequences from LeNCED1 for RNAi constructs:
For conserved region
For specific region

The fragments were cloned into pENTR-D-TOPO (Invitrogen) and recombined into modified Gateway pK7GWIWG2(II)-Q10:DsRED binary vector (Limpens et al., 2005, PNAS, 102: 10375-10380), which was then used to transform A. *rhizogenes* and subsequently tomato (see above).

### Example 8. Transformation of rice to select the carotenoid cleavage enzyme involved in strigolactone formation.

The carotenoid cleavage enzyme family in rice *(Oryza sativa)* was classified into 7 clusters containing 15 putative genes (SEQ ID 21, 23, 25, 27, 29, 31, 33, 35, 37, 39, 41, 43, 45, 47 and 49). In order to test which of these genes is involved in the formation of the strigolactones, we designed RNAi constructs for all of these genes. The inverted repeat regions (300 to 500 bps) for the constructs were amplified using specific primers and then subcloned into the pENTR/D-TOPO cloning vector (Invitrogen, Carlsbad, CA) to yield the entry vectors. The RNAi silencing constructs were made using the pANDA vector (Maki and Shimamoto, 2004, Plant Cell Physiol. 45, 490-495, Maki and Shimamoto, 2005, Plant Physiol. 138,1903-1913). The final RNAi vectors were developed by an LR clonase reaction between an entry vector and the pANDA vector. Transgenic rice plants were generated from transformed calli (cv Nipponbare) by selecting for hygromycin resistance. The protocol used was according to Ouwerkerk, Institute of Biology, Leiden University. The transgenic plants are being characterized using the Striga germination bioassays described in Examples 1 and 3 and show significantly reduced germination for one of the RNAi constructs.

## Claims

1. A method suitable for making a recombinant plant being resistant to one or more species of parasitic plants belonging to the genera *Orobanche* or *Striga,* said method comprises:
a. generating a gene silencing vector comprising a promoter active in plant cells operably linked to a sense and/or antisense nucleic acid sequence of an apocarotenoid pathway gene selected from a gene encoding a carotenoid cleavage dioxygenase 7 (CCD7) or a carotenoid cleavage dioxygenase 8 (CCD8),
b. transforming a plant cell, plant tissue or plant with the vector of step (a), and
c. selecting transformed cells, tissues or plants and regenerating a recombinant plant therefrom,
d. testing the germination of *Orobanche* and/or *Striga* seeds in the presence of roots, root exudates or root extracts of the recombinant plant and selecting a plant which results in a lower seed germination compared to a non-recombinant control plant,
wherein said recombinant plant produces reduced amounts of at least one strigolactone in the root tissue.

2. The method according to claim 1, wherein said apocarotenoid pathway gene is selected from any one of SEQ ID NO: 35 and SEQ ID NO: 47 or from a nucleic acid sequence having at least 70% sequence identity over the entire length to any one of these sequences and having the carotenoid cleavage dioxygenase activity of the corresponding protein encoded by said apocarotenoid pathway gene selected from any one of SEQ ID NO: 35 and SEQ ID NO: 47, respectively.

3. The method according to any of the preceding claims, wherein the plant is selected from maize, rice, millet, sorghum, cowpea, tomato, tobacco, melon, rapeseed, pea and sunflower.

4. A method for making a recombinant trap or catch crop plant of parasitic plants belonging to the genera *Orobanche* or *Striga,* said method comprises:
a. generating an expression vector comprising a promoter active in plant cells operably linked to a nucleic acid sequence encoding an apocarotenoid pathway enzyme selected from a carotenoid cleavage dioxygenase 7 (CCD7) or a carotenoid cleavage dioxygenase 8 (CCD8),
b. transforming a plant cell, plant tissue or plant with the vector of step (a), and
c. selecting transformed cells, tissues or plants and regenerating a recombinant plant therefrom, and
d. testing the germination of *Orobanche* and/or *Striga* seeds in the presence of tissue, tissue exudates or tissue extracts of the recombinant plant and selecting a plant which results in a higher seed germination compared to a non-recombinant control plant,
wherein that said recombinant plant produces enhanced amounts of at least one strigolactone in at least one of its tissues.

5. A method for producing a non-recombinant plant which produces significantly reduced or significantly enhanced amounts of one or more strigolactone germination stimulants, the method comprising:
a. subjecting cells, tissues or plants to mutagenesis,
b. selecting plants having one or more mutations in at least one carotenoid pathway gene selected from a carotenoid cleavage dioxygenase 7 (CCD7) or a carotenoid cleavage dioxygenase 8 (CCD8) and producing significantly reduced levels or significantly enhanced levels of one or more strigolactone germination stimulants.

6. Use of a nucleic acid sequence encoding a protein having at least 70% amino acid identity over the entire length to and having the carotenoid cleavage dioxygenase activity of SEQ ID NO: 36 or SEQ ID NO: 48, for generating a recombinant plant being resistant to at least one species of *Orobanche* and/or *Striga,* by transforming a plant with a gene silencing vector comprising a promoter active in plant cells operably linked to said nucleic acid sequence..

## Patentansprüche

1. Verfahren zum Herstellen einer rekombinanten Pflanze, die resistent gegenüber einer oder mehreren Arten von parasitären Pflanzen ist, die zur Gattung *Orobanche* oder *Striga* gehören, wobei das Verfahren umfasst:
a. Erzeugen eines Genexpressionshemmungsvektors umfassend einen in Pflanzenzellen aktiven Promoter, der operativ mit einer Sense- und/oder Antisense-Nukleinsäuresequenz eines apocarotenoiden Pathway-Gens verbunden ist, das ausgewählt ist aus einem Gen, das für eine carotenoidspaltende Dioxygenase 7 (CCD7) oder eine carotenoidspaltende Dioxygenase 8 (CCD8) kodiert,
b. Transformieren einer Pflanzenzelle, eines Pflanzengewebes oder einer Pflanze mit dem Vektor aus Stufe (a), und
c. Selektieren von transformierten Zellen, Gewebe oder Pflanzen und Regenerieren einer rekombinanten Pflanze daraus,
d. Testen der Keimung von *Orobanche* und/oder *Striga* Samen in Anwesenheit von Wurzeln, Wurzelexsudaten oder Wurzelextrakten der rekombinanten Pflanze und Selektieren einer Pflanze, die zur einer niedrigeren Samenkeimung im Vergleich zu einer nicht rekombinanten Kontrollpflanze führt,
wobei die rekombinante Pflanze reduzierte Mengen mindestens eines Strigolactons im Wurzelgewebe produziert.

2. Verfahren nach Anspruch 1, wobei das apocarotenoide Pathway-Gen ausgewählt ist aus einer von SEQ ID NO: 35 und SEQ ID NO: 47 oder aus einer Nukleinsäuresequenz mit einer mindestens 70%igen Sequenzidentität über die gesamte Länge zu irgend einer dieser Sequenzen und mit der carotenoidspaltenden Dioxygenase Aktivität des entsprechenden Proteins, das von dem apocarotenoiden Pathway-Gens ausgewählt aus einer von SEQ ID NO: 35 bzw. SEQ ID NO: 47 kodiert wird.

3. Verfahren nach einem der vorangehenden Ansprüche, wobei die Pflanze ausgewählt ist aus Mais, Reis, Hirse, Sorghum, Kuhbohne, Tomate, Tabak, Melone, Raps, Erbse und Sonnenblume.

4. Verfahren zum Herstellen einer rekombinanten Falle oder Fangnutzpflanze für parasitäre Pflanzen der Gattung *Orobanche* oder *Striga,* wobei das Verfahren umfasst:
a. Erzeugen eines Expressionsvektors umfassend einen in Pflanzenzellen aktiven Promoter, der operativ mit einer Nukleinsäuresequenz verbunden ist, die für ein apocarotenoides Pathway-Enzym kodiert, das ausgewählt ist aus einer carotenoidspaltenden Dioxygenase 7 (CCD7) oder einer carotenoidspaltenden Dioxygenase 8 (CCD8),
b. Transformieren einer Pflanzenzelle, eines Pflanzengewebes oder einer Pflanze mit dem Vektor aus Stufe (a), und
c. Selektieren von transformierten Zellen, Gewebe oder Pflanzen und Regenerieren einer rekombinanten Pflanze daraus,
d. Testen der Keimung von *Orobanche* und/oder *Striga* Samen in Anwesenheit von Gewebe, Gewebeexsudaten oder Gewebeextrakten der rekombinanten Pflanze und Selektieren einer Pflanze, die zur einer höheren Samenkeimung im Vergleich zu einer nicht rekombinanten Kontrollpflanze führt,
wobei die rekombinante Pflanze erhöhte Mengen mindestens eines Strigolactons in mindestens einem ihrer Gewebe produziert.

5. Verfahren zur Herstellung einer nicht-rekombinanten Pflanze, welche erheblich reduzierte oder erheblich erhöhte Mengen eines oder mehrerer Strigolacton-Keimungsstimulanzien produziert, wobei das Verfahren umfasst:
a. Unterwerfen von Zellen, Gewebe oder Pflanzen der Mutagenese,
b. Selektieren von Pflanzen mit einer oder mehreren Mutationen in mindestens einem carotenoiden Pathway-Gen ausgewählt aus einer carotenoidspaltenden Dioxygenase 7 (CCD7) oder einer carotenoidspaltenden Dioxygenase 8 (CCD8), und die erheblich reduzierte oder erheblich erhöhte Mengen eines oder mehrerer Strigolacton Keimungsstimulanzien produziert.

6. Verwendung einer Nukleinsäuresequenz, die für ein Protein mit mindestens 70%iger Aminosäurenidentität über die gesamte Länge und mit der carotenoidspaltenden Dioxygenase Aktivität von SEQ ID NO: 36 oder SEQ ID NO: 48 kodiert, zur Generierung einer rekombinanten Pflanze, die gegenüber mindestens einer Art von *Orobanche* oder *Striga* resistent ist, durch Transformieren einer Pflanze mit einem Genexpressionshemmungsvektor umfassend einen in Pflanzenzellen aktiven Promoter, der operativ mit der Nukleinsäuresequenz verbunden ist.

## Revendications

1. Procédé adapté pour produire une plante recombinante qui est résistante à une ou à plusieurs espèce(s) de plantes parasites appartenant au genre *Orobanche* ou *Striga,* ledit procédé comprend le fait :
a. de générer un vecteur d'inactivation génique comprenant un promoteur actif dans des cellules végétales lié de manière fonctionnelle à une séquence d'acides nucléiques sens et/ou anti-sens d'un gène de la voie des apocaroténoïdes choisi parmi un gène codant pour une dioxygénase de clivage des caroténoïdes 7 (CCD7) ou une dioxygénase de clivage des caroténoïdes 8 (CCD8),
b. de transformer une cellule végétale, un tissu végétal ou une plante avec le vecteur de l'étape (a), et
c. de sélectionner des cellules, des tissus ou des plantes transformé(e)s et de régénérer une plante recombinante à partir de ceux-ci/celles-ci,
d. de tester la germination de graines d'*Orobanche* et/ou de *Striga* en présence de racines, d'exsudats racinaires ou d'extraits de racines de la plante recombinante et de sélectionner une plante qui entraîne une germination de graines plus faible par rapport à celle d'une plante témoin non recombinante,
dans lequel ladite plante recombinante produit des quantités réduites d'au moins une strigolactone dans le tissu de racine.

2. Procédé selon la revendication 1, dans lequel ledit gène de la voie des apocaroténoïdes est choisi parmi l'une quelconque de la séquence SEQ ID NO : 35 et de la séquence SEQ ID NO : 47 ou parmi une séquence d'acides nucléiques présentant au moins 70% d'identité de séquence sur la longueur entière avec l'une quelconque de ces séquences et ayant l'activité de dioxygénase de clivage des caroténoïdes de la protéine correspondante codée par ledit gène de la voie des apocaroténoïdes choisi parmi l'une quelconque de la séquence SEQ ID NO: 35 et de la séquence SEQ ID NO: 47, respectivement.

3. Procédé selon l'une des revendications précédentes, dans lequel la plante est choisie parmi le maïs, le riz, le millet, le sorgho, le dolique à oeil noir, la tomate, le tabac, le melon, la graine de colza, le pois et le tournesol.

4. Procédé pour la production d'une plante recombinante de culture piège ou dérobée de plantes parasites appartenant au genre *Orobanche* ou *Striga,* ledit procédé comprend le fait :
a. de générer un vecteur d'expression comprenant un promoteur actif dans des cellules végétales lié de manière fonctionnelle à une séquence d'acides nucléiques codant pour une enzyme de la voie des apocaroténoïdes choisie parmi une dioxygénase de clivage des caroténoïdes 7 (CCD7) ou une dioxygénase de clivage des caroténoïdes 8 (CCD8),
b. de transformer une cellule végétale, un tissu végétal ou une plante avec le vecteur de l'étape (a), et
c. de sélectionner des cellules, des tissus ou des plantes transformé(e)s et de régénérer une plante recombinante à partir de ceux-ci/celles-ci, et
d. de tester la germination de graines *d'Orobanche* et/ou de *Striga* en présence de tissus, d'exsudats tissulaires ou d'extraits tissulaires de la plante recombinante et de sélectionner une plante qui entraîne une germination de graines plus importante par rapport à celle d'une plante témoin non recombinante,
dans lequel ladite plante recombinante produit des quantités accrues d'au moins une strigolactone dans au moins l'un de ses tissus.

5. Procédé pour la production d'une plante non recombinante qui produit des quantités réduites de manière significative ou des quantités accrues de manière significative d'un ou de plusieurs stimulant(s) de la germination de strigolactone, le procédé comprenant le fait :
a. de soumettre des cellules, des tissus ou des plantes à une mutagenèse,
b. de sélectionner des plantes présentant une ou plusieurs mutation(s) dans au moins un gène de la voie des caroténoïdes choisi parmi une dioxygénase de clivage des caroténoïdes 7 (CCD7) ou une dioxygénase de clivage des caroténoïdes 8 (CCD8) et de produire des niveaux réduits de manière significative ou des niveaux accrus de manière significative d'un ou de plusieurs stimulant(s) de la germination de strigolactone.

6. Utilisation d'une séquence d'acides nucléiques codant pour une protéine présentant au moins 70% d'identité d'acides aminés sur la longueur entière avec la séquence SEQ ID NO : 36 ou la séquence SEQ ID NO : 48 et ayant l'activité de dioxygénase de clivage des caroténoïdes de la séquence SEQ ID NO : 36 ou de la séquence SEQ ID NO : 48, pour générer une plante recombinante qui soit résistante à au moins une espèce *d'Orobanche* et/ou de *Striga,* par transformation d'une plante avec un vecteur d'inactivation génique comprenant un promoteur actif dans des cellules végétales lié de manière fonctionnelle à ladite séquence d'acides nucléiques.


### SEQUENCE LISTING

<110> Plant Research International
<120> Resistance against parasitic weeds
<130> unknown
<140> P217578PCT
   <141> 2006-03-14
<160> 50
<170> PatentIn version 3.3
<210> 1
   <211> 1233
   <212> DNA
   <213> Zea mays
<400> 1
   atggccatca tactcgtacg agcagcgtcg ccggggctct ccgccgccga cagcatcagc     60
   caccagggga ctctccagtg ctccaccctg ctcaagacga agaggccggc ggcgcgccgg    120
   tggatgccct gctcgctcct tggcctccac ccgtgggagg ctggccgtcc ctcccccgcc    180
   gtctactcca gcctcgccgt caacccggcg ggagaggccg tcgtctcgtc cgagcagaag    240
   gtctacgacg tcgtgctcaa gcaggccgca ttgctcaaac gccagctgcg cacgccggtc    300
   ctcgacgcca ggccccagga catggacatg ccacgcaacg ggctcaagga agcctacgac    360
   cgctgcggcg agatctgtga ggagtatgcc aagacgtttt acctcggaac tatgttgatg    420
   acagaggagc ggcgccgcgc catatgggcc atctatgtgt ggtgtaggag gacagatgag    480
   cttgtagatg ggccaaacgc caactacatt acaccaacag ctttggaccg gtgggagaag    540
   agacttgagg atctgttcac gggacgtcct tacgacatgc ttgatgccgc tctctctgat    600
   accatctcaa ggttccccat agacattcag ccattcaggg acatgattga agggatgagg    660
   agtgatctta ggaagacaag gtataacaac ttcgacgagc tctacatgta ctgctactat    720
   gttgctggaa ctgtcgggtt aatgagcgta cctgtgatgg gcatcgcaac cgagtctaaa    780
   gcaacaactg aaagcgtata cagtgctgcc ttggctctgg gaattgcgaa ccaactcacg    840
   aacatactcc gggatgttgg agaggatgct agaagaggaa ggatatattt accacaagat    900
   gagcttgcac aggcagggct ctctgatgag gacatcttca aaggggtcgt cacgaaccgg    960
   tggagaaact tcatgaagag gcagatcaag agggccagga tgtttttgga ggaggcagag   1020
   agaggggtaa ctgagctctc acaggctagc agatggccag tatgggcttc cctgttgttg   1080
   tacaggcaga tcctggatga gatcgaagcc aacgactaca acaacttcac gaagagggcg   1140
   tatgttggta aagggaagaa gttgctagca cttcctgtgg catatggaaa atcgctactg   1200
   ctcccatgtt cattgagaaa tggccagacc tag                                1233
<210> 2
   <211> 410
   <212> PRT
   <213> Zea mays
<400> 2
   Met Ala Ile Ile Leu Val Arg Ala Ala Ser Pro Gly Leu Ser Ala Ala
   1 5 10 15
   Asp Ser Ile Ser His Gln Gly Thr Leu Gln Cys Ser Thr Leu Leu Lys
   20 25 30
   Thr Lys Arg Pro Ala Ala Arg Arg Trp Met Pro Cys Ser Leu Leu Gly
   35 40 45
   Leu His Pro Trp Glu Ala Gly Arg Pro Ser Pro Ala Val Tyr Ser Ser
   50 55 60
   Leu Ala Val Asn Pro Ala Gly Glu Ala Val Val Ser Ser Glu Gln Lys
   65 70 75 80
   Val Tyr Asp Val Val Leu Lys Gln Ala Ala Leu Leu Lys Arg Gln Leu
   85 90 95
   Arg Thr Pro Val Leu Asp Ala Arg Pro Gln Asp Met Asp Met Pro Arg
   100 105 110
   Asn Gly Leu Lys Glu Ala Tyr Asp Arg Cys Gly Glu Ile Cys Glu Glu
   115 120 125
   Tyr Ala Lys Thr Phe Tyr Leu Gly Thr Met Leu Met Thr Glu Glu Arg
   130 135 140
   Arg Arg Ala Ile Trp Ala Ile Tyr Val Trp Cys Arg Arg Thr Asp Glu
   145 150 155 160
   Leu Val Asp Gly Pro Asn Ala Asn Tyr Ile Thr Pro Thr Ala Leu Asp
   165 170 175
   Arg Trp Glu Lys Arg Leu Glu Asp Leu Phe Thr Gly Arg Pro Tyr Asp
   180 185 190
   Met Leu Asp Ala Ala Leu Ser Asp Thr Ile Ser Arg Phe Pro Ile Asp
   195 200 205
   Ile Gln Pro Phe Arg Asp Met Ile Glu Gly Met Arg Ser Asp Leu Arg
   210 215 220
   Lys Thr Arg Tyr Asn Asn Phe Asp Glu Leu Tyr Met Tyr Cys Tyr Tyr
   225 230 235 240
   Val Ala Gly Thr Val Gly Leu Met Ser Val Pro Val Met Gly Ile Ala
   245 250 255
   Thr Glu Ser Lys Ala Thr Thr Glu Ser Val Tyr Ser Ala Ala Leu Ala
   260 265 270
   Leu Gly Ile Ala Asn Gln Leu Thr Asn Ile Leu Arg Asp Val Gly Glu
           275                 280                 285
   Asp Ala Arg Arg Gly Arg Ile Tyr Leu Pro Gln Asp Glu Leu Ala Gln
       290                 295                 300
   Ala Gly Leu Ser Asp Glu Asp Ile Phe Lys Gly Val Val Thr Asn Arg
   305                 310                 315                 320
   Trp Arg Asn Phe Met Lys Arg Gln Ile Lys Arg Ala Arg Met Phe Phe
                   325                 330                 335
   Glu Glu Ala Glu Arg Gly Val Thr Glu Leu Ser Gln Ala Ser Arg Trp
               340                 345                 350
   Pro Val Trp Ala Ser Leu Leu Leu Tyr Arg Gln Ile Leu Asp Glu Ile
           355                 360                 365
   Glu Ala Asn Asp Tyr Asn Asn Phe Thr Lys Arg Ala Tyr Val Gly Lys
       370                 375                 380
   Gly Lys Lys Leu Leu Ala Leu Pro Val Ala Tyr Gly Lys Ser Leu Leu
   385                 390                 395                 400
   Leu Pro Cys Ser Leu Arg Asn Gly Gln Thr
                   405                 410
<210> 3
   <211> 2264
   <212> DNA
   <213> Zea mays
<400> 3
   ctccaaatgc ggaggtctcg actcttctct cttcctccat ctttatcatc gccccacgta    60
   cacacccaat tcctcgcaac tgggctcccc cgcctccacg acactgcccc ccgtctcaag   120
   tccgccgcct ccattcttca gctctcctat cctccgccta gaatatcttc atcggtattt   180
   taccaacctg gatcaattta ctcacgatac tctgaagcgt atacatatgc catatgggaa   240
   atgacttcat agctgtgggt tgtcttatgg ctccttgaat ttgcagtagt ctgcctgtac   300
   ctattggctg aagcagagct gacccccact ttatcaagag ttgctcaacg atggacactg   360
   gctgcctgtc atctatgaat attactggag ctagccagac aagatctttt gcggggcaac   420
   ttcctcctca gagatgtttt gcgagtagtc actatacaag ctttgccgtg aaaaaacttg   480
   tctcaaggaa taaaggaagg agatcacacc gtagacatcc tgccttgcag gttgtctgca   540
   aggattttcc aagacctcca ctagaaagca caataaacta tttggaagct ggacagctct   600
   cttcattttt tagaaacagc gaacgcccca gtaagccgtt gcaggtcgtg gttgctggtg   660
   caggattggc tggtctatca acagcgaagt atctggcaga tgctggccat aaacccatat   720
   tgcttgaggc aagagatgtt ttgggtggaa aggtagctgc ttggaaggat gaagatggag   780
   attggtacga gactgggctt catatatttt ttggagctta tcccaacata cagaatctgt     840
   ttggcgagct taggattgag gatcgtttgc agtggaaaga acactctatg atattcgcca     900
   tgccaaacaa gccaggagaa ttcagccggt tcgatttccc agaaactttg ccagcaccta     960
   taaatgggat atgggccata ttgagaaaca atgaaatgct tacttggccg gagaaggtga    1020
   agtttgcaat cggacttctg ccagcaatgg ttggtggtca accttatgtt gaagctcaag    1080
   atggcttaac cgtttcagaa tggatgaaaa agcagggtgt tcctgatcgg gtgaacgatg    1140
   aggtttttat tgcaatgtcc aaggcactca atttcataaa tcctgatgag ctatctatgc    1200
   agtgcatttt gattgctttg aaccgatttc ttcaggagaa gcatggttct aaaatggcat    1260
   tcttggatgg taatccgcct gaaaggctat gcatgcctat tgttgatcac attcggtcta    1320
   ggggtggaga ggtccgcctg aattctcgta ttaaaaagat agagctgaat cctgatggaa    1380
   ctgtaaaaca cttcgcactt agtgatggaa ctcaaataac tggagatgct tatgtttgtg    1440
   caacaccagt cgatatcttc aagcttcttg tacctcaaga gtggagtgaa attacttatt    1500
   tcaagaaact ggagaagttg gtgggagttc ctgttatcaa tgttcatata tggtttgaca    1560
   gaaaactgaa caacacatat gaccaccttc ttttcagcag gagttcactt ttaagtgtct    1620
   atgcagacat gtcagtaacc tgcaaggaat actatgaccc aaaccgttca atgctggagt    1680
   tggtctttgc tcctgcagac gaatggattg gtcgaagtga cactgaaatc atcgatgcaa    1740
   ctatggaaga gctagccaag ttatttcctg atgaaattgc tgctgatcag agtaaagcaa    1800
   agattcttaa gtatcatatt gtgaagacac cgagatcggt ttacaaaact gtcccaaact    1860
   gtgagccttg ccggcctctc caaaggtcac ctatcgaagg tttctatcta gctggtgatt    1920
   acacaaagca gaaatacctg gcttctatgg aaggtgcagt cctatccggg aagctttgtg    1980
   cccagtccat agtgcaggat tatagcaggc tcgcactcag gagccagaaa agcctacaat    2040
   caggagaagt tcccgtccca tcttagttgt agttggcttt agctatcgtc atccccactg    2100
   ggtgctatct tatctcctat ttcaatggga acccacccaa tggtcatgtt ggagacaaca    2160
   cctgttatgg tcctttgacc atctcgtggt gactgtagtt gatgtcatat tcggatatat    2220
   atgtaaaagg acctgcatag caattgttag accttggaaa aaaa                     2264
<210> 4
   <211> 571
   <212> PRT
   <213> Zea mays
<400> 4
   Met Asp Thr Gly Cys Leu Ser Ser Met Asn Ile Thr Gly Ala Ser Gln
   1               5                   10                  15
   Thr Arg Ser Phe Ala Gly Gln Leu Pro Pro Gln Arg Cys Phe Ala Ser
               20                  25                  30
   Ser His Tyr Thr Ser Phe Ala Val Lys Lys Leu Val Ser Arg Asn Lys
           35                  40                  45
   Gly Arg Arg Ser His Arg Arg His Pro Ala Leu Gln Val Val Cys Lys
       50                  55                  60
   Asp Phe Pro Arg Pro Pro Leu Glu Ser Thr Ile Asn Tyr Leu Glu Ala
   65                  70                  75                  80
   Gly Gln Leu Ser Ser Phe Phe Arg Asn Ser Glu Arg Pro Ser Lys Pro
                   85                  90                  95
   Leu Gln Val Val Val Ala Gly Ala Gly Leu Ala Gly Leu Ser Thr Ala
               100                 105                 110
   Lys Tyr Leu Ala Asp Ala Gly His Lys Pro Ile Leu Leu Glu Ala Arg
           115                 120                 125
   Asp Val Leu Gly Gly Lys Val Ala Ala Trp Lys Asp Glu Asp Gly Asp
       130                 135                 140
   Trp Tyr Glu Thr Gly Leu His Ile Phe Phe Gly Ala Tyr Pro Asn Ile
   145                 150                 155                 160
   Gln Asn Leu Phe Gly Glu Leu Arg Ile Glu Asp Arg Leu Gln Trp Lys
                   165                 170                 175
   Glu His Ser Met Ile Phe Ala Met Pro Asn Lys Pro Gly Glu Phe Ser
               180                 185                 190
   Arg Phe Asp Phe Pro Glu Thr Leu Pro Ala Pro Ile Asn Gly Ile Trp
           195                 200                 205
   Ala Ile Leu Arg Asn Asn Glu Met Leu Thr Trp Pro Glu Lys Val Lys
       210                 215                 220
   Phe Ala Ile Gly Leu Leu Pro Ala Met Val Gly Gly Gln Pro Tyr Val
   225                 230                 235                 240
   Glu Ala Gln Asp Gly Leu Thr Val Ser Glu Trp Met Lys Lys Gln Gly
                   245                 250                 255
   Val Pro Asp Arg Val Asn Asp Glu Val Phe Ile Ala Met Ser Lys Ala
               260                 265                 270
   Leu Asn Phe Ile Asn Pro Asp Glu Leu Ser Met Gln Cys Ile Leu Ile
           275                 280                 285
   Ala Leu Asn Arg Phe Leu Gln Glu Lys His Gly Ser Lys Met Ala Phe
       290                 295                 300
   Leu Asp Gly Asn Pro Pro Glu Arg Leu Cys Met Pro Ile Val Asp His
   305                 310                 315                 320
   Ile Arg Ser Arg Gly Gly Glu Val Arg Leu Asn Ser Arg Ile Lys Lys
                   325                 330                 335
   Ile Glu Leu Asn Pro Asp Gly Thr Val Lys His Phe Ala Leu Ser Asp
               340                 345                 350
   Gly Thr Gln Ile Thr Gly Asp Ala Tyr Val Cys Ala Thr Pro Val Asp
           355                 360                 365
   Ile Phe Lys Leu Leu Val Pro Gln Glu Trp Ser Glu Ile Thr Tyr Phe
       370                 375                 380
   Lys Lys Leu Glu Lys Leu Val Gly Val Pro Val Ile Asn Val His Ile
   385                 390                 395                 400
   Trp Phe Asp Arg Lys Leu Asn Asn Thr Tyr Asp His Leu Leu Phe Ser
                   405                 410                 415
   Arg Ser Ser Leu Leu Ser Val Tyr Ala Asp Met Ser Val Thr Cys Lys
               420                 425                 430
   Glu Tyr Tyr Asp Pro Asn Arg Ser Met Leu Glu Leu Val Phe Ala Pro
           435                 440                 445
   Ala Asp Glu Trp Ile Gly Arg Ser Asp Thr Glu Ile Ile Asp Ala Thr
       450                 455                 460
   Met Glu Glu Leu Ala Lys Leu Phe Pro Asp Glu Ile Ala Ala Asp Gln
   465                 470                 475                 480
   Ser Lys Ala Lys Ile Leu Lys Tyr His Ile Val Lys Thr Pro Arg Ser
                   485                 490                 495
   Val Tyr Lys Thr Val Pro Asn Cys Glu Pro Cys Arg Pro Leu Gln Arg
               500                 505                 510
   Ser Pro Ile Glu Gly Phe Tyr Leu Ala Gly Asp Tyr Thr Lys Gln Lys
           515                 520                 525
   Tyr Leu Ala Ser Met Glu Gly Ala Val Leu Ser Gly Lys Leu Cys Ala
       530                 535                 540
   Gln Ser Ile Val Gln Asp Tyr Ser Arg Leu Ala Leu Arg Ser Gln Lys
   545                 550                 555                 560
   Ser Leu Gln Ser Gly Glu Val Pro Val Pro Ser
                   565                 570
<210> 5
   <211> 2265
   <212> DNA
   <213> Zea mays
<400> 5
   ccctgccacg acgcccgcga caaatccctg cgcgacggca tcttcgcctc ccatcccctc 60
   ccagcttccc ctcccactcc ggccctcaca caaattgccc ctcttcttct cctcctcttt 120
   acacgctgcc gaccacggct gccgccaacc acccgcccca cccgtccacc gctgccgagt 180
   gctagccatt tggagctgcc gcgccatggc gtccgtggcc gccaccacca cgctggcacc 240
   ggcactcgcc ccgcgccggg cgcggccagg gactgggctc gtgccgccgc gccgggcctc 300
   ggccgtcgct gctcgctcga ccgtaacgtc tccgacatgg cgtcaacgct cccaaaggtt 360
   attcccaccc gagccagagc actacagggg cccgaagctc aaggtggcca tcataggggc 420
   aggccttgcg ggcatgtcca ccgctgttga gctcttggac cagggccatg aggttgattt 480
   gtacgagtcc cgtccgttta tcggtggcaa ggttggctcc tttgttgaca ggcaaggaaa 540
   ccatatcgag atggggctgc atgtgttctt cgggtgctac agcaatctct tccgcctcat 600
   gaagaaggtt ggcgctgata ataatctgct ggtgaaggaa catacccata cttttgtaaa 660
   taaagggggc acgattggtg aacttgattt tcggttcccg gtgggagctc cgttacatgg 720
   cattcaagca ttcctaagaa ctaatcagct caaggtttat gataaagcaa gaaatgcagt 780
   tgctcttgcc cttagtccag ttgttcgggc tctggttgat cctgatggtg cattgcagca 840
   agtgcgggac ttggatgata taagtttcag tgattggttc atgtccaaag ggggtactcg 900
   ggagagtatc acaagaatgt gggatcctgt tcgttacgct ttgggtttca ttgactgtga 960
   taatatcagt gcacgttgca tgcttactat tttcaccttg tttgccacaa agacagaggc 1020
   atccctgtta cgcatgttaa agggttcacc tgatgtttac ttaagtggtc caataaagaa 1080
   gtatataaca gacaggggtg gtaggtttca cttaaggtgg ggatgcagag aggttctcta 1140
   tgagaagtca cctgatggag agacctatgt taagggcctt ctactcacca aggctacaag 1200
   tagagagata atcaaagctg atgcatacgt cgcagcctgt gatgttccag gtatcaaaag 1260
   attacttcca tcagaatgga gggagtggga aatgtttgac aatatctaca agttagatgg 1320
   tgtccctgtt gtcactgtcc agctccgcta caacggatgg gtcactgaac ttcaagattt 1380
   ggagaaatca agacaactgc aaagggcggt tgggttggat aaccttttgt acacggcgga 1440
   tgcagacttt tcctgttttt cggaccttgc tctctcatct cctgctgatt actacattga 1500
   agggcaaggt tccctgatcc aagctgtgct gactcctgga gatccataca tgccattgcc 1560
   aaacgaggag atcattagta aggttcaaaa gcaggttgta gaactgttcc catcttcccg 1620
   gggcttagaa gttacatggt ccagtgtggt aaagatcgga caatcgctgt accgtgaggc 1680
   tcctggaaac gacccattca ggcctgatca gaagacgccc gttaaaaact tcttcctctc 1740
   tggatcttac acgaaacagg actacatcga cagcatggaa ggagcaactc tctccggcag 1800
   gcgaacgtcg gcctacatct gcggtgccgg ggaggagctg ctggccctcc gaaagaagct 1860
   actcatcgac gacggcgaga aggcgctggg gaacgttcaa gtcctgcagg ctagctgaac 1920
   aacccctcct gcactgcaga gaagcttgga tctttccaac cacacataca tgctggaatg 1980
   gacaaaccaa ccaaccattg tcttttctcg cttcagggtg ctggcgattc ccgcagcaac 2040
   ctcctgtgta tcgtatccaa tttgagcatt agatctgccc cccccccctg caggcgtttc 2100
   tttcctatcc ctgatccgag aagcagggtg tagtctaggt ggctggcata cgggattaca 2160
   tcaggcagtg tgtaagttca gctggaactc gattggtaat tgggatggat gattgatgat 2220
   atatatatag cacacactgt tcttgcgtct tgcaaaaaaa aaaaa 2265
<210> 6
   <211> 570
   <212> PRT
   <213> Zea mays
<400> 6
   Met Ala Ser Val Ala Ala Thr Thr Thr Leu Ala Pro Ala Leu Ala Pro
   1               5                   10                  15
   Arg Arg Ala Arg Pro Gly Thr Gly Leu Val Pro Pro Arg Arg Ala Ser
               20                  25                  30
   Ala Val Ala Ala Arg Ser Thr Val Thr Ser Pro Thr Trp Arg Gln Arg
           35                  40                  45
   Ser Gln Arg Leu Phe Pro Pro Glu Pro Glu His Tyr Arg Gly Pro Lys
       50                  55                  60
   Leu Lys Val Ala Ile Ile Gly Ala Gly Leu Ala Gly Met Ser Thr Ala
   65                  70                  75                  80
   Val Glu Leu Leu Asp Gln Gly His Glu Val Asp Leu Tyr Glu Ser Arg
                   85                  90                  95
   Pro Phe Ile Gly Gly Lys Val Gly Ser Phe Val Asp Arg Gln Gly Asn
               100                 105                 110
   His Ile Glu Met Gly Leu His Val Phe Phe Gly Cys Tyr Ser Asn Leu
           115                 120                 125
   Phe Arg Leu Met Lys Lys Val Gly Ala Asp Asn Asn Leu Leu Val Lys
       130                 135                 140
   Glu His Thr His Thr Phe Val Asn Lys Gly Gly Thr Ile Gly Glu Leu
   145                 150                 155                 160
   Asp Phe Arg Phe Pro Val Gly Ala Pro Leu His Gly Ile Gln Ala Phe
                   165                 170                 175
   Leu Arg Thr Asn Gln Leu Lys Val Tyr Asp Lys Ala Arg Asn Ala Val
               180                 185                 190
   Ala Leu Ala Leu Ser Pro Val Val Arg Ala Leu Val Asp Pro Asp Gly
           195                 200                 205
   Ala Leu Gln Gln Val Arg Asp Leu Asp Asp Ile Ser Phe Ser Asp Trp
   210 215 220
   Phe Met Ser Lys Gly Gly Thr Arg Glu Ser Ile Thr Arg Met Trp Asp
   225 230 235 240
   Pro Val Arg Tyr Ala Leu Gly Phe Ile Asp Cys Asp Asn Ile Ser Ala
   245 250 255
   Arg Cys Met Leu Thr Ile Phe Thr Leu Phe Ala Thr Lys Thr Glu Ala
   260 265 270
   Ser Leu Leu Arg Met Leu Lys Gly Ser Pro Asp Val Tyr Leu Ser Gly
   275 280 285
   Pro Ile Lys Lys Tyr Ile Thr Asp Arg Gly Gly Arg Phe His Leu Arg
   290 295 300
   Trp Gly Cys Arg Glu Val Leu Tyr Glu Lys Ser Pro Asp Gly Glu Thr
   305 310 315 320
   Tyr Val Lys Gly Leu Leu Leu Thr Lys Ala Thr Ser Arg Glu Ile Ile
   325 330 335
   Lys Ala Asp Ala Tyr Val Ala Ala Cys Asp Val Pro Gly Ile Lys Arg
   340 345 350
   Leu Leu Pro Ser Glu Trp Arg Glu Trp Glu Met Phe Asp Asn Ile Tyr
   355 360 365
   Lys Leu Asp Gly Val Pro Val Val Thr Val Gln Leu Arg Tyr Asn Gly
   370 375 380
   Trp Val Thr Glu Leu Gln Asp Leu Glu Lys Ser Arg Gln Leu Gln Arg
   385 390 395 400
   Ala Val Gly Leu Asp Asn Leu Leu Tyr Thr Ala Asp Ala Asp Phe Ser
   405 410 415
   Cys Phe Ser Asp Leu Ala Leu Ser Ser Pro Ala Asp Tyr Tyr Ile Glu
   420 425 430
   Gly Gln Gly Ser Leu Ile Gln Ala Val Leu Thr Pro Gly Asp Pro Tyr
   435 440 445
   Met Pro Leu Pro Asn Glu Glu Ile Ile Ser Lys Val Gln Lys Gln Val
   450 455 460
   Val Glu Leu Phe Pro Ser Ser Arg Gly Leu Glu Val Thr Trp Ser Ser
   465 470 475 480
   Val Val Lys Ile Gly Gln Ser Leu Tyr Arg Glu Ala Pro Gly Asn Asp
   485 490 495
   Pro Phe Arg Pro Asp Gln Lys Thr Pro Val Lys Asn Phe Phe Leu Ser
               500                 505                 510
   Gly Ser Tyr Thr Lys Gln Asp Tyr Ile Asp Ser Met Glu Gly Ala Thr
           515                 520                 525
   Leu Ser Gly Arg Arg Thr Ser Ala Tyr Ile Cys Gly Ala Gly Glu Glu
       530                 535                 540
   Leu Leu Ala Leu Arg Lys Lys Leu Leu Ile Asp Asp Gly Glu Lys Ala
   545                 550                 555                 560
   Leu Gly Asn Val Gln Val Leu Gln Ala Ser
                   565                 570
<210> 7
   <211> 1848
   <212> DNA
   <213> Lycopersicon esculentum
<400> 7
   atgtgtacct tgagttttat gtatcctaat tcacttcttg atggtacctg caagactgta     60
   gctttgggtg atagcaaacc aagatacaat aaacagagaa gttcttgttt tgaccctttg     120
   ataattggaa attgtactga tcagcagcag ctttgtggct tgagttgggg ggtggacaag   180
   gctaagggaa gaagaggggg tactgtttcc aatttgaaag cagttgtaga tgtagacaaa   240
   agagtggaga gctatggcag tagtgatgta gaaggaaatg agagtggcag ctatgatgcc   300
   attgttatag gttcaggaat aggtggattg gtggcagcga cgcagctggc ggttaaggga   360
   gctaaggttt tagttctgga gaagtatgtt attcctggtg gaagctctgg cttttacgag   420
   agggatggtt ataagtttga tgttggttca tcagtgatgt ttggattcag tgataaggga   480
   aacctcaatt taattactca agcattggca gcagtaggac gtaaattaga agttatacct   540
   gacccaacaa ctgtacattt ccacctgcca aatgaccttt ctgttcgtat acaccgagag   600
   tatgatgact tcattgaaga gcttgtgagt aaatttccac atgaaaagga agggattatc   660
   aaattttaca gtgaatgctg gaagatcttt aattctctga attcattgga actgaagtct   720
   ttggaggaac ccatctacct ttttggccag ttctttaaga agccccttga atgcttgact   780
   cttgcctact atttgcccca gaatgctggt agcatcgctc ggaagtatat aagagatcct   840
   gggttgctgt cttttataga tgcagagtgc tttatcgtga gtacagttaa tgcattacaa   900
   acaccaatga tcaatgcaag catggttcta tgtgacagac attttggcgg aatcaactac   960
   cccgttggtg gagttggcga gatcgccaaa tccttagcaa aaggcttgga tgatcacgga  1020
   agtcagatac tttatagggc aaatgttaca agtatcattt tggacaatgg caaagctgtg  1080
   ggagtgaagc tttctgacgg gaggaagttt tatgctaaaa ccatagtatc gaatgctacc  1140
   agatgggata cttttggaaa gcttttaaaa gctgagaatc tgccaaaaga agaagaaaat  1200
   ttccagaaag cttatgtaaa agcaccttct tttctttcta ttcatatggg agttaaagca   1260
   gatgtactcc caccagacac agattgtcac cattttgtcc tcgaggatga ttggacaaat  1320
   ttggagaaac catatggaag tatattcttg agtattccaa cagttcttga ttcctcattg  1380
   gccccagaag gacaccatat tcttcacatt tttacaacat cgagcattga agattgggag  1440
   ggactctctc cgaaagacta tgaagcgaag aaagaggttg ttgctgaaag gattataagc  1500
   agacttgaaa aaacactctt cccagggctt aagtcatcta ttctctttaa ggaggtggga  1560
   actccaaaga cccacagacg ataccttgct cgtgatagtg gtacctatgg accaatgcca  1620
   cgcggaacac ctaagggact cctgggaatg cctttcaata ccactgctat agatggtcta  1680
   tattgtgttg gcgatagttg cttcccagga caaggtgtta tagctgtagc cttttcagga  1740
   gtaatgtgcg ctcatcgtgt tgcagctgac ttagggtttg aaaaaaaatc agatgtgctg  1800
   gacagtgctc ttcttagact acttggttgg ttaaggacac tagcatga                1848
<210> 8
   <211> 615
   <212> PRT
   <213> Lycopersicon esculentum
<400> 8
   Met Cys Thr Leu Ser Phe Met Tyr Pro Asn Ser Leu Leu Asp Gly Thr
   1               5                   10                  15
   Cys Lys Thr Val Ala Leu Gly Asp Ser Lys Pro Arg Tyr Asn Lys Gln
               20                  25                  30
   Arg Ser Ser Cys Phe Asp Pro Leu Ile Ile Gly Asn Cys Thr Asp Gln
           35                  40                  45
   Gln Gln Leu Cys Gly Leu Ser Trp Gly Val Asp Lys Ala Lys Gly Arg
       50                  55                  60
   Arg Gly Gly Thr Val Ser Asn Leu Lys Ala Val Val Asp Val Asp Lys
   65                  70                  75                  80
   Arg Val Glu Ser Tyr Gly Ser Ser Asp Val Glu Gly Asn Glu Ser Gly
                   85                  90                  95
   Ser Tyr Asp Ala Ile Val Ile Gly Ser Gly Ile Gly Gly Leu Val Ala
               100                 105                 110
   Ala Thr Gln Leu Ala Val Lys Gly Ala Lys Val Leu Val Leu Glu Lys
           115                 120                 125
   Tyr Val Ile Pro Gly Gly Ser Ser Gly Phe Tyr Glu Arg Asp Gly Tyr
       130                 135                 140
   Lys Phe Asp Val Gly Ser Ser Val Met Phe Gly Phe Ser Asp Lys Gly
   145                 150                 155                 160
   Asn Leu Asn Leu Ile Thr Gln Ala Leu Ala Ala Val Gly Arg Lys Leu
                   165                 170                 175
   Glu Val Ile Pro Asp Pro Thr Thr Val His Phe His Leu Pro Asn Asp
               180                 185                 190
   Leu Ser Val Arg Ile His Arg Glu Tyr Asp Asp Phe Ile Glu Glu Leu
           195                 200                 205
   Val Ser Lys Phe Pro His Glu Lys Glu Gly Ile Ile Lys Phe Tyr Ser
       210                 215                 220
   Glu Cys Trp Lys Ile Phe Asn Ser Leu Asn Ser Leu Glu Leu Lys Ser
   225                 230                 235                 240
   Leu Glu Glu Pro Ile Tyr Leu Phe Gly Gln Phe Phe Lys Lys Pro Leu
                   245                 250                 255
   Glu Cys Leu Thr Leu Ala Tyr Tyr Leu Pro Gln Asn Ala Gly Ser Ile
               260                 265                 270
   Ala Arg Lys Tyr Ile Arg Asp Pro Gly Leu Leu Ser Phe Ile Asp Ala
           275                 280                 285
   Glu Cys Phe Ile Val Ser Thr Val Asn Ala Leu Gln Thr Pro Met Ile
       290                 295                 300
   Asn Ala Ser Met Val Leu Cys Asp Arg His Phe Gly Gly Ile Asn Tyr
   305                 310                 315                 320
   Pro Val Gly Gly Val Gly Glu Ile Ala Lys Ser Leu Ala Lys Gly Leu
                   325                 330                 335
   Asp Asp His Gly Ser Gln Ile Leu Tyr Arg Ala Asn Val Thr Ser Ile
               340                 345                 350
   Ile Leu Asp Asn Gly Lys Ala Val Gly Val Lys Leu Ser Asp Gly Arg
           355                 360                 365
   Lys Phe Tyr Ala Lys Thr Ile Val Ser Asn Ala Thr Arg Trp Asp Thr
       370                 375                 380
   Phe Gly Lys Leu Leu Lys Ala Glu Asn Leu Pro Lys Glu Glu Glu Asn
   385                 390                 395                 400
   Phe Gln Lys Ala Tyr Val Lys Ala Pro Ser Phe Leu Ser Ile His Met
                   405                 410                 415
   Gly Val Lys Ala Asp Val Leu Pro Pro Asp Thr Asp Cys His His Phe
               420                 425                 430
   Val Leu Glu Asp Asp Trp Thr Asn Leu Glu Lys Pro Tyr Gly Ser Ile
   435 440 445
   Phe Leu Ser Ile Pro Thr Val Leu Asp Ser Ser Leu Ala Pro Glu Gly
   450 455 460
   His His Ile Leu His Ile Phe Thr Thr Ser Ser Ile Glu Asp Trp Glu
   465 470 475 480
   Gly Leu Ser Pro Lys Asp Tyr Glu Ala Lys Lys Glu Val Val Ala Glu
   485 490 495
   Arg Ile Ile Ser Arg Leu Glu Lys Thr Leu Phe Pro Gly Leu Lys Ser
   500 505 510
   Ser Ile Leu Phe Lys Glu Val Gly Thr Pro Lys Thr His Arg Arg Tyr
   515 520 525
   Leu Ala Arg Asp Ser Gly Thr Tyr Gly Pro Met Pro Arg Gly Thr Pro
   530 535 540
   Lys Gly Leu Leu Gly Met Pro Phe Asn Thr Thr Ala Ile Asp Gly Leu
   545 550 555 560
   Tyr Cys Val Gly Asp Ser Cys Phe Pro Gly Gln Gly Val Ile Ala Val
   565 570 575
   Ala Phe Ser Gly Val Met Cys Ala His Arg Val Ala Ala Asp Leu Gly
   580 585 590
   Phe Glu Lys Lys Ser Asp Val Leu Asp Ser Ala Leu Leu Arg Leu Leu
   595 600 605
   Gly Trp Leu Arg Thr Leu Ala
   610 615
<210> 9
   <211> 2276
   <212> DNA
   <213> Zea mays
<400> 9
   taggccagtc gcccagacac agagaggaaa catcgtctgg tcattgtaag gcctgaataa    60
   acatggggtc ggggcatggc aattcccggt aaccaccagc gcaatttgga cggtttgaaa   120
   ggtgacaagt agtgggggta agcttccgat gctgcgctcg tggtggttgt gaaggccggc   180
   gttgtctctt acctgcgaag gccggcgcag gatctatcgg tagaatcggc cagcagcgtt   240
   ttcgtggact tgtccttcgt ctttaaggcc aaaagtaata ttcaaaaaac atttgaggtg   300
   cttgggtgaa gaatgccaca atggaattgg atcgacggtg gtgcccccac tcaaagcagc   360
   tgcagcttat caccaccgcc tcaggctcag gattgcacgg ctccgccgcg ctcgaattcc   420
   ccccaccttc caccaggcca gcaccatggc cactaccgcc ctcctcctcc gcactcacca   480
   ccatccctgc aagccgccgg cgccgcgcgc gtccgtactc tgccgcgcca cggcggggat 540
   ggctgggccg gcgtcggccg cggcgctgcg gtccctggcc ccgcccacgc ggccggagct 600
   gctgtcgctc gacctgcccc gctacgaccc agcgcccgcc cgccccgtgg acctcgccgt 660
   ggtgggcggc gggcccgcgg gcctcgctgt ggcgcagcgc gtcgcggagg cgggcctgtc 720
   ggtgtgcgcc atcgacccgt cccccgcggt cgtgtggccc aacaactacg gcgtgtgggt 780
   ggacgagttc gaggccatgg gcctctccca ctgcctcgac accgtctggc cctccgcctc 840
   cgtcttcatc gacgatggcg gcgccaagtc gctcgaccgc ccctacgccc gcgtcgcgcg 900
   ccgcaagctc aagtccacca tgatggaccg ctgcgtcgcc aacggcgtcg tcttccacca 960
   ggccaaggtc gccaaggccg tccactacga cgcctcgtcc ctcctcatct gtgacgacgg 1020
   cgtcgccgtc ccggcaagcg tcgtgctcga cgccacgggc ttctcgcgct gcctcgtgca 1080
   gtacgacaag ccgtacaacc cggggtacca ggtcgcctac ggcatcctcg ccgaggtcga 1140
   cgcccacccg ttcgacatcg acaagatgct cttcatggac tggcgcgact cgcacctccc 1200
   cgaagggtcg gagatcaggg agcgcaaccg ccgcatcccc accttcctct acgccatgcc 1260
   cttctccccc accaggatct tcctcgagga gacgtccctg gtcgcgcgcc cggggctcgc 1320
   catggacgac atccaggagc gcatggccgc gcgcctcagg cacctgggta tacgcgtccg 1380
   aagcgtcgag gaggacgagc gctgcgtcat ccccatggga gggccgctgc ccgtcctgcc 1440
   gcagagggtt gtcggcatcg gcggcacggc agggatggtg cacccgtcca cgggctacat 1500
   ggtggcgcgc acgcttgcca ccgcgcctat cgtggcggac gccatcgtaa ggttcctcga 1560
   caccggcacc ggcaacggca tgggtggcct ggcaggggac gcgctctccg ccgaggtgtg 1620
   gaagcagctg tggccagcca acaggcggcg gcagagggag ttcttctgct tcggcatgga 1680
   cgtcctgctc aagctggacc tcgagggaac gcggcggttc ttcgacgcct tcttcgacct 1740
   ggagccacac tactggcacg gtttcctgtc atccagactg ttcctgccgg agctcttgat 1800
   gttcggcctc gcactgttcg ggaacgcctc caactcgtcg aggctggaga tcatggccaa 1860
   gggcaccgtg cctcttggca agatgattgg caacttgata caggacagag atgggtgagg 1920
   agggtatgta tacctacatt tttcacgtga agatctgatc tccattggat ctctgatttt 1980
   ggtatcgatg attttcactg tatttacgat ttgcaaagat ggattcacaa aacacagtta 2040
   gcaacagcag ttcaggacct cctgtcagat ataggaattg ctgctgcaac gctacttcag 2100
   tatggtgatt acagaggtgt atagttgcac ttgcacactg agggatgtcg tgagaatcta 2160
   cgtatcagat atcatggtct tcatataaaa gatcaaattt ccaacaaaat atgaaattga 2220
   cagttgtgta ttatgatagg gtgttttcga tatttcaaga acatagaaag aacggg 2276
<210> 10
   <211> 490
   <212> PRT
   <213> Zea mays
<400> 10
   Met Ala Thr Thr Ala Leu Leu Leu Arg Thr His His His Pro Cys Lys
   1 5 10 15
   Pro Pro Ala Pro Arg Ala Ser Val Leu Cys Arg Ala Thr Ala Gly Met
   20 25 30
   Ala Gly Pro Ala Ser Ala Ala Ala Leu Arg Ser Leu Ala Pro Pro Thr
   35 40 45
   Arg Pro Glu Leu Leu Ser Leu Asp Leu Pro Arg Tyr Asp Pro Ala Pro
   50 55 60
   Ala Arg Pro Val Asp Leu Ala Val Val Gly Gly Gly Pro Ala Gly Leu
   65 70 75 80
   Ala Val Ala Gln Arg Val Ala Glu Ala Gly Leu Ser Val Cys Ala Ile
   85 90 95
   Asp Pro Ser Pro Ala Val Val Trp Pro Asn Asn Tyr Gly Val Trp Val
   100 105 110
   Asp Glu Phe Glu Ala Met Gly Leu Ser His Cys Leu Asp Thr Val Trp
   115 120 125
   Pro Ser Ala Ser Val Phe Ile Asp Asp Gly Gly Ala Lys Ser Leu Asp
   130 135 140
   Arg Pro Tyr Ala Arg Val Ala Arg Arg Lys Leu Lys Ser Thr Met Met
   145 150 155 160
   Asp Arg Cys Val Ala Asn Gly Val Val Phe His Gln Ala Lys Val Ala
   165 170 175
   Lys Ala Val His Tyr Asp Ala Ser Ser Leu Leu Ile Cys Asp Asp Gly
   180 185 190
   Val Ala Val Pro Ala Ser Val Val Leu Asp Ala Thr Gly Phe Ser Arg
   195 200 205
   Cys Leu Val Gln Tyr Asp Lys Pro Tyr Asn Pro Gly Tyr Gln Val Ala
   210 215 220
   Tyr Gly Ile Leu Ala Glu Val Asp Ala His Pro Phe Asp Ile Asp Lys
   225 230 235 240
   Met Leu Phe Met Asp Trp Arg Asp Ser His Leu Pro Glu Gly Ser Glu
   245 250 255
   Ile Arg Glu Arg Asn Arg Arg Ile Pro Thr Phe Leu Tyr Ala Met Pro
   260 265 270
   Phe Ser Pro Thr Arg Ile Phe Leu Glu Glu Thr Ser Leu Val Ala Arg
   275 280 285
   Pro Gly Leu Ala Met Asp Asp Ile Gln Glu Arg Met Ala Ala Arg Leu
       290                 295                 300
   Arg His Leu Gly Ile Arg Val Arg Ser Val Glu Glu Asp Glu Arg Cys
   305                 310                 315                 320
   Val Ile Pro Met Gly Gly Pro Leu Pro Val Leu Pro Gln Arg Val Val
                   325                 330                 335
   Gly Ile Gly Gly Thr Ala Gly Met Val His Pro Ser Thr Gly Tyr Met
               340                 345                 350
   Val Ala Arg Thr Leu Ala Thr Ala Pro Ile Val Ala Asp Ala Ile Val
           355                 360                 365
   Arg Phe Leu Asp Thr Gly Thr Gly Asn Gly Met Gly Gly Leu Ala Gly
       370                 375                 380
   Asp Ala Leu Ser Ala Glu Val Trp Lys Gln Leu Trp Pro Ala Asn Arg
   385                 390                 395                 400
   Arg Arg Gln Arg Glu Phe Phe Cys Phe Gly Met Asp Val Leu Leu Lys
                   405                 410                 415
   Leu Asp Leu Glu Gly Thr Arg Arg Phe Phe Asp Ala Phe Phe Asp Leu
               420                 425                 430
   Glu Pro His Tyr Trp His Gly Phe Leu Ser Ser Arg Leu Phe Leu Pro
           435                 440                 445
   Glu Leu Leu Met Phe Gly Leu Ala Leu Phe Gly Asn Ala Ser Asn Ser
       450                 455                 460
   Ser Arg Leu Glu Ile Met Ala Lys Gly Thr Val Pro Leu Gly Lys Met
   465                 470                 475                 480
   Ile Gly Asn Leu Ile Gln Asp Arg Asp Gly
                   485                 490
<210> 11
   <211> 879
   <212> DNA
   <213> Oryza sativa
<400> 11
   atggccgtcg cgaggctggt ggtcatcacc cccgccgtcc tcctcggccg caccgcccgc     60
   gtctcgccgt cggcggtgcc gcggctgcgg cccatcgtcg ccggccgccg cgccgtggcg    120
   gcgcccacac gcgccgtcct gggagacggg gcgggtgtcg gcggcgagga ggatgcggtg    180
   gtggcggtgg tggaggagga cgcggtcgcc cggcgcgcgg cgaggaagcg gtcggagcgg    240
   cgcacgtacc tggtggcggc ggtgatgtcc agcctcgggt tcacgtccat ggccgccgcc    300
   gccgtctact accgcttcgc ctggcaaatg gaggccggcg gcggcgacgt tccggcgacg 360
   gagatggtcg gcacgttcgc gttgtcggtg ggggcggcgg tggggatgga gttctgggcg 420
   cggtgggcgc accgggcgct gtggcacgcg tcgctgtggc acatgcacga gtcgcaccac 480
   cgcccgcgcg acggcccgtt cgagctcaac gacgtcttcg ccatcgccaa cgccgccccg 540
   gccatctccc tcctcgccta cggcctcctc aaccgcggcc tcctccccgg cctctgcttc 600
   ggcgcggggc ttgggattac gctgttcggg atggcgtaca tgttcgtcca cgacggcctg 660
   gtccaccggc gcttccccgt ggggcccatc gagaacgtgc cctacttccg ccgagttgct 720
   gccgcccacc agatacatca cacggacaag ttcgaaggcg tgccctacgg cctgttcctc 780
   ggacccaagg agttggagga ggtgggtggg actgaggagc tggacaagga gatcaagaag 840
   aggatcaaga ggaaggaggc catggacgcc atcagatga 879
<210> 12
   <211> 292
   <212> PRT
   <213> Oryza sativa
<400> 12
   Met Ala Val Ala Arg Leu Val Val Ile Thr Pro Ala Val Leu Leu Gly
   1 5 10 15
   Arg Thr Ala Arg Val Ser Pro Ser Ala Val Pro Arg Leu Arg Pro Ile
   20 25 30
   Val Ala Gly Arg Arg Ala Val Ala Ala Pro Thr Arg Ala Val Leu Gly
   35 40 45
   Asp Gly Ala Gly Val Gly Gly Glu Glu Asp Ala Val Val Ala Val Val
   50 55 60
   Glu Glu Asp Ala Val Ala Arg Arg Ala Ala Arg Lys Arg Ser Glu Arg
   65 70 75 80
   Arg Thr Tyr Leu Val Ala Ala Val Met Ser Ser Leu Gly Phe Thr Ser
   85 90 95
   Met Ala Ala Ala Ala Val Tyr Tyr Arg Phe Ala Trp Gln Met Glu Ala
   100 105 110
   Gly Gly Gly Asp Val Pro Ala Thr Glu Met Val Gly Thr Phe Ala Leu
   115 120 125
   Ser Val Gly Ala Ala Val Gly Met Glu Phe Trp Ala Arg Trp Ala His
   130 135 140
   Arg Ala Leu Trp His Ala Ser Leu Trp His Met His Glu Ser His His
   145 150 155 160
   Arg Pro Arg Asp Gly Pro Phe Glu Leu Asn Asp Val Phe Ala Ile Ala
                   165                 170                 175
   Asn Ala Ala Pro Ala Ile Ser Leu Leu Ala Tyr Gly Leu Leu Asn Arg
               180                 185                 190
   Gly Leu Leu Pro Gly Leu Cys Phe Gly Ala Gly Leu Gly Ile Thr Leu
           195                 200                 205
   Phe Gly Met Ala Tyr Met Phe Val His Asp Gly Leu Val His Arg Arg
       210                 215                 220
   Phe Pro Val Gly Pro Ile Glu Asn Val Pro Tyr Phe Arg Arg Val Ala
   225                 230                 235                 240
   Ala Ala His Gln Ile His His Thr Asp Lys Phe Glu Gly Val Pro Tyr
                   245                 250                 255
   Gly Leu Phe Leu Gly Pro Lys Glu Leu Glu Glu Val Gly Gly Thr Glu
               260                 265                 270
   Glu Leu Asp Lys Glu Ile Lys Lys Arg Ile Lys Arg Lys Glu Ala Met
           275                 280                 285
   Asp Ala Ile Arg
       290
<210> 13
   <211> 1881
   <212> DNA
   <213> Oryza sativa
<400> 13
   atggcgctcc tctccgcgac cgcccccgcc aagacgcgct tctcgctctt ctcccacgag 60
   gaggcgcagc acccgcaccc gcacgcgctc tcggcgtgct gcggcggcgg cgccagcggc 120
   aagaggcagc gggcgcgggc cagggtggcg gcggcaatgc ggccggcgga cgcggccgcc 180
   tccgtcgcgc aggcggcgtc gccgggcggc ggcggcgagg gcacgcggag gccgcgggtg 240
   ctcgtggccg gcggcggcat cggggggctg gtgctggcgc tggcggcgag gcggaagggg 300
   tacgaggtga cggtgttcga gcgcgacatg agcgcggtgc gcggcgaggg gcagtaccgc 360
   gggccgatac agatccagag caacgcgctc gcggcgctgg cggccatcga catgtccgtc 420
   gccgaggagg tcatgcgcga aggctgcgtc accggcgacc gcatcaacgg cctcgtcgac 480
   ggcatctccg gctcctggta catcaagttt gatacattta ctcctgcagc tgagcgaggt 540
   ctcccagtta caagggttat tagccgaatg acgctgcagc agattcttgc tcgtgcggtt 600
   ggtgatgatg ctatactgaa cgatagccat gttgttgatt tcatagatga tggcaacaag 660
   gtaactgcaa ttttggagga tggccggaaa tttgaaggtg accttttggt tggtgctgat 720
   ggaatatggt caaaggtgag gaaggtgctt ttcgggcaat cagaagcaac ttattcagaa 780
   tatacttgct acactggcat tgcagacttt gtgcctcctg acattgacac agttgggtac 840
   cgtgtatttc ttggtcacaa acaatatttc gtctcctcag atgtcggtgc tggaaaaatg 900
   cagtggtatg catttcataa ggaacctgct ggtggcactg atcctgaaaa tggtaaaaat 960
   aaaagattgc tcgagatatt taatggttgg tgcgataacg tcgttgatct gataaatgca 1020
   actgatgagg aagcaattct tcgccgggat atatatgacc gaccacctac ttttaactgg 1080
   ggaaaaggtc gtgtcacttt gctaggtgac tctgtacatg ctatgcagcc aaatctgggt 1140
   caaggtggct gcatggctat tgaggatggt taccagctgg ctgtagaact tgagaagtcc 1200
   tggcaggaga gtgcgaagtc tggaactcct atggatatag tttcctcctt gaggagatat 1260
   gagaaggaga gaatactacg tgtttctgtt atacatgggt tggcaagaat ggcagcaatc 1320
   atggctacca cttatagacc atacttgggt gtgggtctgg gaccattgtc gtttttaacg 1380
   aagttgagga taccacatcc tggaagagtt ggtgggagat tcttcatcaa gtatggaatg 1440
   cctttgatgt tgagctgggt tctaggagga aacagcacga agttagaagg aagaccgtta 1500
   agctgtaggc tttctgacaa ggcaaacgac cagcttcgtc gatggtttga ggatgacgat 1560
   gcattggaac aagccatggg tggagagtgg tacctcctcc ccacaagttc tggagactcg 1620
   caacccattc gattaatcag ggatgaaaaa aagtcactct ccattggaag ccggtcagat 1680
   cccagcaatt cgactgcttc cctggcattg cccttgccac agatatcaga aaaccatgct 1740
   actatcacat gcaagaataa ggccttttat gtgactgata atggaagtga acatggtaca 1800
   tggattaccg acaacgaagg tagacgctat cggcgtacct ccgaacttcc ctgtccgttt 1860
   ccatccctcg gatgccattg a 1881
<210> 14
   <211> 626
   <212> PRT
   <213> Oryza sativa
<400> 14
   Met Ala Leu Leu Ser Ala Thr Ala Pro Ala Lys Thr Arg Phe Ser Leu
   1               5                   10                  15
   Phe Ser His Glu Glu Ala Gln His Pro His Pro His Ala Leu Ser Ala
               20                  25                  30
   Cys Cys Gly Gly Gly Ala Ser Gly Lys Arg Gln Arg Ala Arg Ala Arg
           35                  40                  45
   Val Ala Ala Ala Met Arg Pro Ala Asp Ala Ala Ala Ser Val Ala Gln
       50                  55                  60
   Ala Ala Ser Pro Gly Gly Gly Gly Glu Gly Thr Arg Arg Pro Arg Val
   65                  70                  75                  80
   Leu Val Ala Gly Gly Gly Ile Gly Gly Leu Val Leu Ala Leu Ala Ala
                   85                  90                  95
   Arg Arg Lys Gly Tyr Glu Val Thr Val Phe Glu Arg Asp Met Ser Ala
   100 105 110
   Val Arg Gly Glu Gly Gln Tyr Arg Gly Pro Ile Gln Ile Gln Ser Asn
   115 120 125
   Ala Leu Ala Ala Leu Ala Ala Ile Asp Met Ser Val Ala Glu Glu Val
   130 135 140
   Met Arg Glu Gly Cys Val Thr Gly Asp Arg Ile Asn Gly Leu Val Asp
   145 150 155 160
   Gly Ile Ser Gly Ser Trp Tyr Ile Lys Phe Asp Thr Phe Thr Pro Ala
   165 170 175
   Ala Glu Arg Gly Leu Pro Val Thr Arg Val Ile Ser Arg Met Thr Leu
   180 185 190
   Gln Gln Ile Leu Ala Arg Ala Val Gly Asp Asp Ala Ile Leu Asn Asp
   195 200 205
   Ser His Val Val Asp Phe Ile Asp Asp Gly Asn Lys Val Thr Ala Ile
   210 215 220
   Leu Glu Asp Gly Arg Lys Phe Glu Gly Asp Leu Leu Val Gly Ala Asp
   225 230 235 240
   Gly Ile Trp Ser Lys Val Arg Lys Val Leu Phe Gly Gln Ser Glu Ala
   245 250 255
   Thr Tyr Ser Glu Tyr Thr Cys Tyr Thr Gly Ile Ala Asp Phe Val Pro
   260 265 270
   Pro Asp Ile Asp Thr Val Gly Tyr Arg Val Phe Leu Gly His Lys Gln
   275 280 285
   Tyr Phe Val Ser Ser Asp Val Gly Ala Gly Lys Met Gln Trp Tyr Ala
   290 295 300
   Phe His Lys Glu Pro Ala Gly Gly Thr Asp Pro Glu Asn Gly Lys Asn
   305 310 315 320
   Lys Arg Leu Leu Glu Ile Phe Asn Gly Trp Cys Asp Asn Val Val Asp
   325 330 335
   Leu Ile Asn Ala Thr Asp Glu Glu Ala Ile Leu Arg Arg Asp Ile Tyr
   340 345 350
   Asp Arg Pro Pro Thr Phe Asn Trp Gly Lys Gly Arg Val Thr Leu Leu
   355 360 365
   Gly Asp Ser Val His Ala Met Gln Pro Asn Leu Gly Gln Gly Gly Cys
   370 375 380
   Met Ala Ile Glu Asp Gly Tyr Gln Leu Ala Val Glu Leu Glu Lys Ser
   385 390 395 400
   Trp Gln Glu Ser Ala Lys Ser Gly Thr Pro Met Asp Ile Val Ser Ser
   405 410 415
   Leu Arg Arg Tyr Glu Lys Glu Arg Ile Leu Arg Val Ser Val Ile His
   420 425 430
   Gly Leu Ala Arg Met Ala Ala Ile Met Ala Thr Thr Tyr Arg Pro Tyr
   435 440 445
   Leu Gly Val Gly Leu Gly Pro Leu Ser Phe Leu Thr Lys Leu Arg Ile
   450 455 460
   Pro His Pro Gly Arg Val Gly Gly Arg Phe Phe Ile Lys Tyr Gly Met
   465 470 475 480
   Pro Leu Met Leu Ser Trp Val Leu Gly Gly Asn Ser Thr Lys Leu Glu
   485 490 495
   Gly Arg Pro Leu Ser Cys Arg Leu Ser Asp Lys Ala Asn Asp Gln Leu
   500 505 510
   Arg Arg Trp Phe Glu Asp Asp Asp Ala Leu Glu Gln Ala Met Gly Gly
   515 520 525
   Glu Trp Tyr Leu Leu Pro Thr Ser Ser Gly Asp Ser Gln Pro Ile Arg
   530 535 540
   Leu Ile Arg Asp Glu Lys Lys Ser Leu Ser Ile Gly Ser Arg Ser Asp
   545 550 555 560
   Pro Ser Asn Ser Thr Ala Ser Leu Ala Leu Pro Leu Pro Gln Ile Ser
   565 570 575
   Glu Asn His Ala Thr Ile Thr Cys Lys Asn Lys Ala Phe Tyr Val Thr
   580 585 590
   Asp Asn Gly Ser Glu His Gly Thr Trp Ile Thr Asp Asn Glu Gly Arg
   595 600 605
   Arg Tyr Arg Arg Thr Ser Glu Leu Pro Cys Pro Phe Pro Ser Leu Gly
   610 615 620
   Cys His
   625
<210> 15
   <211> 1497
   <212> DNA
   <213> Solanum tuberosum
<400> 15
   atggaaaccc ttctaaagcc tttgacatct cttttacttt cctctcctac accacacagg     60
   tctattttcc aacaaaatcc cccttctcta aatcccacca ccaaaaaaaa atcaagaaaa    120
   tgtcatttta gaaacgaaag cagtaaactt ttttgtagct ttcttgattt agcacccata    180
   tcaaagccag agtcttttga tgttaacatc tcattggttg atcctaattc aggtcgggct    240
   caattcgacg tgatcatcat cggagctggc cctgctggtc tcaggctagc tgaacacgtt    300
   tctaaatatg gtattaaggt atgttgtgtt gacccttcac cactttccat gtggccaaat    360
   aattatggtg tttgggttga tgagtttgag aatttaggat tggaagattg tttagatcat    420
   aaatggccta tgacttgtgt acatataaat gatcataaaa ctaagtattt gggaagacca    480
   tatggtagag taagtagaaa gaagctgaag ttgagattgt tgaatagttg tgttgaaaac    540
   agagtgaagt tttataaagc taaggtttgg aaagtggaac atgaagaatt tgagtcttca    600
   attgtttgtg atgatggtaa gaagataaga ggtagtttgg ttgttgatgc aagtggtttt    660
   gctagtgatt ttatagagta tgacaagcca agaaaccatg gttatcaaat tgctcatggg    720
   gttttagtag aagttgataa tcatccattt gatttggata aaatggtgct tatggattgg    780
   agggattctc atttaggtaa tgagccatat ttaagggtga ataatgctaa agaaccaaca    840
   ttcttgtatg caatgccatt tgatagaaat ttggttttct tggaggagac ttctttggtg    900
   agtcgtcctg tgttatcgta tatggaagta aaaagaagga tggtagcaag attaaggcat    960
   ttggggatca aagtgagaag tgttattgag gaagagaaat gtgtgatccc tatgggagga   1020
   ccacttccgc ggattcctca aaatgttatg gctattggtg ggaattcggg gatagttcat   1080
   ccatcgacag ggtacatggt ggctaggagc atggctttgg caccagtact ggctgaagcc   1140
   atcgtcaagg ggcttggctc aacaagaatg ataagagggt ctcaacttta ccatagagtt   1200
   tggaatggtt tgtggccttt ggatagaaga tgtattggag aatgttattc atttgggatg   1260
   gagacattgt tgaagcttga tttgaaaggg actaggagat tgtttgatgc tttctttgat   1320
   cttgatccta aatactggca agggttcctt tcttcaagat tgtctgtcaa agaacttgct   1380
   atactcagct tgtgtctttt tggacatggc tcaaatttga ctaggttgga tattgttaca   1440
   aaatgtcctg ttcctttggt tagactgatt ggcaatctag caatagagag cctttga      1497
<210> 16
   <211> 498
   <212> PRT
   <213> Solanum tuberosum
<400> 16
   Met Glu Thr Leu Leu Lys Pro Leu Thr Ser Leu Leu Leu Ser Ser Pro
   1               5                   10                  15
   Thr Pro His Arg Ser Ile Phe Gln Gln Asn Pro Pro Ser Leu Asn Pro
               20                  25                  30
   Thr Thr Lys Lys Lys Ser Arg Lys Cys His Phe Arg Asn Glu Ser Ser
   35 40 45
   Lys Leu Phe Cys Ser Phe Leu Asp Leu Ala Pro Ile Ser Lys Pro Glu
   50 55 60
   Ser Phe Asp Val Asn Ile Ser Leu Val Asp Pro Asn Ser Gly Arg Ala
   65 70 75 80
   Gln Phe Asp Val Ile Ile Ile Gly Ala Gly Pro Ala Gly Leu Arg Leu
   85 90 95
   Ala Glu His Val Ser Lys Tyr Gly Ile Lys Val Cys Cys Val Asp Pro
   100 105 110
   Ser Pro Leu Ser Met Trp Pro Asn Asn Tyr Gly Val Trp Val Asp Glu
   115 120 125
   Phe Glu Asn Leu Gly Leu Glu Asp Cys Leu Asp His Lys Trp Pro Met
   130 135 140
   Thr Cys Val His Ile Asn Asp His Lys Thr Lys Tyr Leu Gly Arg Pro
   145 150 155 160
   Tyr Gly Arg Val Ser Arg Lys Lys Leu Lys Leu Arg Leu Leu Asn Ser
   165 170 175
   Cys Val Glu Asn Arg Val Lys Phe Tyr Lys Ala Lys Val Trp Lys Val
   180 185 190
   Glu His Glu Glu Phe Glu Ser Ser Ile Val Cys Asp Asp Gly Lys Lys
   195 200 205
   Ile Arg Gly Ser Leu Val Val Asp Ala Ser Gly Phe Ala Ser Asp Phe
   210 215 220
   Ile Glu Tyr Asp Lys Pro Arg Asn His Gly Tyr Gln Ile Ala His Gly
   225 230 235 240
   Val Leu Val Glu Val Asp Asn His Pro Phe Asp Leu Asp Lys Met Val
   245 250 255
   Leu Met Asp Trp Arg Asp Ser His Leu Gly Asn Glu Pro Tyr Leu Arg
   260 265 270
   Val Asn Asn Ala Lys Glu Pro Thr Phe Leu Tyr Ala Met Pro Phe Asp
   275 280 285
   Arg Asn Leu Val Phe Leu Glu Glu Thr Ser Leu Val Ser Arg Pro Val
   290 295 300
   Leu Ser Tyr Met Glu Val Lys Arg Arg Met Val Ala Arg Leu Arg His
   305                 310                 315                 320
   Leu Gly Ile Lys Val Arg Ser Val Ile Glu Glu Glu Lys Cys Val Ile
                   325                 330                 335
   Pro Met Gly Gly Pro Leu Pro Arg Ile Pro Gln Asn Val Met Ala Ile
               340                 345                 350
   Gly Gly Asn Ser Gly Ile Val His Pro Ser Thr Gly Tyr Met Val Ala
           355                 360                 365
   Arg Ser Met Ala Leu Ala Pro Val Leu Ala Glu Ala Ile Val Lys Gly
       370                 375                 380
   Leu Gly Ser Thr Arg Met Ile Arg Gly Ser Gln Leu Tyr His Arg Val
   385                 390                 395                 400
   Trp Asn Gly Leu Trp Pro Leu Asp Arg Arg Cys Ile Gly Glu Cys Tyr
                   405                 410                 415
   Ser Phe Gly Met Glu Thr Leu Leu Lys Leu Asp Leu Lys Gly Thr Arg
               420                 425                 430
   Arg Leu Phe Asp Ala Phe Phe Asp Leu Asp Pro Lys Tyr Trp Gln Gly
           435                 440                 445
   Phe Leu Ser Ser Arg Leu Ser Val Lys Glu Leu Ala Ile Leu Ser Leu
       450                 455                 460
   Cys Leu Phe Gly His Gly Ser Asn Leu Thr Arg Leu Asp Ile Val Thr
   465                 470                 475                 480
   Lys Cys Pro Val Pro Leu Val Arg Leu Ile Gly Asn Leu Ala Ile Glu
                   485                 490                 495
   Ser Leu
<210> 17
   <211> 2498
   <212> DNA
   <213> Zea mays
<400> 17
   caacaacaga ctacggagga ccgccgccgc ccacgcaaaa acccacccga tccccgcaaa 60
   aacccccgca cgcgatccac gccaattcca gtcaccgcga tgcagggtct cgccccgccc 120
   acctctgttt ccatacaccg gcacctgccg gcccggtcca gggcccgggc ctccaattcc 180
   gtcaggttct cgccgcgcgc cgtcagctcc gtgccgcccg ccgagtgcct ccaggcgccg 240
   ttccacaagc ccgtcgccga cctgcctgcg ccgtccagga agcccgccgc cattgccgtc 300
   ccagggcacg ccgcggcgcc gaggaaagcg gagggcggca agaagcagct caacttgttc 360
   cagcgcgccg cggcggccgc gctcgacgcg ttcgaggaag ggttcgtggc caacgtcctc 420
   gagcggcccc acgggctgcc cagcacggcc gacccggccg tgcagatcgc cggcaacttc 480
   gcgcccgtcg gggagaggcc gcccgtgcac gagctccccg tctccggccg catcccgccc 540
   ttcatcgacg gggtctacgc gcgcaacggc gccaacccct gcttcgaccc cgtcgcgggg 600
   caccacctct tcgacggcga cggcatggtg cacgcgctgc ggatacgcaa cggcgccgcc 660
   gagtcctacg cctgccgctt cacggagacc gcgcgcctgc gccaggagcg cgcgatcggc 720
   cgccccgtct tccccaaggc cattggcgag ctgcacgggc actccgggat cgcgcgcctc 780
   gccctgttct acgcgcgcgc cgcgtgcggc ctcgtggacc cctcggccgg caccggcgtg 840
   gccaacgccg gcctcgtcta cttcaacggc cgcctgctcg ccatgtccga ggacgacctc 900
   ccctaccacg tccgcgtggc ggacgacggc gacctcgaga ccgtcggccg ctacgacttc 960
   gacgggcagc tcggctgcgc catgatcgcg caccccaagc tggacccggc caccggggag 1020
   ctccacgcgc tcagctacga cgtcatcaag aggccgtacc tcaagtactt ctacttcagg 1080
   cccgacggca ccaagtccga cgacgtggag atcccgctgg agcagcccac gatgatccac 1140
   gacttcgcca tcaccgagaa cttcgtggtt gtgcccgacc accaggtggt gttcaagctc 1200
   caggagatgc tgcgcggcgg gtcgcccgtg gtgctggaca aggagaagac gtcgcggttc 1260
   ggcgtgctcc ccaagcacgc cgcggacgcg tcggagatgg cgtgggtgga cgtgccggac 1320
   tgcttctgct tccacctgtg gaacgcgtgg gaggacgagg cgacgggcga ggtggtggtg 1380
   atcggctcct gcatgacccc cgccgactcc atcttcaacg agtccgacga gcgcctggag 1440
   agcgtgctga ccgagatccg cctggacgcg cgcacgggcc ggtccacgcg ccgcgccgtc 1500
   ctgccgccgt cgcagcagga gaacctggag gtgggcatgg tgaaccgcaa cctgctgggc 1560
   cgcgagagcc ggtacgcgta cctcgcggtg gcggagccgt ggcccaagga gtcgggcttc 1620
   gccaaggagg acctgtccac gggcgagctc accaagttcg agtacggcga gggccggttc 1680
   ggcggcgagc cctgcttcgt tcccatggac ccggccgcgg cccacccgcg cggcgaggac 1740
   gacgggtacg tgctcacctt cgtccacgac gagcgcgccg gcacgtcgga gctacttgtg 1800
   gtcaatgccg ccgacatccg gctggaggcc acggttcagc tgccgtcccg cgtgcccttc 1860
   ggcttccacg gcaccttcat cacgggccag gagctcgagg cccaggcggc ctgaccgagc 1920
   tccacgtttc tcggaggagg aacagaggag ccagccttgg atcaggggag aagtcaccag 1980
   agggagccca gatcagttcc ccggggtctt cctgtccccc cacgcacacc agagttacag 2040
   ttggtagttg ctttgctttg ccttttttta acattatttc atttgacact agtgtaagta 2100
   aattggtata gtggcagctt agagagagag agagattagt agaaaggcgc ccagctcgta 2160
   gcttaacagc tggtggtgcc tctggctagt atagagtaca tatttttttt tcttcttctg 2220
   tgctttatcc cttttttttt tctccctttg gatgacatgg atgtgcatcc agctccagtt 2280
   gttgtcaccc agtccagtgg actggggtgt ctgggatctt ggttgctgtt acttgctcgc 2340
   cattgccacc cagttgctgc cgctgctgct gaggtgttgc tggcttggct tgtatactct 2400
   gtgttccctg tggctatcgg tgcgtgtaca tttgttcaca gtatatgact gatggtatta 2460
   aaataagaac cggtgacggc ttctgtttca aaaaaaaa 2498
<210> 18
   <211> 604
   <212> PRT
   <213> Zea mays
<400> 18
   Met Gln Gly Leu Ala Pro Pro Thr Ser Val Ser Ile His Arg His Leu
   1 5 10 15
   Pro Ala Arg Ser Arg Ala Arg Ala Ser Asn Ser Val Arg Phe Ser Pro
   20 25 30
   Arg Ala Val Ser Ser Val Pro Pro Ala Glu Cys Leu Gln Ala Pro Phe
   35 40 45
   His Lys Pro Val Ala Asp Leu Pro Ala Pro Ser Arg Lys Pro Ala Ala
   50 55 60
   Ile Ala Val Pro Gly His Ala Ala Ala Pro Arg Lys Ala Glu Gly Gly
   65 70 75 80
   Lys Lys Gln Leu Asn Leu Phe Gln Arg Ala Ala Ala Ala Ala Leu Asp
   85 90 95
   Ala Phe Glu Glu Gly Phe Val Ala Asn Val Leu Glu Arg Pro His Gly
   100 105 110
   Leu Pro Ser Thr Ala Asp Pro Ala Val Gln Ile Ala Gly Asn Phe Ala
   115 120 125
   Pro Val Gly Glu Arg Pro Pro Val His Glu Leu Pro Val Ser Gly Arg
   130 135 140
   Ile Pro Pro Phe Ile Asp Gly Val Tyr Ala Arg Asn Gly Ala Asn Pro
   145 150 155 160
   Cys Phe Asp Pro Val Ala Gly His His Leu Phe Asp Gly Asp Gly Met
   165 170 175
   Val His Ala Leu Arg Ile Arg Asn Gly Ala Ala Glu Ser Tyr Ala Cys
   180 185 190
   Arg Phe Thr Glu Thr Ala Arg Leu Arg Gln Glu Arg Ala Ile Gly Arg
   195 200 205
   Pro Val Phe Pro Lys Ala Ile Gly Glu Leu His Gly His Ser Gly Ile
   210 215 220
   Ala Arg Leu Ala Leu Phe Tyr Ala Arg Ala Ala Cys Gly Leu Val Asp
   225 230 235 240
   Pro Ser Ala Gly Thr Gly Val Ala Asn Ala Gly Leu Val Tyr Phe Asn
   245 250 255
   Gly Arg Leu Leu Ala Met Ser Glu Asp Asp Leu Pro Tyr His Val Arg
   260 265 270
   Val Ala Asp Asp Gly Asp Leu Glu Thr Val Gly Arg Tyr Asp Phe Asp
   275 280 285
   Gly Gln Leu Gly Cys Ala Met Ile Ala His Pro Lys Leu Asp Pro Ala
   290 295 300
   Thr Gly Glu Leu His Ala Leu Ser Tyr Asp Val Ile Lys Arg Pro Tyr
   305 310 315 320
   Leu Lys Tyr Phe Tyr Phe Arg Pro Asp Gly Thr Lys Ser Asp Asp Val
   325 330 335
   Glu Ile Pro Leu Glu Gln Pro Thr Met Ile His Asp Phe Ala Ile Thr
   340 345 350
   Glu Asn Phe Val Val Val Pro Asp His Gln Val Val Phe Lys Leu Gln
   355 360 365
   Glu Met Leu Arg Gly Gly Ser Pro Val Val Leu Asp Lys Glu Lys Thr
   370 375 380
   Ser Arg Phe Gly Val Leu Pro Lys His Ala Ala Asp Ala Ser Glu Met
   385 390 395 400
   Ala Trp Val Asp Val Pro Asp Cys Phe Cys Phe His Leu Trp Asn Ala
   405 410 415
   Trp Glu Asp Glu Ala Thr Gly Glu Val Val Val Ile Gly Ser Cys Met
   420 425 430
   Thr Pro Ala Asp Ser Ile Phe Asn Glu Ser Asp Glu Arg Leu Glu Ser
   435 440 445
   Val Leu Thr Glu Ile Arg Leu Asp Ala Arg Thr Gly Arg Ser Thr Arg
   450 455 460
   Arg Ala Val Leu Pro Pro Ser Gln Gln Glu Asn Leu Glu Val Gly Met
   465 470 475 480
   Val Asn Arg Asn Leu Leu Gly Arg Glu Ser Arg Tyr Ala Tyr Leu Ala
   485 490 495
   Val Ala Glu Pro Trp Pro Lys Glu Ser Gly Phe Ala Lys Glu Asp Leu
   500 505 510
   Ser Thr Gly Glu Leu Thr Lys Phe Glu Tyr Gly Glu Gly Arg Phe Gly
   515 520 525
   Gly Glu Pro Cys Phe Val Pro Met Asp Pro Ala Ala Ala His Pro Arg
   530 535 540
   Gly Glu Asp Asp Gly Tyr Val Leu Thr Phe Val His Asp Glu Arg Ala
   545 550 555 560
   Gly Thr Ser Glu Leu Leu Val Val Asn Ala Ala Asp Ile Arg Leu Glu
   565 570 575
   Ala Thr Val Gln Leu Pro Ser Arg Val Pro Phe Gly Phe His Gly Thr
   580 585 590
   Phe Ile Thr Gly Gln Glu Leu Glu Ala Gln Ala Ala
   595 600
<210> 19
   <211> 1928
   <212> DNA
   <213> Zea mays
<400> 19
   ggccactccc cttccatctc gtcccttcgc tacaagtcat ctcgccgcaa ccggaacccg 60
   cagccatggg gacggaggcg gagcagccgg acatggacag ccaccgaaac gacggcgtcg 120
   tggtggtgcc agcgccgcgc ccgcgtaagg ggctcgcctc ctgggcgctt gacctgctcg 180
   agtccctcgc cgtgcgcctc ggccacgaca agaccaagcc gctccactgg ctctccggca 240
   acttcgcccc cgtcgtcgag gagaccccgc cggccccaaa ccttagcgtc cgcggacacc 300
   tcccggagtg cttgaatgga gagtttgtca gggttgggcc taatccgaag tttgctcctg 360
   ttgcggggta tcactggttt gatggagacg ggatgattca tgccatgcgt attaaggatg 420
   gaaaagctac ctatgtatca agatatgtga agactgcccg cctcaaacaa gaggagtatt 480
   ttggtggagc aaagtttatg aagattggag accttaaggg attttttgga ttgtttatgg 540
   tccaaatgca gcaacttcgg aaaaaattca aagtcttgga ttttacctat ggatttggga 600
   cagctaatac tgcacttata tatcatcatg gtaaactcat ggccttgtca gaagcagata 660
   agccatatgt tgttaaggtc cttgaagatg gagacttgca gactcttggc ttgttggatt 720
   atgacaaaag gttgaaacat tcttttactg cccatccaaa ggttgaccct tttacagatg 780
   aaatgttcac attcggatat tcacatgaac ctccatactg tacataccgt gtgattaaca 840
   aagaaggagc tatgcttgat cctgtgccaa taacaatacc ggaatctgta atgatgcatg 900
   attttgccat cacagagaat tactctattt ttatggacct ccctttattg ttccgaccaa 960
   aggaaatggt gaagaacggt gagtttatct acaagtttga tcctacaaag aaaggtcgtt 1020
   ttggtattct cccccgctat gcaaaggatg acaaactcat cagatggttt caactcccta 1080
   attgtttcat attccataat gctaatgctt gggaagaggg tgatgaagtt gttctcatta 1140
   cctgccgcct tgagaatcca gatttggaca aggtgaatgg atatcaaagt gacaagctcg 1200
   aaaacttcgg gaatgagctg tacgagatga gattcaacat gaaaacgggt gctgcttcac 1260
   aaaagcaact gtctgtttct gctgtggatt ttcctcgtgt taatgagagc tatactggca 1320
   gaaagcagcg gtatgtctac tgcactatac ttgacagcat tgcgaaggtg actggcatca 1380
   taaagtttga tctgcatgct gaaccggaaa gtggtgtgaa agaacttgaa gtgggaggaa 1440
   atgtacaagg catatatgac ctgggacctg gtagatttgg ttcagaggca atttttgttc 1500
   ccaagcatcc aggtgtgtct ggagaagaag atgacggcta tttgatattc tttgtacacg 1560
   acgagaacac agggaaatct gaagtaaatg ttatcgatgc aaagacaatg tctgctgatc 1620
   cagttgcggt ggttgagctt cctaataggg ttccttatgg attccatgcc ttttttgtaa 1680
   ctgaggacca actggctcga caggcggagg ggcagtgaag atacggcacc tgcagattct 1740
   gcacacgcgg gtacaggttg gaaattattg cagaggacat gtatatgtat aggacaagtt 1800
   tattacacat gtattcgaac cacacattac acaagtttat tgcaggacgt gtattcgaac 1860
   cacacattac acatgtatat gtataggaca agtttattac agtcctgatt gattgcaatt 1920
   tgcaaatt 1928
<210> 20
   <211> 550
   <212> PRT
   <213> Zea mays
<400> 20
   Met Gly Thr Glu Ala Glu Gln Pro Asp Met Asp Ser His Arg Asn Asp
   1               5                   10                  15
   Gly Val Val Val Val Pro Ala Pro Arg Pro Arg Lys Gly Leu Ala Ser
               20                  25                  30
   Trp Ala Leu Asp Leu Leu Glu Ser Leu Ala Val Arg Leu Gly His Asp
           35                  40                  45
   Lys Thr Lys Pro Leu His Trp Leu Ser Gly Asn Phe Ala Pro Val Val
       50                  55                  60
   Glu Glu Thr Pro Pro Ala Pro Asn Leu Ser Val Arg Gly His Leu Pro
   65                  70                  75                  80
   Glu Cys Leu Asn Gly Glu Phe Val Arg Val Gly Pro Asn Pro Lys Phe
                   85                  90                  95
   Ala Pro Val Ala Gly Tyr His Trp Phe Asp Gly Asp Gly Met Ile His
               100                 105                 110
   Ala Met Arg Ile Lys Asp Gly Lys Ala Thr Tyr Val Ser Arg Tyr Val
           115                 120                 125
   Lys Thr Ala Arg Leu Lys Gln Glu Glu Tyr Phe Gly Gly Ala Lys Phe
       130                 135                 140
   Met Lys Ile Gly Asp Leu Lys Gly Phe Phe Gly Leu Phe Met Val Gln
   145                 150                 155                 160
   Met Gln Gln Leu Arg Lys Lys Phe Lys Val Leu Asp Phe Thr Tyr Gly
                   165                 170                 175
   Phe Gly Thr Ala Asn Thr Ala Leu Ile Tyr His His Gly Lys Leu Met
               180                 185                 190
   Ala Leu Ser Glu Ala Asp Lys Pro Tyr Val Val Lys Val Leu Glu Asp
           195                 200                 205
   Gly Asp Leu Gln Thr Leu Gly Leu Leu Asp Tyr Asp Lys Arg Leu Lys
       210                 215                 220
   His Ser Phe Thr Ala His Pro Lys Val Asp Pro Phe Thr Asp Glu Met
   225                 230                 235                 240
   Phe Thr Phe Gly Tyr Ser His Glu Pro Pro Tyr Cys Thr Tyr Arg Val
                   245                 250                 255
   Ile Asn Lys Glu Gly Ala Met Leu Asp Pro Val Pro Ile Thr Ile Pro
               260                 265                 270
   Glu Ser Val Met Met His Asp Phe Ala Ile Thr Glu Asn Tyr Ser Ile
           275                 280                 285
   Phe Met Asp Leu Pro Leu Leu Phe Arg Pro Lys Glu Met Val Lys Asn
       290                 295                 300
   Gly Glu Phe Ile Tyr Lys Phe Asp Pro Thr Lys Lys Gly Arg Phe Gly
   305                 310                 315                 320
   Ile Leu Pro Arg Tyr Ala Lys Asp Asp Lys Leu Ile Arg Trp Phe Gln
                   325                 330                 335
   Leu Pro Asn Cys Phe Ile Phe His Asn Ala Asn Ala Trp Glu Glu Gly
               340                 345                 350
   Asp Glu Val Val Leu Ile Thr Cys Arg Leu Glu Asn Pro Asp Leu Asp
           355                 360                 365
   Lys Val Asn Gly Tyr Gln Ser Asp Lys Leu Glu Asn Phe Gly Asn Glu
       370                 375                 380
   Leu Tyr Glu Met Arg Phe Asn Met Lys Thr Gly Ala Ala Ser Gln Lys
   385                 390                 395                 400
   Gln Leu Ser Val Ser Ala Val Asp Phe Pro Arg Val Asn Glu Ser Tyr
   405 410 415
   Thr Gly Arg Lys Gln Arg Tyr Val Tyr Cys Thr Ile Leu Asp Ser Ile
   420 425 430
   Ala Lys Val Thr Gly Ile Ile Lys Phe Asp Leu His Ala Glu Pro Glu
   435 440 445
   Ser Gly Val Lys Glu Leu Glu Val Gly Gly Asn Val Gln Gly Ile Tyr
   450 455 460
   Asp Leu Gly Pro Gly Arg Phe Gly Ser Glu Ala Ile Phe Val Pro Lys
   465 470 475 480
   His Pro Gly Val Ser Gly Glu Glu Asp Asp Gly Tyr Leu Ile Phe Phe
   485 490 495
   Val His Asp Glu Asn Thr Gly Lys Ser Glu Val Asn Val Ile Asp Ala
   500 505 510
   Lys Thr Met Ser Ala Asp Pro Val Ala Val Val Glu Leu Pro Asn Arg
   515 520 525
   Val Pro Tyr Gly Phe His Ala Phe Phe Val Thr Glu Asp Gln Leu Ala
   530 535 540
   Arg Gln Ala Glu Gly Gln
   545 550
<210> 21
   <211> 1827
   <212> DNA
   <213> Oryza sativa
<400> 21
   atggcgacga tcacgacgcc aggatatgct cacatacagc ggcagcacgg caggtgctcg      60
   acgacggcgg gaaggcgtgg ggcgtccaat tcggtgagat tctccgcgcg cgcggttagc     120
   tccgtgccgc acgcggcggc ggcgtcatcg gcgccggcgt tcctgccggt gccgttcgtg     180
   cctggggccg acgcaccgtc gccgtcgggg aagagtgcca ttggcgtccc gaaggcgccg     240
   aggaaggggg aggaggggaa gaggctcaac ttcttccagc gcgccgcggc gatggcgctc     300
   gacgcgttcg aggaggggtt tgtggcgaat gtcctcgagc gcccgcacgg gctgccgagc     360
   acggccgacc ccgcggtgca gatcgccggc aacttcgcgc cggtcggtga gacgccgccc     420
   gcgcgcgcgc tgccggtgtc ggggcgcatc ccgcccttca tcaacggcgt ctacgcgcgc     480
   aacggcgcca acccgcactt cgaccccgtc gccgggcacc acctgttcga cggcgatggc     540
   atggtgcacg ccgtcaggat acgcaacggc gccgccgagt cgtacgcgtg ccggttcacg     600
   gagaccgcgc ggctgcggca ggagcgcgcg atggggcggc ccatgttccc caaggccatt     660
   ggggagctcc atggccactc cggcatcgcg cgccttgctc tgttctacgc gcgcgccgcc     720
   tgcggcctcc tcgacccgtc acacggcacc ggcgtcgcca acgccggcct catctacttc     780
   aacggcaggc tcctcgccat gtcggaggac gacctcccct accaggtgcg cgtcaccgcc     840
   gacggcgacc tcgagaccgt cggccgctac gacttcgacg ggcagctcgg ctgcgccatg     900
   atcgcgcacc ccaagctcga cccggccacc ggagagctcc acgcgctcag ctacgacgtg     960
   atcaagaagc cgtacctcaa gtacttctac ttcgcgcccg acggcaccaa gtcggccgac    1020
   gtcgagatcc cgctcgacca gcccaccatg atccacgact tcgccatcac cgagaactac    1080
   gtggtggtac ccgaccacca ggtggtgttc aagctccagg agatgctccg cggcggctcg    1140
   cccgtggtgc tcgacaagga gaagacgtcg cggttcgggg tgctccccaa gcacgccgcg    1200
   gacgcgtcgg agatggtgtg ggtggacgtc ccggactgct tctgcttcca cctctggaac    1260
   gcgtgggagg aggcggacac cgacgaggtg gtggtgatcg gctcgtgcat gacccccgcc    1320
   gactccatct tcaacgagtc cgacgaccgc ctcgagagcg tcctcaccga gatccgcctc    1380
   aacacccgca ccggcgagtc gacgcggcgc gccatcctgc cgccgtcgag ccaggtcaac    1440
   ctcgaggtgg gcatggtcaa ccgcaacctc ctcggccgca agacgcggta cgcctacctc    1500
   gccgtggccg agccgtggcc caaggtgtcg ggcttcgcca aggtggacct cgccacgggt    1560
   gagctcacca agttcgagta cggcgagggc cggttcggcg gcgagccctg cttcgtcccc    1620
   atggacgccg ccgccgccac gccccgcggc gaggacgacg gctacatcct gtccttcgtc    1680
   cacgacgagc gcgccgggac ctccgagctc ctcgtcgtca atgccgccga catgcgcctt    1740
   gaggccaccg tgcagctgcc gtcccgcgtg ccgtacggct tccacggcac gttcatcacc    1800
   ggcgacgagc tcaccaccca ggcctga                                        1827
<210> 22
   <211> 608
   <212> PRT
   <213> Oryza sativa
<400> 22
   Met Ala Thr Ile Thr Thr Pro Gly Tyr Ala His Ile Gln Arg Gln His
   1               5                   10                  15
   Gly Arg Cys Ser Thr Thr Ala Gly Arg Arg Gly Ala Ser Asn Ser Val
               20                  25                  30
   Arg Phe Ser Ala Arg Ala Val Ser Ser Val Pro His Ala Ala Ala Ala
           35                  40                  45
   Ser Ser Ala Pro Ala Phe Leu Pro Val Pro Phe Val Pro Gly Ala Asp
       50                  55                  60
   Ala Pro Ser Pro Ser Gly Lys Ser Ala Ile Gly Val Pro Lys Ala Pro
   65                  70                  75                  80
   Arg Lys Gly Glu Glu Gly Lys Arg Leu Asn Phe Phe Gln Arg Ala Ala
                   85                  90                  95
   Ala Met Ala Leu Asp Ala Phe Glu Glu Gly Phe Val Ala Asn Val Leu
               100                 105                 110
   Glu Arg Pro His Gly Leu Pro Ser Thr Ala Asp Pro Ala Val Gln Ile
           115                 120                 125
   Ala Gly Asn Phe Ala Pro Val Gly Glu Thr Pro Pro Ala Arg Ala Leu
       130                 135                 140
   Pro Val Ser Gly Arg Ile Pro Pro Phe Ile Asn Gly Val Tyr Ala Arg
   145                 150                 155                 160
   Asn Gly Ala Asn Pro His Phe Asp Pro Val Ala Gly His His Leu Phe
                   165                 170                 175
   Asp Gly Asp Gly Met Val His Ala Val Arg Ile Arg Asn Gly Ala Ala
               180                 185                 190
   Glu Ser Tyr Ala Cys Arg Phe Thr Glu Thr Ala Arg Leu Arg Gln Glu
           195                 200                 205
   Arg Ala Met Gly Arg Pro Met Phe Pro Lys Ala Ile Gly Glu Leu His
       210                 215                 220
   Gly His Ser Gly Ile Ala Arg Leu Ala Leu Phe Tyr Ala Arg Ala Ala
   225                 230                 235                 240
   Cys Gly Leu Leu Asp Pro Ser His Gly Thr Gly Val Ala Asn Ala Gly
                   245                 250                 255
   Leu Ile Tyr Phe Asn Gly Arg Leu Leu Ala Met Ser Glu Asp Asp Leu
               260                 265                 270
   Pro Tyr Gln Val Arg Val Thr Ala Asp Gly Asp Leu Glu Thr Val Gly
           275                 280                 285
   Arg Tyr Asp Phe Asp Gly Gln Leu Gly Cys Ala Met Ile Ala His Pro
       290                 295                 300
   Lys Leu Asp Pro Ala Thr Gly Glu Leu His Ala Leu Ser Tyr Asp Val
   305                 310                 315                 320
   Ile Lys Lys Pro Tyr Leu Lys Tyr Phe Tyr Phe Ala Pro Asp Gly Thr
                   325                 330                 335
   Lys Ser Ala Asp Val Glu Ile Pro Leu Asp Gln Pro Thr Met Ile His
               340                 345                 350
   Asp Phe Ala Ile Thr Glu Asn Tyr Val Val Val Pro Asp His Gln Val
           355                 360                 365
   Val Phe Lys Leu Gln Glu Met Leu Arg Gly Gly Ser Pro Val Val Leu
   370 375 380
   Asp Lys Glu Lys Thr Ser Arg Phe Gly Val Leu Pro Lys His Ala Ala
   385 390 395 400
   Asp Ala Ser Glu Met Val Trp Val Asp Val Pro Asp Cys Phe Cys Phe
   405 410 415
   His Leu Trp Asn Ala Trp Glu Glu Ala Asp Thr Asp Glu Val Val Val
   420 425 430
   Ile Gly Ser Cys Met Thr Pro Ala Asp Ser Ile Phe Asn Glu Ser Asp
   435 440 445
   Asp Arg Leu Glu Ser Val Leu Thr Glu Ile Arg Leu Asn Thr Arg Thr
   450 455 460
   Gly Glu Ser Thr Arg Arg Ala Ile Leu Pro Pro Ser Ser Gln Val Asn
   465 470 475 480
   Leu Glu Val Gly Met Val Asn Arg Asn Leu Leu Gly Arg Lys Thr Arg
   485 490 495
   Tyr Ala Tyr Leu Ala Val Ala Glu Pro Trp Pro Lys Val Ser Gly Phe
   500 505 510
   Ala Lys Val Asp Leu Ala Thr Gly Glu Leu Thr Lys Phe Glu Tyr Gly
   515 520 525
   Glu Gly Arg Phe Gly Gly Glu Pro Cys Phe Val Pro Met Asp Ala Ala
   530 535 540
   Ala Ala Thr Pro Arg Gly Glu Asp Asp Gly Tyr Ile Leu Ser Phe Val
   545 550 555 560
   His Asp Glu Arg Ala Gly Thr Ser Glu Leu Leu Val Val Asn Ala Ala
   565 570 575
   Asp Met Arg Leu Glu Ala Thr Val Gln Leu Pro Ser Arg Val Pro Tyr
   580 585 590
   Gly Phe His Gly Thr Phe Ile Thr Gly Asp Glu Leu Thr Thr Gln Ala
   595 600 605
<210> 23
   <211> 1842
   <212> DNA
   <213> Oryza sativa
<400> 23
   atgccgacca ccttcacgcc caattccccc gcctcctcgt gttccataca ccaccgcgcc      60
   tccccgtcga ggggtgcccg caattcggtg cggttcacgc gcccgcgcgc cgccgccgcg     120
   gcgacgaact cggtgctcag cgcgccgtcg tccgtgccgc ccgcgtacgt gccgccgccg     180
   ccgccgccgc cgaccaagat gttcccggag gcgggcgacg cggcggcggc caaggctgcg     240
   gcgaggaggt gtggcaagaa gaaggatggg ctgaacttct tccagcgcgc ggcggcggtg     300
   gcgctcgacg cgttcgagga agggttcatc acgaatgtgc tggagaggcc gcacgcgctg     360
   ccgcggacgg ccgacccggc ggtgcagatc gccgggaact tcgcgccggt gggggagcag     420
   ccgccggtgc ggtcgctccc ggtgtccggc cgcatcccgc ccttcatcaa tggcgtctac     480
   gcccgcaacg gcgccaaccc gcacttcgag cccaccgcgg gccaccacct gttcgacggc     540
   gacggcatgg tccacgccgt ccgcatccgc aacggcgccg ccgagtccta cgcctgccgc     600
   ttcaccgaga ctgcgcgcct cggccaggag cgcgccctcg gccgcgccgt cttccccaag     660
   gccatcggcg agctccatgg ccactccggc atcgcccgcc tcgccctctt ctacgcgcgg     720
   gggctctgcg gcctcgtcga cccgtcgcac ggcaccggcg tcgccaacgc cggcctcgtc     780
   tacttcaacg gccgcctcct cgccatgtcc gaggacgacc tcccgtacca ggtccgcgtc     840
   accgccgacg gcgacctcga gacggtgggg cgctacgact tcgacggcca gctcggctgc     900
   gccatgatcg cccaccccaa gctcgacccg gtctccggcg agctcttcgc cctcagctac     960
   gacgtgatca agaagccata cctgaaatac ttctacttcg acgccgatgg caccaagtca    1020
   cccgacgtcg agatcgagct tgagcagccg acgatgatcc acgacttcgc catcaccgag    1080
   aacttcgtgg tggtacccga ccaccaggtg gtgttcaagc tcggcgagat gttccgcggc    1140
   ggctcgccgg tggtgctcga cagggagaag acgtcgcggt tcggcgtgct ccccaagcac    1200
   gcgacgagct cgttggagat ggtgtgggtc gacgtccccg actgcttctg cttccacctg    1260
   tggaatgcgt gggaggaggc cgagtccggc gaggtggtgg tggtgggatc ctgcatgacg    1320
   cccgccgact ccatcttcaa cgagtcggac gaacacctcg agagcgtgct caccgagatc    1380
   cgcctcaaca cgcgcaccgg cgagtccacc cgccgcgccg tgctgccgcc ggcggcgcag    1440
   gtgaacctcg aggtcggcat ggtgaaccgc gccatgctcg gccggaagac gaggtacgcc    1500
   tacctcgccg tcgccgagcc gtggcccaag gtgtccggct tcgccaaggt ggacctcgcc    1560
   accggcgagc tcaccaagtt cgagtacggc gagggccggt tcggcggcga gccgtgcttc    1620
   gtgcccatgg gcggcgccgg cgccgccgcg tccccggcgc gcggcgagga cgacggctac    1680
   atcctctcct tcgtccgcga cgaggccgcg ggcacatccg agctcctcgt cgtgaacgcc    1740
   gccgacatga ggctggaggc caccgtccag ctgccgtcgc gcgtccccta cggcttccac    1800
   ggcaccttca tcaacgccgg cgagctcgcc acgcaggcct ag                       1842
<210> 24
   <211> 613
   <212> PRT
   <213> Oryza sativa
<400> 24
   Met Pro Thr Thr Phe Thr Pro Asn Ser Pro Ala Ser Ser Cys Ser Ile
   1               5                   10                  15
   His His Arg Ala Ser Pro Ser Arg Gly Ala Arg Asn Ser Val Arg Phe
   20 25 30
   Thr Arg Pro Arg Ala Ala Ala Ala Ala Thr Asn Ser Val Leu Ser Ala
   35 40 45
   Pro Ser Ser Val Pro Pro Ala Tyr Val Pro Pro Pro Pro Pro Pro Pro
   50 55 60
   Thr Lys Met Phe Pro Glu Ala Gly Asp Ala Ala Ala Ala Lys Ala Ala
   65 70 75 80
   Ala Arg Arg Cys Gly Lys Lys Lys Asp Gly Leu Asn Phe Phe Gln Arg
   85 90 95
   Ala Ala Ala Val Ala Leu Asp Ala Phe Glu Glu Gly Phe Ile Thr Asn
   100 105 110
   Val Leu Glu Arg Pro His Ala Leu Pro Arg Thr Ala Asp Pro Ala Val
   115 120 125
   Gln Ile Ala Gly Asn Phe Ala Pro Val Gly Glu Gln Pro Pro Val Arg
   130 135 140
   Ser Leu Pro Val Ser Gly Arg Ile Pro Pro Phe Ile Asn Gly Val Tyr
   145 150 155 160
   Ala Arg Asn Gly Ala Asn Pro His Phe Glu Pro Thr Ala Gly His His
   165 170 175
   Leu Phe Asp Gly Asp Gly Met Val His Ala Val Arg Ile Arg Asn Gly
   180 185 190
   Ala Ala Glu Ser Tyr Ala Cys Arg Phe Thr Glu Thr Ala Arg Leu Gly
   195 200 205
   Gln Glu Arg Ala Leu Gly Arg Ala Val Phe Pro Lys Ala Ile Gly Glu
   210 215 220
   Leu His Gly His Ser Gly Ile Ala Arg Leu Ala Leu Phe Tyr Ala Arg
   225 230 235 240
   Gly Leu Cys Gly Leu Val Asp Pro Ser His Gly Thr Gly Val Ala Asn
   245 250 255
   Ala Gly Leu Val Tyr Phe Asn Gly Arg Leu Leu Ala Met Ser Glu Asp
   260 265 270
   Asp Leu Pro Tyr Gln Val Arg Val Thr Ala Asp Gly Asp Leu Glu Thr
   275 280 285
   Val Gly Arg Tyr Asp Phe Asp Gly Gln Leu Gly Cys Ala Met Ile Ala
   290 295 300
   His Pro Lys Leu Asp Pro Val Ser Gly Glu Leu Phe Ala Leu Ser Tyr
   305 310 315 320
   Asp Val Ile Lys Lys Pro Tyr Leu Lys Tyr Phe Tyr Phe Asp Ala Asp
   325 330 335
   Gly Thr Lys Ser Pro Asp Val Glu Ile Glu Leu Glu Gln Pro Thr Met
   340 345 350
   Ile His Asp Phe Ala Ile Thr Glu Asn Phe Val Val Val Pro Asp His
   355 360 365
   Gln Val Val Phe Lys Leu Gly Glu Met Phe Arg Gly Gly Ser Pro Val
   370 375 380
   Val Leu Asp Arg Glu Lys Thr Ser Arg Phe Gly Val Leu Pro Lys His
   385 390 395 400
   Ala Thr Ser Ser Leu Glu Met Val Trp Val Asp Val Pro Asp Cys Phe
   405 410 415
   Cys Phe His Leu Trp Asn Ala Trp Glu Glu Ala Glu Ser Gly Glu Val
   420 425 430
   Val Val Val Gly Ser Cys Met Thr Pro Ala Asp Ser Ile Phe Asn Glu
   435 440 445
   Ser Asp Glu His Leu Glu Ser Val Leu Thr Glu Ile Arg Leu Asn Thr
   450 455 460
   Arg Thr Gly Glu Ser Thr Arg Arg Ala Val Leu Pro Pro Ala Ala Gln
   465 470 475 480
   Val Asn Leu Glu Val Gly Met Val Asn Arg Ala Met Leu Gly Arg Lys
   485 490 495
   Thr Arg Tyr Ala Tyr Leu Ala Val Ala Glu Pro Trp Pro Lys Val Ser
   500 505 510
   Gly Phe Ala Lys Val Asp Leu Ala Thr Gly Glu Leu Thr Lys Phe Glu
   515 520 525
   Tyr Gly Glu Gly Arg Phe Gly Gly Glu Pro Cys Phe Val Pro Met Gly
   530 535 540
   Gly Ala Gly Ala Ala Ala Ser Pro Ala Arg Gly Glu Asp Asp Gly Tyr
   545 550 555 560
   Ile Leu Ser Phe Val Arg Asp Glu Ala Ala Gly Thr Ser Glu Leu Leu
   565 570 575
   Val Val Asn Ala Ala Asp Met Arg Leu Glu Ala Thr Val Gln Leu Pro
   580 585 590
   Ser Arg Val Pro Tyr Gly Phe His Gly Thr Phe Ile Asn Ala Gly Glu
   595 600 605
   Leu Ala Thr Gln Ala
   610
<210> 25
   <211> 1749
   <212> DNA
   <213> Oryza sativa
<400> 25
   atggcgtcct ccgcgccttc cgcccccggc ctcgcgccgg tcgccaagcc gccgccgccg      60
   ccgtccaagg tgaaggtggc gacagcaacc gtgccaacca atggcaagat caagcagggt     120
   gcgaggccaa tgcgtgtctc ggcgccgccg gtggagccgc ggcggcggat gaacccgctc     180
   cagcggctgg cggcggcggc gattgacgcc gtggaggaag gcctcgtcgc cgggttgctc     240
   gagcgggggc acgcgctgcc gcgcaccgct gatccggccg tgcagatcgc cgggaactac     300
   gcgcccgtcg gggagcgccc gccggtgagg gggctgccgg tgtccggccg cctcccggcg     360
   tgcctcgacg gggtgtacgt ccgcaacggc gccaacccgc tccacgcgcc gcgcgccggg     420
   caccacctgt tcgacggcga cgggatgctg cacgccgtgc ggctcgccgg ggggcgcgcc     480
   gagtcgtacg cgtgccggtt cacggagacg gcgcggctgc ggcaggagcg ggagatggga     540
   cgccccgtgt tccccaaggc catcggcgag ctccacggcc actccggcgt cgcgcggctt     600
   ctgctgttcg gctcgcgcgc gctctgcggc gtgctcgacg cgtcccgggg catcggcgtc     660
   gccaacgccg gcctcgtcta ccacgacggc cgcctcctcg ccatgtccga ggacgacctc     720
   ccctaccacg tccgtgtcac ccacgacggc gacctcgaga ccgtcgggag gtacgacttc     780
   catgggcagc tcgacgccga cggcaccatg atcgcgcacc ccaagctcga cccggtcacc     840
   ggcgagctct tcgcgctcag ctacaatgtc gtgtccaagc cgtacctcaa gtacttctac     900
   ttcaccgccg acggccgcaa gtcccgagac gtcgacatcc ccgtcggcgc gccgacgatg     960
   atccacgact tcgccgtcac cgagaactat gccgtcgtcc ccgaccagca gatcgtgttc    1020
   aagctccagg agatggtgcg cggcggctcg ccggtggtat acgacaggga gaaggcgtcg    1080
   cggttcggcg tgctcccgaa gcgcgccgcc gacgcgtcgg agctccggtg ggtggaggtc    1140
   cccggctgct tctgcttcca cctctggaac gcgtgggagg acgacgccac cggcgagatc    1200
   gtggtcatcg gctcctgcat gacgccgccg gacgccgtgt tcaacgagcc gtcgcagtcg    1260
   ccggaggagg agagcttccg cagcgtgctc tccgagatcc gcctcgaccc gcgcaccggc    1320
   gtgtcgcggc ggcgcgacgt gctgcgcgac gccgccgagc aggtgaacct cgaggccggc    1380
   atggtgaacc ggcagctgct cggccggaag acgcggtacg cctacctcgc catcgccgag    1440
   ccatggccga gggtgtcggg cttcgccaag gtggacctcg agagcggcac ggcggagaag    1500
   ttcatctacg gcgaggggag gtacggcggc gagccatgct tcgttccgcg cgccggcgcc    1560
   gcggcggagg acgacggcca cgtgctgtgc ttcgtccacg acgaggagcg cggcacgtcg    1620
   gagctggtgg tggtggacgc cggcagcgag gcgatggagg aggtcgcggc cgtgaagctg    1680
   ccggggcgcg tgccgtacgg attgcacggc accttcattg gcgccaacga gctgcagcga    1740
   caagcttag                                                            1749
<210> 26
   <211> 582
   <212> PRT
   <213> Oryza sativa
<400> 26
   Met Ala Ser Ser Ala Pro Ser Ala Pro Gly Leu Ala Pro Val Ala Lys
   1               5                   10                  15
   Pro Pro Pro Pro Pro Ser Lys Val Lys Val Ala Thr Ala Thr Val Pro
               20                  25                  30
   Thr Asn Gly Lys Ile Lys Gln Gly Ala Arg Pro Met Arg Val Ser Ala
           35                  40                  45
   Pro Pro Val Glu Pro Arg Arg Arg Met Asn Pro Leu Gln Arg Leu Ala
       50                  55                  60
   Ala Ala Ala Ile Asp Ala Val Glu Glu Gly Leu Val Ala Gly Leu Leu
   65                  70                  75                  80
   Glu Arg Gly His Ala Leu Pro Arg Thr Ala Asp Pro Ala Val Gln Ile
                   85                  90                  95
   Ala Gly Asn Tyr Ala Pro Val Gly Glu Arg Pro Pro Val Arg Gly Leu
               100                 105                 110
   Pro Val Ser Gly Arg Leu Pro Ala Cys Leu Asp Gly Val Tyr Val Arg
           115                 120                 125
   Asn Gly Ala Asn Pro Leu His Ala Pro Arg Ala Gly His His Leu Phe
       130                 135                 140
   Asp Gly Asp Gly Met Leu His Ala Val Arg Leu Ala Gly Gly Arg Ala
   145                 150                 155                 160
   Glu Ser Tyr Ala Cys Arg Phe Thr Glu Thr Ala Arg Leu Arg Gln Glu
                   165                 170                 175
   Arg Glu Met Gly Arg Pro Val Phe Pro Lys Ala Ile Gly Glu Leu His
               180                 185                 190
   Gly His Ser Gly Val Ala Arg Leu Leu Leu Phe Gly Ser Arg Ala Leu
   195 200 205
   Cys Gly Val Leu Asp Ala Ser Arg Gly Ile Gly Val Ala Asn Ala Gly
   210 215 220
   Leu Val Tyr His Asp Gly Arg Leu Leu Ala Met Ser Glu Asp Asp Leu
   225 230 235 240
   Pro Tyr His Val Arg Val Thr His Asp Gly Asp Leu Glu Thr Val Gly
   245 250 255
   Arg Tyr Asp Phe His Gly Gln Leu Asp Ala Asp Gly Thr Met Ile Ala
   260 265 270
   His Pro Lys Leu Asp Pro Val Thr Gly Glu Leu Phe Ala Leu Ser Tyr
   275 280 285
   Asn Val Val Ser Lys Pro Tyr Leu Lys Tyr Phe Tyr Phe Thr Ala Asp
   290 295 300
   Gly Arg Lys Ser Arg Asp Val Asp Ile Pro Val Gly Ala Pro Thr Met
   305 310 315 320
   Ile His Asp Phe Ala Val Thr Glu Asn Tyr Ala Val Val Pro Asp Gln
   325 330 335
   Gln Ile Val Phe Lys Leu Gln Glu Met Val Arg Gly Gly Ser Pro Val
   340 345 350
   Val Tyr Asp Arg Glu Lys Ala Ser Arg Phe Gly Val Leu Pro Lys Arg
   355 360 365
   Ala Ala Asp Ala Ser Glu Leu Arg Trp Val Glu Val Pro Gly Cys Phe
   370 375 380
   Cys Phe His Leu Trp Asn Ala Trp Glu Asp Asp Ala Thr Gly Glu Ile
   385 390 395 400
   Val Val Ile Gly Ser Cys Met Thr Pro Pro Asp Ala Val Phe Asn Glu
   405 410 415
   Pro Ser Gln Ser Pro Glu Glu Glu Ser Phe Arg Ser Val Leu Ser Glu
   420 425 430
   Ile Arg Leu Asp Pro Arg Thr Gly Val Ser Arg Arg Arg Asp Val Leu
   435 440 445
   Arg Asp Ala Ala Glu Gln Val Asn Leu Glu Ala Gly Met Val Asn Arg
   450 455 460
   Gln Leu Leu Gly Arg Lys Thr Arg Tyr Ala Tyr Leu Ala Ile Ala Glu
   465 470 475 480
   Pro Trp Pro Arg Val Ser Gly Phe Ala Lys Val Asp Leu Glu Ser Gly
   485 490 495
   Thr Ala Glu Lys Phe Ile Tyr Gly Glu Gly Arg Tyr Gly Gly Glu Pro
   500 505 510
   Cys Phe Val Pro Arg Ala Gly Ala Ala Ala Glu Asp Asp Gly His Val
   515 520 525
   Leu Cys Phe Val His Asp Glu Glu Arg Gly Thr Ser Glu Leu Val Val
   530 535 540
   Val Asp Ala Gly Ser Glu Ala Met Glu Glu Val Ala Ala Val Lys Leu
   545 550 555 560
   Pro Gly Arg Val Pro Tyr Gly Leu His Gly Thr Phe Ile Gly Ala Asn
   565 570 575
   Glu Leu Gln Arg Gln Ala
   580
<210> 27
   <211> 1917
   <212> DNA
   <213> Oryza sativa
<400> 27
   atgcaaagga tttgccctgc tcactgctcg gtcactcact cactcaccat gaagtccatg 60
   aggctttcct acatccctcc tgctgcttct gctgctccac agagccccag ctatggcagg 120
   aagaagaacg cctccgccgc tccgccatcg gctgccgcct ccaccaccgt tctcacctcc 180
   ccgctggtga ccaccacccg cactccgaag cagaccgagc aagaggacga gcagttggta 240
   gccaagacca agactacgag aactgttatt gctacgacga atggcagggc ggcgccgagc 300
   cagtctcggc ctcgccgccg gcctgccccc gccgccgcgg cgtcggccgc ttcgctgccg 360
   atgacgttct gcaacgcgct ggaggaggtg atcaacacgt tcatcgaccc gccggcgctt 420
   cggccggcgg tggacccgcg gaacgtgctg accagcaact tcgtgcccgt ggacgagctg 480
   ccgccgacgc cctgccccgt cgtgcgcggc gccatcccgc gctgcctcgc cggcggcgcc 540
   tacatccgca acgggcccaa cccgcagcac ctcccgcgcg ggccgcacca cctgttcgac 600
   ggcgacggca tgctgcactc cctcctcctc ccgtcgcccg cgtcgtccgg cgacgacccc 660
   gtcctgtgct cgcgctacgt gcagacgtac aagtacctcg tggagcgcga cgccggcgcg 720
   cccgtcctgc ccaacgtctt ctccggcttc cacggcgtgg ccgggatggc gcgcggcgcc 780
   gtcgtggcgg ccagggtcct gaccgggcag atgaatccgt tggagggcgt cgggctcgcc 840
   aacaccagcc tcgcctactt cgccggccgc ctctacgcgc tcggcgagtc cgacctcccc 900
   tacgccgtgc gcgtccaccc ggacaccggc gaggtgacca cgcacggcag gtgcgacttc 960
   ggcggccgcc tcgtcatggg catgaccgcg caccccaaga aggaccccgt caccggcgag 1020
   ctcttcgcgt tccgctacgg ccccgtgccg ccgttcgtga cgtacttccg gttcgacccg 1080
   gccggcaaca agggcgccga cgtgcccatc ttctccgtgc agcagccgtc gttcctgcac 1140
   gacttcgcca tcaccgagcg gtacgccatc ttcccggaga tccagatcgt gatgaagccc 1200
   atggacatgg tggtgggcgg cggctcgccc gtggggtcgg accccggcaa ggtgccccgc 1260
   ctcggcgtga tcccgcgcta cgccaccgac gagtcggaga tgcggtggtt cgaggtgccg 1320
   ggcttcaaca tcatgcactc ggtgaacgcg tgggaggagg ccggcggcga ggagctggtg 1380
   ctggtggcgc ccaacgtcct ctccatcgag cacgccctgg agcacatgga gctagtgcac 1440
   tcctgcgtcg agaaggtgcg catcaacctc cgcaccggcg tcgtcacgcg caccccgctc 1500
   gccgccggga acttcgactt ccccgtgatc aacccggctt tcctcggccg ccgcaacagg 1560
   tacggctact tcggcgtcgg cgaccccgcg cccaagatcg gcggcgtggc caagctcgac 1620
   ttcgaccgcg ccggcgaggg cgactgcacc gtggcgcagc gcgacttcgg gcccgggtgc 1680
   ttcgccggcg aaccgttctt cgtggccgac gacgtcgagg gcaacggcaa cgaggatgac 1740
   gggtacttgg tgtgctacgt ccacgacgag gccaccggcg agaaccggtt cgtggtgatg 1800
   gacgcgcggt cgccggacct ggagatcgtc gcggaggtgc agctgcccgg acgcgtcccc 1860
   tacggcttcc atggcctgtt cgtcacgcag gccgagctcc agtcacagca ccaatga 1917
<210> 28
   <211> 638
   <212> PRT
   <213> Oryza sativa
<400> 28
   Met Gln Arg Ile Cys Pro Ala His Cys Ser Val Thr His Ser Leu Thr
   1               5                   10                  15
   Met Lys Ser Met Arg Leu Ser Tyr Ile Pro Pro Ala Ala Ser Ala Ala
               20                  25                  30
   Pro Gln Ser Pro Ser Tyr Gly Arg Lys Lys Asn Ala Ser Ala Ala Pro
           35                  40                  45
   Pro Ser Ala Ala Ala Ser Thr Thr Val Leu Thr Ser Pro Leu Val Thr
       50                  55                  60
   Thr Thr Arg Thr Pro Lys Gln Thr Glu Gln Glu Asp Glu Gln Leu Val
   65                  70                  75                  80
   Ala Lys Thr Lys Thr Thr Arg Thr Val Ile Ala Thr Thr Asn Gly Arg
                   85                  90                  95
   Ala Ala Pro Ser Gln Ser Arg Pro Arg Arg Arg Pro Ala Pro Ala Ala
               100                 105                 110
   Ala Ala Ser Ala Ala Ser Leu Pro Met Thr Phe Cys Asn Ala Leu Glu
   115 120 125
   Glu Val Ile Asn Thr Phe Ile Asp Pro Pro Ala Leu Arg Pro Ala Val
   130 135 140
   Asp Pro Arg Asn Val Leu Thr Ser Asn Phe Val Pro Val Asp Glu Leu
   145 150 155 160
   Pro Pro Thr Pro Cys Pro Val Val Arg Gly Ala Ile Pro Arg Cys Leu
   165 170 175
   Ala Gly Gly Ala Tyr Ile Arg Asn Gly Pro Asn Pro Gln His Leu Pro
   180 185 190
   Arg Gly Pro His His Leu Phe Asp Gly Asp Gly Met Leu His Ser Leu
   195 200 205
   Leu Leu Pro Ser Pro Ala Ser Ser Gly Asp Asp Pro Val Leu Cys Ser
   210 215 220
   Arg Tyr Val Gln Thr Tyr Lys Tyr Leu Val Glu Arg Asp Ala Gly Ala
   225 230 235 240
   Pro Val Leu Pro Asn Val Phe Ser Gly Phe His Gly Val Ala Gly Met
   245 250 255
   Ala Arg Gly Ala Val Val Ala Ala Arg Val Leu Thr Gly Gln Met Asn
   260 265 270
   Pro Leu Glu Gly Val Gly Leu Ala Asn Thr Ser Leu Ala Tyr Phe Ala
   275 280 285
   Gly Arg Leu Tyr Ala Leu Gly Glu Ser Asp Leu Pro Tyr Ala Val Arg
   290 295 300
   Val His Pro Asp Thr Gly Glu Val Thr Thr His Gly Arg Cys Asp Phe
   305 310 315 320
   Gly Gly Arg Leu Val Met Gly Met Thr Ala His Pro Lys Lys Asp Pro
   325 330 335
   Val Thr Gly Glu Leu Phe Ala Phe Arg Tyr Gly Pro Val Pro Pro Phe
   340 345 350
   Val Thr Tyr Phe Arg Phe Asp Pro Ala Gly Asn Lys Gly Ala Asp Val
   355 360 365
   Pro Ile Phe Ser Val Gln Gln Pro Ser Phe Leu His Asp Phe Ala Ile
   370 375 380
   Thr Glu Arg Tyr Ala Ile Phe Pro Glu Ile Gln Ile Val Met Lys Pro
   385 390 395 400
   Met Asp Met Val Val Gly Gly Gly Ser Pro Val Gly Ser Asp Pro Gly
   405 410 415
   Lys Val Pro Arg Leu Gly Val Ile Pro Arg Tyr Ala Thr Asp Glu Ser
   420 425 430
   Glu Met Arg Trp Phe Glu Val Pro Gly Phe Asn Ile Met His Ser Val
   435 440 445
   Asn Ala Trp Glu Glu Ala Gly Gly Glu Glu Leu Val Leu Val Ala Pro
   450 455 460
   Asn Val Leu Ser Ile Glu His Ala Leu Glu His Met Glu Leu Val His
   465 470 475 480
   Ser Cys Val Glu Lys Val Arg Ile Asn Leu Arg Thr Gly Val Val Thr
   485 490 495
   Arg Thr Pro Leu Ala Ala Gly Asn Phe Asp Phe Pro Val Ile Asn Pro
   500 505 510
   Ala Phe Leu Gly Arg Arg Asn Arg Tyr Gly Tyr Phe Gly Val Gly Asp
   515 520 525
   Pro Ala Pro Lys Ile Gly Gly Val Ala Lys Leu Asp Phe Asp Arg Ala
   530 535 540
   Gly Glu Gly Asp Cys Thr Val Ala Gln Arg Asp Phe Gly Pro Gly Cys
   545 550 555 560
   Phe Ala Gly Glu Pro Phe Phe Val Ala Asp Asp Val Glu Gly Asn Gly
   565 570 575
   Asn Glu Asp Asp Gly Tyr Leu Val Cys Tyr Val His Asp Glu Ala Thr
   580 585 590
   Gly Glu Asn Arg Phe Val Val Met Asp Ala Arg Ser Pro Asp Leu Glu
   595 600 605
   Ile Val Ala Glu Val Gln Leu Pro Gly Arg Val Pro Tyr Gly Phe His
   610 615 620
   Gly Leu Phe Val Thr Gln Ala Glu Leu Gln Ser Gln His Gln
   625 630 635
<210> 29
   <211> 1731
   <212> DNA
   <213> Oryza sativa
<400> 29
   atggaggtac ccattgctgc catgactttt gcccacccag ccaatgttat gactctggct 60
   tcaaggcagc caaagagtaa aaggtcccat atctcccctg ctaccacggc tcaccgtaat 120
   ctacagactc gcctggctca ccaccaccat gcaacaccag cttcattgcc tatggcaatc 180
   tgcaacacag tagacaaagt gatcaatagg ttcattgacc tgccggagca gcgaccaacg 240
   gtggatccgc ggcgtgtgct ctctggcaac ttcgctcctg ttgatgagct gcccccgaca 300
   agctgccatg tcatccgcgg ctccatccca agctgcctcg ccggtggggt ctacatccgc 360
   aatggtccca acccacagca ccggcttccc cagcgaacac accacctctt cgatggtgat 420
   ggcatgctcc actcccttct cattccctcg gcctcgtcaa cactgttgtc ggagcctgtg 480
   ctttgttcac gctatgtgca cacgtacaag tatctcttgg agcgtgagac cggaggaccg 540
   gttttaccaa acttcttcgc tggcttccat ggagtggccg gcttggctcg tgcagtggtc 600
   atgatcgcaa gagtgcttgc tggtcaaatt aacctgaaca agggcttcgg gctggccaac 660
   actagcatca ctctttttgc agattgccta tatgcgctat gcgaatctga ccttccctac 720
   tccatgcaca tcaacccagc caacggagaa gtcaccacac ttggtcgatg tgactttggt 780
   ggtgatcttt cttttaggat gacagcacac cccaagaagg acccggtcac catggagttg 840
   tttgcttttc gctacaatgt cttccaacca ttcataacat acttctggtt cgatcgagca 900
   ggcagcaagg tcgcagatgt gcccatcttg tccttgcaga aaccatcggt gatgcatgac 960
   tttgcaataa cagagagata tgcaatcttt ccagagtcac aactcatcgt taatcccatg 1020
   gacatggtca tgcgggggag ctcgttggta ggattggacc gtaccatggt gccacggatt 1080
   ggcgtgcttc caaggtacgc caaggatgag tcagacatga gatggtttga ggtgcctaga 1140
   tttaatatgt tgcacacgac gaatggttgg gaagaggctg atggagagga gattgtgctc 1200
   gtggcaccca atatcctatc tatcgaacac atgctaggaa acatggagct catgcgagct 1260
   cgtgtcgaca tggtacgtat caacctctgc accggtgacg tgtcgtgcac tgcactctca 1320
   ccggagagcc ttgagttcgg tgtcatccac caaggttatg ttggtcgcaa aaatcgctat 1380
   ggctactttg gtgtaagtgg tccgttgccc aagatcaagg ggataagaaa gcttgacttt 1440
   gatctcgtcg gctctggtga ttgcacggtt ggacgtcgtg actttggtct agggtgcttt 1500
   gctggggaac cattttttgt tccagacaac atcgacgggt atggaaacga ggatagtggt 1560
   tatgtggtgt gctacaccca tgaagaggac accggagaga gttggtttgt ggtgatggat 1620
   gcaaagtctc cagagctaga cattgttgca gaagtgcaac ttcctagtcg tatcccctat 1680
   ggctttcatg gtatttttgt caaacaggcc gaacttctcg cacaacaata a 1731
<210> 30
   <211> 576
   <212> PRT
   <213> Oryza sativa
<400> 30
   Met Glu Val Pro Ile Ala Ala Met Thr Phe Ala His Pro Ala Asn Val
   1               5                   10                  15
   Met Thr Leu Ala Ser Arg Gln Pro Lys Ser Lys Arg Ser His Ile Ser
   20 25 30
   Pro Ala Thr Thr Ala His Arg Asn Leu Gln Thr Arg Leu Ala His His
   35 40 45
   His His Ala Thr Pro Ala Ser Leu Pro Met Ala Ile Cys Asn Thr Val
   50 55 60
   Asp Lys Val Ile Asn Arg Phe Ile Asp Leu Pro Glu Gln Arg Pro Thr
   65 70 75 80
   Val Asp Pro Arg Arg Val Leu Ser Gly Asn Phe Ala Pro Val Asp Glu
   85 90 95
   Leu Pro Pro Thr Ser Cys His Val Ile Arg Gly Ser Ile Pro Ser Cys
   100 105 110
   Leu Ala Gly Gly Val Tyr Ile Arg Asn Gly Pro Asn Pro Gln His Arg
   115 120 125
   Leu Pro Gln Arg Thr His His Leu Phe Asp Gly Asp Gly Met Leu His
   130 135 140
   Ser Leu Leu Ile Pro Ser Ala Ser Ser Thr Leu Leu Ser Glu Pro Val
   145 150 155 160
   Leu Cys Ser Arg Tyr Val His Thr Tyr Lys Tyr Leu Leu Glu Arg Glu
   165 170 175
   Thr Gly Gly Pro Val Leu Pro Asn Phe Phe Ala Gly Phe His Gly Val
   180 185 190
   Ala Gly Leu Ala Arg Ala Val Val Met Ile Ala Arg Val Leu Ala Gly
   195 200 205
   Gln Ile Asn Leu Asn Lys Gly Phe Gly Leu Ala Asn Thr Ser Ile Thr
   210 215 220
   Leu Phe Ala Asp Cys Leu Tyr Ala Leu Cys Glu Ser Asp Leu Pro Tyr
   225 230 235 240
   Ser Met His Ile Asn Pro Ala Asn Gly Glu Val Thr Thr Leu Gly Arg
   245 250 255
   Cys Asp Phe Gly Gly Asp Leu Ser Phe Arg Met Thr Ala His Pro Lys
   260 265 270
   Lys Asp Pro Val Thr Met Glu Leu Phe Ala Phe Arg Tyr Asn Val Phe
   275 280 285
   Gln Pro Phe Ile Thr Tyr Phe Trp Phe Asp Arg Ala Gly Ser Lys Val
   290 295 300
   Ala Asp Val Pro Ile Leu Ser Leu Gln Lys Pro Ser Val Met His Asp
   305 310 315 320
   Phe Ala Ile Thr Glu Arg Tyr Ala Ile Phe Pro Glu Ser Gln Leu Ile
   325 330 335
   Val Asn Pro Met Asp Met Val Met Arg Gly Ser Ser Leu Val Gly Leu
   340 345 350
   Asp Arg Thr Met Val Pro Arg Ile Gly Val Leu Pro Arg Tyr Ala Lys
   355 360 365
   Asp Glu Ser Asp Met Arg Trp Phe Glu Val Pro Arg Phe Asn Met Leu
   370 375 380
   His Thr Thr Asn Gly Trp Glu Glu Ala Asp Gly Glu Glu Ile Val Leu
   385 390 395 400
   Val Ala Pro Asn Ile Leu Ser Ile Glu His Met Leu Gly Asn Met Glu
   405 410 415
   Leu Met Arg Ala Arg Val Asp Met Val Arg Ile Asn Leu Cys Thr Gly
   420 425 430
   Asp Val Ser Cys Thr Ala Leu Ser Pro Glu Ser Leu Glu Phe Gly Val
   435 440 445
   Ile His Gln Gly Tyr Val Gly Arg Lys Asn Arg Tyr Gly Tyr Phe Gly
   450 455 460
   Val Ser Gly Pro Leu Pro Lys Ile Lys Gly Ile Arg Lys Leu Asp Phe
   465 470 475 480
   Asp Leu Val Gly Ser Gly Asp Cys Thr Val Gly Arg Arg Asp Phe Gly
   485 490 495
   Leu Gly Cys Phe Ala Gly Glu Pro Phe Phe Val Pro Asp Asn Ile Asp
   500 505 510
   Gly Tyr Gly Asn Glu Asp Ser Gly Tyr Val Val Cys Tyr Thr His Glu
   515 520 525
   Glu Asp Thr Gly Glu Ser Trp Phe Val Val Met Asp Ala Lys Ser Pro
   530 535 540
   Glu Leu Asp Ile Val Ala Glu Val Gln Leu Pro Ser Arg Ile Pro Tyr
   545 550 555 560
   Gly Phe His Gly Ile Phe Val Lys Gln Ala Glu Leu Leu Ala Gln Gln
   565 570 575
<210> 31
   <211> 522
   <212> DNA
   <213> Oryza sativa
<400> 31
   atgaggtggt tcgacgtgcc gggctgcttc tgcttccaca tctggaacgc ctgggacgaa      60
   cccgccgtcg tcatcgtctg ctcctgtatc acgccgcccg acgcgctctt gtcctccgtg     120
   cgcgccgtcc tgtccgaggt ccggctcgac ctccggacgg ggcggtacag ccggcgcgag     180
   ctcgtgccag ggctcaacct cgaggccggc acggtgaacc ggtcgctgct cggccgccgc     240
   actcgcttca cctacctcgc cgtcgccgag ccgtggccgc ggtgccgcgg cgtggccaag     300
   gtggaccttg gcaccggcga gctcgcggcc gtgcacgagt acggcgaggg acggttcagc     360
   ggcgagccca cgttcgtgcc ggcgacgtcg gcgacgtccg gcaccggcac cggaggcagg     420
   gaggacgacg ggcacgtggt ggtgatggtc cacgacgagg cggcgggcac ggcggagctg     480
   gtggtgctcg acgccgggaa gatggaggtg gcggcgacgt ag                        522
<210> 32
   <211> 173
   <212> PRT
   <213> Oryza sativa
<400> 32
   Met Arg Trp Phe Asp Val Pro Gly Cys Phe Cys Phe His Ile Trp Asn
   1               5                   10                  15
   Ala Trp Asp Glu Pro Ala Val Val Ile Val Cys Ser Cys Ile Thr Pro
               20                  25                  30
   Pro Asp Ala Leu Leu Ser Ser Val Arg Ala Val Leu Ser Glu Val Arg
           35                  40                  45
   Leu Asp Leu Arg Thr Gly Arg Tyr Ser Arg Arg Glu Leu Val Pro Gly
       50                  55                  60
   Leu Asn Leu Glu Ala Gly Thr Val Asn Arg Ser Leu Leu Gly Arg Arg
   65                  70                  75                  80
   Thr Arg Phe Thr Tyr Leu Ala Val Ala Glu Pro Trp Pro Arg Cys Arg
                   85                  90                  95
   Gly Val Ala Lys Val Asp Leu Gly Thr Gly Glu Leu Ala Ala Val His
               100                 105                 110
   Glu Tyr Gly Glu Gly Arg Phe Ser Gly Glu Pro Thr Phe Val Pro Ala
           115                 120                 125
   Thr Ser Ala Thr Ser Gly Thr Gly Thr Gly Gly Arg Glu Asp Asp Gly
       130                 135                 140
   His Val Val Val Met Val His Asp Glu Ala Ala Gly Thr Ala Glu Leu
   145                 150                 155                 160
   Val Val Leu Asp Ala Gly Lys Met Glu Val Ala Ala Thr
                   165                 170
<210> 33
   <211> 1992
   <212> DNA
   <213> Oryza sativa
<400> 33
   atggagagca cgtgctctcc aatttatcct actaatataa cacaagaaac gcaatcacga 60
   ctgaaaagga gaccaaaaac ggtgccatcc atgtataccc ttcaaccacg cgtatgcatc 120
   tccagctcca ggtcatccat ctcccctaaa gctgctcgtc tatctcatca gaaagcatcc 180
   actggaaagc cgtatttcag ggagattcag gtgcatctaa gctccaaact gggagaggca 240
   tcaaatgcca tgaacagtac ttaccaacag ctgctggatt catttgttga tcacaccttc 300
   acattcaaat gtcaaccttt gcgtcccact gagagcaatt ttgcaccagt cgacgagatc 360
   ggggagatca cacgggttat agagattgaa ggagagattc cagcagattt tcctgagggt 420
   gtctacataa gaaatggcgg aaaccctctc tatggaggcc tccaatctgt tagttccatt 480
   ttcgggcagt cccataacat ctgggtggaa ggagaaggca tgctccatgc tgtatacttc 540
   tgcaaaagta ataacagcac atggtcaatc tcgtacaata accgctacgt gcaatctgaa 600
   acatttagga ttgagaaaga acgtcagaaa ccatgctttc tccctatgac cgatgggaac 660
   cctcccgcca tgctcatcgc ctctgttctt aacacagtat ggtgcctgct gctattacta 720
   catcgcattt cttctgtaat ttttttggga ttgtcgcaaa cattttggaa atgctatatg 780
   ttgactgttg agtatgtgca gctgagattc cgaaaggtaa tgaaaagcat gagcaacact 840
   agcgtgtttg agcatgcagg gagagtttat gcagcatcag aggatgatgt tccccacgag 900
   gttgacttgc ataacctcag tacactaggc agctggcatc tcggtggtga atggaagttg 960
   ccatttacag cacaccccaa ggtaatccct gggtcaaaag agatggtcat tttcgggatc 1020
   aatgcggtac agcctttcct aacagttggc ataatctcag aggatggaga aaaacttaaa 1080
   caaaaggttg gtctcaagct agacagatgc acatattgtc atgaaattgg ggtcacgggg 1140
   acgtacaaca tcatcataga ttcaccactt accctcaacc caactaggat gctaagaggg 1200
   gctccagtgc ttgagtttga agaggaaagc tattcaagaa ttggggttat gccccattac 1260
   ggtgatgcag actcagtaat atggttttat gtagaaccat tctgcacatt ccatcttgtc 1320
   aattgcttcg aggaaggtca tgaggttgta gtaaggggat ttcatgtacc aagttctgcg 1380
   attatgggtc caaggcagaa gaacatggtt atggatactt ccagtcaaga accaaatgaa 1440
   gagaactttt ctcgcttgta tgaatggaga ttgaacctaa aaacaaggac tgttgcaggc 1500
   aaatacttaa ctagcttaga tgttgccttg gaattcccag ttatcaatga taaattttct 1560
   ggcctacgcc ataggtacgc atatgtacaa gtggcggatt gttcagcttg tttcggaggt 1620
   ggtcatgaaa tagaaactaa tttcagtcta aatcttgcag ctcgaccaaa gtttattggt 1680
   tttgcaaaac tttgtcttga agaaaaacaa aacatagcta ctaagataga cagagaagat 1740
   ctaataaagg tagagtatca tcagctagca aaaaatcaat tctgctcagg agtaacgttt 1800
   gtgcctaagg cagcaggtgc gcatgaagat gatggctgga tagtttcatt tgttcatgat 1860
   gaggaaacca atatatcaaa agtacacatc attgacgccc gaaactttga gagtgaacct 1920
   atagccaaga taatattacc acaaagagta ccctatggtt tgcatggagc attcattaca 1980
   aaaagaacat ga 1992
<210> 34
   <211> 663
   <212> PRT
   <213> Oryza sativa
<400> 34
   Met Glu Ser Thr Cys Ser Pro Ile Tyr Pro Thr Asn Ile Thr Gln Glu
   1               5                   10                  15
   Thr Gln Ser Arg Leu Lys Arg Arg Pro Lys Thr Val Pro Ser Met Tyr
               20                  25                  30
   Thr Leu Gln Pro Arg Val Cys Ile Ser Ser Ser Arg Ser Ser Ile Ser
           35                  40                  45
   Pro Lys Ala Ala Arg Leu Ser His Gln Lys Ala Ser Thr Gly Lys Pro
       50                  55                  60
   Tyr Phe Arg Glu Ile Gln Val His Leu Ser Ser Lys Leu Gly Glu Ala
   65                  70                  75                  80
   Ser Asn Ala Met Asn Ser Thr Tyr Gln Gln Leu Leu Asp Ser Phe Val
                   85                  90                  95
   Asp His Thr Phe Thr Phe Lys Cys Gln Pro Leu Arg Pro Thr Glu Ser
               100                 105                 110
   Asn Phe Ala Pro Val Asp Glu Ile Gly Glu Ile Thr Arg Val Ile Glu
           115                 120                 125
   Ile Glu Gly Glu Ile Pro Ala Asp Phe Pro Glu Gly Val Tyr Ile Arg
       130                 135                 140
   Asn Gly Gly Asn Pro Leu Tyr Gly Gly Leu Gln Ser Val Ser Ser Ile
   145                 150                 155                 160
   Phe Gly Gln Ser His Asn Ile Trp Val Glu Gly Glu Gly Met Leu His
                   165                 170                 175
   Ala Val Tyr Phe Cys Lys Ser Asn Asn Ser Thr Trp Ser Ile Ser Tyr
               180                 185                 190
   Asn Asn Arg Tyr Val Gln Ser Glu Thr Phe Arg Ile Glu Lys Glu Arg
           195                 200                 205
   Gln Lys Pro Cys Phe Leu Pro Met Thr Asp Gly Asn Pro Pro Ala Met
       210                 215                 220
   Leu Ile Ala Ser Val Leu Asn Thr Val Trp Cys Leu Leu Leu Leu Leu
   225                 230                 235                 240
   His Arg Ile Ser Ser Val Ile Phe Leu Gly Leu Ser Gln Thr Phe Trp
                   245                 250                 255
   Lys Cys Tyr Met Leu Thr Val Glu Tyr Val Gln Leu Arg Phe Arg Lys
               260                 265                 270
   Val Met Lys Ser Met Ser Asn Thr Ser Val Phe Glu His Ala Gly Arg
           275                 280                 285
   Val Tyr Ala Ala Ser Glu Asp Asp Val Pro His Glu Val Asp Leu His
       290                 295                 300
   Asn Leu Ser Thr Leu Gly Ser Trp His Leu Gly Gly Glu Trp Lys Leu
   305                 310                 315                 320
   Pro Phe Thr Ala His Pro Lys Val Ile Pro Gly Ser Lys Glu Met Val
                   325                 330                 335
   Ile Phe Gly Ile Asn Ala Val Gln Pro Phe Leu Thr Val Gly Ile Ile
               340                 345                 350
   Ser Glu Asp Gly Glu Lys Leu Lys Gln Lys Val Gly Leu Lys Leu Asp
           355                 360                 365
   Arg Cys Thr Tyr Cys His Glu Ile Gly Val Thr Gly Thr Tyr Asn Ile
       370                 375                 380
   Ile Ile Asp Ser Pro Leu Thr Leu Asn Pro Thr Arg Met Leu Arg Gly
   385                 390                 395                 400
   Ala Pro Val Leu Glu Phe Glu Glu Glu Ser Tyr Ser Arg Ile Gly Val
                   405                 410                 415
   Met Pro His Tyr Gly Asp Ala Asp Ser Val Ile Trp Phe Tyr Val Glu
               420                 425                 430
   Pro Phe Cys Thr Phe His Leu Val Asn Cys Phe Glu Glu Gly His Glu
           435                 440                 445
   Val Val Val Arg Gly Phe His Val Pro Ser Ser Ala Ile Met Gly Pro
       450                 455                 460
   Arg Gln Lys Asn Met Val Met Asp Thr Ser Ser Gln Glu Pro Asn Glu
   465 470 475 480
   Glu Asn Phe Ser Arg Leu Tyr Glu Trp Arg Leu Asn Leu Lys Thr Arg
   485 490 495
   Thr Val Ala Gly Lys Tyr Leu Thr Ser Leu Asp Val Ala Leu Glu Phe
   500 505 510
   Pro Val Ile Asn Asp Lys Phe Ser Gly Leu Arg His Arg Tyr Ala Tyr
   515 520 525
   Val Gln Val Ala Asp Cys Ser Ala Cys Phe Gly Gly Gly His Glu Ile
   530 535 540
   Glu Thr Asn Phe Ser Leu Asn Leu Ala Ala Arg Pro Lys Phe Ile Gly
   545 550 555 560
   Phe Ala Lys Leu Cys Leu Glu Glu Lys Gln Asn Ile Ala Thr Lys Ile
   565 570 575
   Asp Arg Glu Asp Leu Ile Lys Val Glu Tyr His Gln Leu Ala Lys Asn
   580 585 590
   Gln Phe Cys Ser Gly Val Thr Phe Val Pro Lys Ala Ala Gly Ala His
   595 600 605
   Glu Asp Asp Gly Trp Ile Val Ser Phe Val His Asp Glu Glu Thr Asn
   610 615 620
   Ile Ser Lys Val His Ile Ile Asp Ala Arg Asn Phe Glu Ser Glu Pro
   625 630 635 640
   Ile Ala Lys Ile Ile Leu Pro Gln Arg Val Pro Tyr Gly Leu His Gly
   645 650 655
   Ala Phe Ile Thr Lys Arg Thr
   660
<210> 35
   <211> 1659
   <212> DNA
   <213> Oryza sativa
<400> 35
   atggcgacct ccctgaccct gatcgccaca ccatgcacgg ctcctagatc atcctcgtcc      60
   ttcgcactcg ctccacggct gccgcctcgt tgcagcaacg cgactgcagc acgtcgccgc     120
   gccgtgaggg caaccacgct gcaatcggat caggaacctg caggatcagg agacagcggc     180
   gccacgacga cgaagctgtc tgcctcgacc agcgtccgcc aagaacgctg ggaaggcgac     240
   ctgcccatcg agggatgcct ccctccttgg ctgaatggca cgtacatcag gaacgggccg     300
   gggatgtggg acgtcgggga gcacgcgttc caccacctgt tcgacggcta cgcgacgctc     360
   gtccgcgtct ccttccgcgg cgggggtggt gcgcgcgcca cgggggcgca ccggcagatc     420
   gagtcggagg cgtacagggc cgccgtggcg cgcggccgcc cggtcctccg cgagttctcc     480
   cactgccccg cgccggccaa gagcctgctc caccgcttcg gcgatctcgt cggcctcgtc     540
   accggcgccg cgctcaccga caaccccaac agcgccgtgc tgccgctcgg cgacggccgg     600
   gtgatgtgcc tcaccgagac caccaagagc tccgtcctca tcgacccgga cacgctcgag     660
   acggtgggca ggttccggta cacggacagg ctgggtggca tggtgcagtc ggcgcacccg     720
   atcgtgaccg acacggagtt cctgacgctg ctgccggacc tcgtccgtcc gggccacctc     780
   gtcgtgagga tggaggccgg gagcaacgag aggaaggtga tcgggagaat ggactgccgc     840
   ggcgggccgt cgcccgggtg gctgcactcg ttcgccgtca cggagaagta cgccgtcgtg     900
   ccggagatgc cgctccggta ctcctccgcc agcctgctcg cctccgagct cgccccgttc     960
   tacgccttcg actgggtccc ggcgtccggc agctacatgc acgtcatgtg caagtccacc    1020
   ggcaagaccg tggcgagcgt ggaggtgcct cctttcatgg cgatccactt catcaacgca    1080
   tacgaggagg aaggcgacga ggccgccgtc gttgtcgact gctgtgagca ctacggtgac    1140
   cctgccatca tcgagacact cgtcctcagt agactgagat tgttaagggg caaggacgtt    1200
   ttacctaacg ccagggtggg gcggttcagg atcccgctgg atgggagccc gttcggcgaa    1260
   cttgagacgg cgctggaccc ggaggagcac gggcggggga tggacatgtg cagcatcaac    1320
   ccggcgcgcc tcggcaggaa gtaccagtat gcctacgcgt gcggcgcgcg tcgcccgtgc    1380
   aacttcccca acacgctcac caagatcgac ctggtggaga agaaggccaa gagctggcac    1440
   gaggagggct ccgtgccgtc cgagcctttc ttcgtcgcga ggccgggagc caccgacgaa    1500
   gacgatggag tggtgatatc tattgtaagc tccgacgatg gcgaagggta cgcactggtg    1560
   ctagacgcga ccacgtttga ggagattgcg cgcgtcaggt tcccctacgg attgccctac    1620
   ggcttccatg gctgctggat ccctgcgacg gaagagtga                           1659
<210> 36
   <211> 552
   <212> PRT
   <213> Oryza sativa
<400> 36
   Met Ala Thr Ser Leu Thr Leu Ile Ala Thr Pro Cys Thr Ala Pro Arg
   1               5                   10                  15
   Ser Ser Ser Ser Phe Ala Leu Ala Pro Arg Leu Pro Pro Arg Cys Ser
               20                  25                  30
   Asn Ala Thr Ala Ala Arg Arg Arg Ala Val Arg Ala Thr Thr Leu Gln
           35                  40                  45
   Ser Asp Gln Glu Pro Ala Gly Ser Gly Asp Ser Gly Ala Thr Thr Thr
       50                  55                  60
   Lys Leu Ser Ala Ser Thr Ser Val Arg Gln Glu Arg Trp Glu Gly Asp
   65 70 75 80
   Leu Pro Ile Glu Gly Cys Leu Pro Pro Trp Leu Asn Gly Thr Tyr Ile
   85 90 95
   Arg Asn Gly Pro Gly Met Trp Asp Val Gly Glu His Ala Phe His His
   100 105 110
   Leu Phe Asp Gly Tyr Ala Thr Leu Val Arg Val Ser Phe Arg Gly Gly
   115 120 125
   Gly Gly Ala Arg Ala Thr Gly Ala His Arg Gln Ile Glu Ser Glu Ala
   130 135 140
   Tyr Arg Ala Ala Val Ala Arg Gly Arg Pro Val Leu Arg Glu Phe Ser
   145 150 155 160
   His Cys Pro Ala Pro Ala Lys Ser Leu Leu His Arg Phe Gly Asp Leu
   165 170 175
   Val Gly Leu Val Thr Gly Ala Ala Leu Thr Asp Asn Pro Asn Ser Ala
   180 185 190
   Val Leu Pro Leu Gly Asp Gly Arg Val Met Cys Leu Thr Glu Thr Thr
   195 200 205
   Lys Ser Ser Val Leu Ile Asp Pro Asp Thr Leu Glu Thr Val Gly Arg
   210 215 220
   Phe Arg Tyr Thr Asp Arg Leu Gly Gly Met Val Gln Ser Ala His Pro
   225 230 235 240
   Ile Val Thr Asp Thr Glu Phe Leu Thr Leu Leu Pro Asp Leu Val Arg
   245 250 255
   Pro Gly His Leu Val Val Arg Met Glu Ala Gly Ser Asn Glu Arg Lys
   260 265 270
   Val Ile Gly Arg Met Asp Cys Arg Gly Gly Pro Ser Pro Gly Trp Leu
   275 280 285
   His Ser Phe Ala Val Thr Glu Lys Tyr Ala Val Val Pro Glu Met Pro
   290 295 300
   Leu Arg Tyr Ser Ser Ala Ser Leu Leu Ala Ser Glu Leu Ala Pro Phe
   305 310 315 320
   Tyr Ala Phe Asp Trp Val Pro Ala Ser Gly Ser Tyr Met His Val Met
   325 330 335
   Cys Lys Ser Thr Gly Lys Thr Val Ala Ser Val Glu Val Pro Pro Phe
   340 345 350
   Met Ala Ile His Phe Ile Asn Ala Tyr Glu Glu Glu Gly Asp Glu Ala
   355 360 365
   Ala Val Val Val Asp Cys Cys Glu His Tyr Gly Asp Pro Ala Ile Ile
   370 375 380
   Glu Thr Leu Val Leu Ser Arg Leu Arg Leu Leu Arg Gly Lys Asp Val
   385 390 395 400
   Leu Pro Asn Ala Arg Val Gly Arg Phe Arg Ile Pro Leu Asp Gly Ser
   405 410 415
   Pro Phe Gly Glu Leu Glu Thr Ala Leu Asp Pro Glu Glu His Gly Arg
   420 425 430
   Gly Met Asp Met Cys Ser Ile Asn Pro Ala Arg Leu Gly Arg Lys Tyr
   435 440 445
   Gln Tyr Ala Tyr Ala Cys Gly Ala Arg Arg Pro Cys Asn Phe Pro Asn
   450 455 460
   Thr Leu Thr Lys Ile Asp Leu Val Glu Lys Lys Ala Lys Ser Trp His
   465 470 475 480
   Glu Glu Gly Ser Val Pro Ser Glu Pro Phe Phe Val Ala Arg Pro Gly
   485 490 495
   Ala Thr Asp Glu Asp Asp Gly Val Val Ile Ser Ile Val Ser Ser Asp
   500 505 510
   Asp Gly Glu Gly Tyr Ala Leu Val Leu Asp Ala Thr Thr Phe Glu Glu
   515 520 525
   Ile Ala Arg Val Arg Phe Pro Tyr Gly Leu Pro Tyr Gly Phe His Gly
   530 535 540
   Cys Trp Ile Pro Ala Thr Glu Glu
   545 550
<210> 37
   <211> 756
   <212> DNA
   <213> Oryza sativa
<400> 37
   atgctacatg actttgcaat cacggagcat tatgccatct tccctgagtc gcaacttgtc     60
   atgtgtccga tgaacatggc tctacgtggt ggctcgctga taggattgga tagtgcaatg    120
   gtacaacgga ttggtgtgct accaaggtat gccgaggacg agtcagagat gaggtggttc    180
   aagctaatgc ctgggaggaa gctaatggag aggagattat attggtgtca accaacaact    240
   tatctgtcac acatgcttgg caacatggaa ctcatgcaag ttcaggttga catgatacat    300
   atcaatctcc gcactggtac cgttttgcgc actgcactct cactggagag ccttgagttt    360
   ggtgtaattc atcagggcta cgtcggtcga tacaaccgct atggatactt tggtgtaagc    420
   gcaccgttac ccaggttctc tggaatcaga aagctcgact ttgccatggt tggtgctgac    480
   gactgcacgg ttgcacgccg tgactttggc cccgggtgct ttgttgggga gccatttttc    540
   gtgccaagca atgataatgg ggatggtaat gaggacaatg gatatgtggt gtgctataca    600
   cacaaagagg ataccggaga gagccagttc gtggtgatgg atgccatgtc tccagaactg    660
   gaaattgttg cagcggtgca gctgcctgcc cgtgttccct atggctttca tggtcgtttt    720
   atcacgcagg ccgaactttt gtcgcagcag aaataa                              756
<210> 38
   <211> 251
   <212> PRT
   <213> Oryza sativa
<400> 38
   Met Leu His Asp Phe Ala Ile Thr Glu His Tyr Ala Ile Phe Pro Glu
   1               5                   10                  15
   Ser Gln Leu Val Met Cys Pro Met Asn Met Ala Leu Arg Gly Gly Ser
               20                  25                  30
   Leu Ile Gly Leu Asp Ser Ala Met Val Gln Arg Ile Gly Val Leu Pro
           35                  40                  45
   Arg Tyr Ala Glu Asp Glu Ser Glu Met Arg Trp Phe Lys Leu Met Pro
       50                  55                  60
   Gly Arg Lys Leu Met Glu Arg Arg Leu Tyr Trp Cys Gln Pro Thr Thr
   65                  70                  75                  80
   Tyr Leu Ser His Met Leu Gly Asn Met Glu Leu Met Gln Val Gln Val
                   85                  90                  95
   Asp Met Ile His Ile Asn Leu Arg Thr Gly Thr Val Leu Arg Thr Ala
               100                 105                 110
   Leu Ser Leu Glu Ser Leu Glu Phe Gly Val Ile His Gln Gly Tyr Val
           115                 120                 125
   Gly Arg Tyr Asn Arg Tyr Gly Tyr Phe Gly Val Ser Ala Pro Leu Pro
       130                 135                 140
   Arg Phe Ser Gly Ile Arg Lys Leu Asp Phe Ala Met Val Gly Ala Asp
   145                 150                 155                 160
   Asp Cys Thr Val Ala Arg Arg Asp Phe Gly Pro Gly Cys Phe Val Gly
                   165                 170                 175
   Glu Pro Phe Phe Val Pro Ser Asn Asp Asn Gly Asp Gly Asn Glu Asp
               180                 185                 190
   Asn Gly Tyr Val Val Cys Tyr Thr His Lys Glu Asp Thr Gly Glu Ser
           195                 200                 205
   Gln Phe Val Val Met Asp Ala Met Ser Pro Glu Leu Glu Ile Val Ala
       210                 215                 220
   Ala Val Gln Leu Pro Ala Arg Val Pro Tyr Gly Phe His Gly Arg Phe
   225                 230                 235                 240
   Ile Thr Gln Ala Glu Leu Leu Ser Gln Gln Lys
                   245                 250
<210> 39
   <211> 969
   <212> DNA
   <213> Oryza sativa
<400> 39
   atgcaaagga tttgccctgc tcactgctcg gtcactcact cactcaccat gaagtccatg     60
   aggctttcct acatccctcc tgctgcttct gctgctccac agagccccag ctatggcagg    120
   aagaagaacg cctccgccgc tccgccatcg gctgccgcct ccaccaccgt tctcacctcc    180
   ccgctggtga ccaccacccg cactccgaag cagaccgagc aagaggacga gcagttggta    240
   gccaagacca agactacgag aactgttatt gctacgacga atggcagggc ggcgccgagc    300
   cagtctcggc ctcgccgccg gcctgccccc gccgccgcgg cgtcggccgc ttcgctgccg    360
   atgacgttct gcaacgcgct ggaggaggtg atcaacacgt tcatcgaccc gccggcgctt    420
   cggccggcgg tggacccgcg gaacgtgctg accagcaact tcgtgcccgt ggacgagctg    480
   ccgccgacgc cctgccccgt cgtgcgcggc gccatcccgc gctgcctcgc cggcggcgcc    540
   tacatccgca acgggcccaa cccgcagcac ctcccgcgcg ggccgcacca cctgttcgac    600
   ggcgacggca tgctgcactc cctcctcctc ccgtcgcccg cgtcgtccgg cgacgacccc    660
   gtcctgtgct cgcgctacgt gcagacgtac aagtacctcg tggagcgcga cgccggcgcg    720
   cccgtcctgc ccaacgtctt ctccggcttc cacggcgtgg ccgggatggc gcgcggcgcc    780
   gtcgtggcgg ccagggtcct gaccgggcag atgaatccgt tggagggcgt cgggctcgcc    840
   aacaccagcc tcgcctactt cgccggccgc ctctacgcgc tcggcgagtc cgacctcccc    900
   tacgccgtgc gcgtccaccc ggacaccggc gaggtgacca cgcacggcag gtgcgacttc    960
   ggcggccgc                                                            969
<210> 40
   <211> 323
   <212> PRT
   <213> Oryza sativa
<400> 40
   Met Gln Arg Ile Cys Pro Ala His Cys Ser Val Thr His Ser Leu Thr
   1               5                   10                  15
   Met Lys Ser Met Arg Leu Ser Tyr Ile Pro Pro Ala Ala Ser Ala Ala
               20                  25                  30
   Pro Gln Ser Pro Ser Tyr Gly Arg Lys Lys Asn Ala Ser Ala Ala Pro
           35                  40                  45
   Pro Ser Ala Ala Ala Ser Thr Thr Val Leu Thr Ser Pro Leu Val Thr
       50                  55                  60
   Thr Thr Arg Thr Pro Lys Gln Thr Glu Gln Glu Asp Glu Gln Leu Val
   65                  70                  75                  80
   Ala Lys Thr Lys Thr Thr Arg Thr Val Ile Ala Thr Thr Asn Gly Arg
                   85                  90                  95
   Ala Ala Pro Ser Gln Ser Arg Pro Arg Arg Arg Pro Ala Pro Ala Ala
               100                 105                 110
   Ala Ala Ser Ala Ala Ser Leu Pro Met Thr Phe Cys Asn Ala Leu Glu
           115                 120                 125
   Glu Val Ile Asn Thr Phe Ile Asp Pro Pro Ala Leu Arg Pro Ala Val
       130                 135                 140
   Asp Pro Arg Asn Val Leu Thr Ser Asn Phe Val Pro Val Asp Glu Leu
   145                 150                 155                 160
   Pro Pro Thr Pro Cys Pro Val Val Arg Gly Ala Ile Pro Arg Cys Leu
                   165                 170                 175
   Ala Gly Gly Ala Tyr Ile Arg Asn Gly Pro Asn Pro Gln His Leu Pro
               180                 185                 190
   Arg Gly Pro His His Leu Phe Asp Gly Asp Gly Met Leu His Ser Leu
           195                 200                 205
   Leu Leu Pro Ser Pro Ala Ser Ser Gly Asp Asp Pro Val Leu Cys Ser
       210                 215                 220
   Arg Tyr Val Gln Thr Tyr Lys Tyr Leu Val Glu Arg Asp Ala Gly Ala
   225                 230                 235                 240
   Pro Val Leu Pro Asn Val Phe Ser Gly Phe His Gly Val Ala Gly Met
                   245                 250                 255
   Ala Arg Gly Ala Val Val Ala Ala Arg Val Leu Thr Gly Gln Met Asn
               260                 265                 270
   Pro Leu Glu Gly Val Gly Leu Ala Asn Thr Ser Leu Ala Tyr Phe Ala
   275 280 285
   Gly Arg Leu Tyr Ala Leu Gly Glu Ser Asp Leu Pro Tyr Ala Val Arg
   290 295 300
   Val His Pro Asp Thr Gly Glu Val Thr Thr His Gly Arg Cys Asp Phe
   305 310 315 320
   Gly Gly Arg
<210> 41
   <211> 1437
   <212> DNA
   <213> Oryza sativa
<400> 41
   atggtggatg gtgtggtgac cttactagag gcaaggaagc ttgtggggaa ggtgatccta      60
   gcggggagtg ctaatttata tgatggatct atccatgagc agagcaattt cgcaccagtt     120
   gatgaaatcg gtggaagaac agagatatgg aggatagaag ggacgatctc agatgatttt     180
   cctgagggtg tttatatcag aaatggttcc aaccctctct ttggagcctt gcacaaggtg     240
   aactcaatct tcggccagtc tgaagacatc tgggtcgagg gagaaggcat gctgcatgcc     300
   ctctacttca ccaagagcag agaaggaaac acttggtcag tctcatacaa caaccgctac     360
   gtgcaatctg acacttttaa tactgaaagg gatcgtcaga gaccatgctt cctctcagca     420
   atcaagggtg accctcttgc cataatcgca gccagcattt tgaacatgct gaggtttggc     480
   aaggtgttta gaaacatgag taataccggt gtgtttgagc acgccgagag ggttttctca     540
   gtggcagaga atgacattcc ctacgagatt gacttggaca atcttggcac actgtgtagc     600
   tgggtcgtcg atggtcagtg gaacatgcca ttcacagcac accccaaggt agccccaggg     660
   tcaggagagt tagtcatata tgggtttaac attgtgaagc ccttcctaac tattggagtt     720
   gtctcagagg atggaaagaa acttgaacgg aaggttgatc tcaagctaga aagatgtaca     780
   tattgccatg aaattggggt cactaaaatg tacaatatta tcatggatat gcctcttaca     840
   gtggacctta ctaggattct aagaggagct ccgttgattg attttgaaac agaaagttat     900
   gcaagaattg gagtcatgcc ccgtcatgga gatgcagatt cagtaatatg gttcgatgta     960
   gaaccatttt gcacattgca tctcatcaat tgctttgagg aagatcatga ggttgtcatc    1020
   agaggatttc gagtgccggg ttccataatt actggcataa cacttgagca cacggctaat    1080
   gaagagccag ctaatcaagg acccagtgag aaatcttttc ctcgcttata tgagtggaga    1140
   ttaaacatga aaagcagggc tgtcacagtg atcaataaca aatatgctgg cctgcatcac    1200
   aagtatgcat atgcacaagt gattgatgtc caagggagcc tggaaggtgg ctgtggaaca    1260
   gtgcgaccaa aatttggagg tttcgcaaaa ctacatcttc aagataacaa caaggcacat    1320
   gtcattgatg ctcagagatt tgaaaatgga ccaattgcta agataacatt gccacaaaga    1380
   gtaccctatg gctttcatgg cacattcatc ccaagaacta catacaagaa gacatga       1437
<210> 42
   <211> 478
   <212> PRT
   <213> Oryza sativa
<400> 42
   Met Val Asp Gly Val Val Thr Leu Leu Glu Ala Arg Lys Leu Val Gly
   1               5                   10                  15
   Lys Val Ile Leu Ala Gly Ser Ala Asn Leu Tyr Asp Gly Ser Ile His
               20                  25                  30
   Glu Gln Ser Asn Phe Ala Pro Val Asp Glu Ile Gly Gly Arg Thr Glu
           35                  40                  45
   Ile Trp Arg Ile Glu Gly Thr Ile Ser Asp Asp Phe Pro Glu Gly Val
       50                  55                  60
   Tyr Ile Arg Asn Gly Ser Asn Pro Leu Phe Gly Ala Leu His Lys Val
   65                  70                  75                  80
   Asn Ser Ile Phe Gly Gln Ser Glu Asp Ile Trp Val Glu Gly Glu Gly
                   85                  90                  95
   Met Leu His Ala Leu Tyr Phe Thr Lys Ser Arg Glu Gly Asn Thr Trp
               100                 105                 110
   Ser Val Ser Tyr Asn Asn Arg Tyr Val Gln Ser Asp Thr Phe Asn Thr
           115                 120                 125
   Glu Arg Asp Arg Gln Arg Pro Cys Phe Leu Ser Ala Ile Lys Gly Asp
       130                 135                 140
   Pro Leu Ala Ile Ile Ala Ala Ser Ile Leu Asn Met Leu Arg Phe Gly
   145                 150                 155                 160
   Lys Val Phe Arg Asn Met Ser Asn Thr Gly Val Phe Glu His Ala Glu
                   165                 170                 175
   Arg Val Phe Ser Val Ala Glu Asn Asp Ile Pro Tyr Glu Ile Asp Leu
               180                 185                 190
   Asp Asn Leu Gly Thr Leu Cys Ser Trp Val Val Asp Gly Gln Trp Asn
           195                 200                 205
   Met Pro Phe Thr Ala His Pro Lys Val Ala Pro Gly Ser Gly Glu Leu
       210                 215                 220
   Val Ile Tyr Gly Phe Asn Ile Val Lys Pro Phe Leu Thr Ile Gly Val
   225                 230                 235                 240
   Val Ser Glu Asp Gly Lys Lys Leu Glu Arg Lys Val Asp Leu Lys Leu
   245 250 255
   Glu Arg Cys Thr Tyr Cys His Glu Ile Gly Val Thr Lys Met Tyr Asn
   260 265 270
   Ile Ile Met Asp Met Pro Leu Thr Val Asp Leu Thr Arg Ile Leu Arg
   275 280 285
   Gly Ala Pro Leu Ile Asp Phe Glu Thr Glu Ser Tyr Ala Arg Ile Gly
   290 295 300
   Val Met Pro Arg His Gly Asp Ala Asp Ser Val Ile Trp Phe Asp Val
   305 310 315 320
   Glu Pro Phe Cys Thr Leu His Leu Ile Asn Cys Phe Glu Glu Asp His
   325 330 335
   Glu Val Val Ile Arg Gly Phe Arg Val Pro Gly Ser Ile Ile Thr Gly
   340 345 350
   Ile Thr Leu Glu His Thr Ala Asn Glu Glu Pro Ala Asn Gln Gly Pro
   355 360 365
   Ser Glu Lys Ser Phe Pro Arg Leu Tyr Glu Trp Arg Leu Asn Met Lys
   370 375 380
   Ser Arg Ala Val Thr Val Ile Asn Asn Lys Tyr Ala Gly Leu His His
   385 390 395 400
   Lys Tyr Ala Tyr Ala Gln Val Ile Asp Val Gln Gly Ser Leu Glu Gly
   405 410 415
   Gly Cys Gly Thr Val Arg Pro Lys Phe Gly Gly Phe Ala Lys Leu His
   420 425 430
   Leu Gln Asp Asn Asn Lys Ala His Val Ile Asp Ala Gln Arg Phe Glu
   435 440 445
   Asn Gly Pro Ile Ala Lys Ile Thr Leu Pro Gln Arg Val Pro Tyr Gly
   450 455 460
   Phe His Gly Thr Phe Ile Pro Arg Thr Thr Tyr Lys Lys Thr
   465 470 475
<210> 43
   <211> 1647
   <212> DNA
   <213> Oryza sativa
<400> 43
   atgatgacag cttctctgca tccatgtgtc tgcaaggcct ctcctgcctt cagacctgcc 60
   tcttctctgg gtgcaagaac ccagccaaaa tccacagcca caaacccaaa gagacctttg 120
   tttcaggagc ttcagaggcg actttctttc aggattgacg aagcctcaaa ggcgctggag 180
   acagcaaaac aggggctttt ggatgcgttg gtcgactcaa ccttcaagtt ttctgaccaa 240
   cctatgctcc catcagagaa caattttgcc ccagtcaatg agatcagtga agccatagag 300
   attctgcaga ttgaaggaga gatacctgaa gatttcccag agggttccaa tccactgttt 360
   ggagccctcc attcgaccgt ctcaatcttc ggaaaatcca gcgaaatatg ggttgaaggc 420
   gagggcatgc tccatgccat ctacttcaca aagaacagtt cagatacttg gtcagtctcc 480
   tatgcaaatc gatatgtgca atctgaaaca ttaaagattg agaaaaccag acagaagcca 540
   tgtttcctcc ctgccatcat gggcgactct gcagccatcg tcgcggccta cattctcaac 600
   tatatgaggt ttggcaaagt gaacaagaac atcagcaaca ccaatgtatt tgagcatgct 660
   ggaaaggtgt atgctgtttc tgagaaccac ctgcctcagg aaatcagcat tcagaatctc 720
   gacacgggtg atagctggga cattaatggg gaatggaaaa ggcctttcac agctcaccca 780
   aaggttgctc ctggatcagg agagctagtc atttttggtt cagatgcaaa gaggcctttc 840
   ctaatggtcg gagttgtctc agctgatgga actcaactaa aacataaagt tgacctcaaa 900
   ctggacagat gtatactctg ccatgacata ggagttactg ttaagtacaa cataataatg 960
   gacatacctc ttaccatcga catcagtaga cttatcagag gcaatcagtt gatcaagttt 1020
   gagaaggaca gctatgcaag aataggagtt atgccccgtt atggtgatgc agaatcagtt 1080
   atgtggtttg acgttgaacc attctgcatg ttccatttta tcaactgctt tgaagagggt 1140
   gatgaggttg ttatcagggg ctttcgtgcg gctgactcca tcattccagg ccctcggatc 1200
   agcctaaaca aaaatgattt gctctctgat ccttctaagt gttctgtgaa acaaggaatt 1260
   aatgaagaat ttttctctcg attataccag tggagattaa acacgaagac aaaggctgtt 1320
   tcagggcaat atttaagtgg aactgagttt tccatggaat tccctgtgat caatgaccac 1380
   tacacaggct tgcatcatag ttatgcctat gcacaagtgg tggattcttt ggaaagctct 1440
   tacggcgtta atgagaaagt aatcctgaaa tatggaggcc ttgcaaaact ttgtcttgaa 1500
   gaggcagata atgtaattgc agaggtgcac attattgacg cccaaacatt tgaaggtgct 1560
   cctgtggcca aaatagtatt gccacaaaga gtaccctatg gttttcatgg aacgttcaga 1620
   tcatcactag caaacacaat gacgtga 1647
<210> 44
   <211> 548
   <212> PRT
   <213> oryza sativa
<400> 44
   Met Met Thr Ala Ser Leu His Pro Cys Val Cys Lys Ala Ser Pro Ala
   1               5                   10                  15
   Phe Arg Pro Ala Ser Ser Leu Gly Ala Arg Thr Gln Pro Lys Ser Thr
               20                  25                  30
   Ala Thr Asn Pro Lys Arg Pro Leu Phe Gln Glu Leu Gln Arg Arg Leu
   35 40 45
   Ser Phe Arg Ile Asp Glu Ala Ser Lys Ala Leu Glu Thr Ala Lys Gln
   50 55 60
   Gly Leu Leu Asp Ala Leu Val Asp Ser Thr Phe Lys Phe Ser Asp Gln
   65 70 75 80
   Pro Met Leu Pro Ser Glu Asn Asn Phe Ala Pro Val Asn Glu Ile Ser
   85 90 95
   Glu Ala Ile Glu Ile Leu Gln Ile Glu Gly Glu Ile Pro Glu Asp Phe
   100 105 110
   Pro Glu Gly Ser Asn Pro Leu Phe Gly Ala Leu His Ser Thr Val Ser
   115 120 125
   Ile Phe Gly Lys Ser Ser Glu Ile Trp Val Glu Gly Glu Gly Met Leu
   130 135 140
   His Ala Ile Tyr Phe Thr Lys Asn Ser Ser Asp Thr Trp Ser Val Ser
   145 150 155 160
   Tyr Ala Asn Arg Tyr Val Gln Ser Glu Thr Leu Lys Ile Glu Lys Thr
   165 170 175
   Arg Gln Lys Pro Cys Phe Leu Pro Ala Ile Met Gly Asp Ser Ala Ala
   180 185 190
   Ile Val Ala Ala Tyr Ile Leu Asn Tyr Met Arg Phe Gly Lys Val Asn
   195 200 205
   Lys Asn Ile Ser Asn Thr Asn Val Phe Glu His Ala Gly Lys Val Tyr
   210 215 220
   Ala Val Ser Glu Asn His Leu Pro Gln Glu Ile Ser Ile Gln Asn Leu
   225 230 235 240
   Asp Thr Gly Asp Ser Trp Asp Ile Asn Gly Glu Trp Lys Arg Pro Phe
   245 250 255
   Thr Ala His Pro Lys Val Ala Pro Gly Ser Gly Glu Leu Val Ile Phe
   260 265 270
   Gly Ser Asp Ala Lys Arg Pro Phe Leu Met Val Gly Val Val Ser Ala
   275 280 285
   Asp Gly Thr Gln Leu Lys His Lys Val Asp Leu Lys Leu Asp Arg Cys
   290 295 300
   Ile Leu Cys His Asp Ile Gly Val Thr Val Lys Tyr Asn Ile Ile Met
   305 310 315 320
   Asp Ile Pro Leu Thr Ile Asp Ile Ser Arg Leu Ile Arg Gly Asn Gln
   325 330 335
   Leu Ile Lys Phe Glu Lys Asp Ser Tyr Ala Arg Ile Gly Val Met Pro
   340 345 350
   Arg Tyr Gly Asp Ala Glu Ser Val Met Trp Phe Asp Val Glu Pro Phe
   355 360 365
   Cys Met Phe His Phe Ile Asn Cys Phe Glu Glu Gly Asp Glu Val Val
   370 375 380
   Ile Arg Gly Phe Arg Ala Ala Asp Ser Ile Ile Pro Gly Pro Arg Ile
   385 390 395 400
   Ser Leu Asn Lys Asn Asp Leu Leu Ser Asp Pro Ser Lys Cys Ser Val
   405 410 415
   Lys Gln Gly Ile Asn Glu Glu Phe Phe Ser Arg Leu Tyr Gln Trp Arg
   420 425 430
   Leu Asn Thr Lys Thr Lys Ala Val Ser Gly Gln Tyr Leu Ser Gly Thr
   435 440 445
   Glu Phe Ser Met Glu Phe Pro Val Ile Asn Asp His Tyr Thr Gly Leu
   450 455 460
   His His Ser Tyr Ala Tyr Ala Gln Val Val Asp Ser Leu Glu Ser Ser
   465 470 475 480
   Tyr Gly Val Asn Glu Lys Val Ile Leu Lys Tyr Gly Gly Leu Ala Lys
   485 490 495
   Leu Cys Leu Glu Glu Ala Asp Asn Val Ile Ala Glu Val His Ile Ile
   500 505 510
   Asp Ala Gln Thr Phe Glu Gly Ala Pro Val Ala Lys Ile Val Leu Pro
   515 520 525
   Gln Arg Val Pro Tyr Gly Phe His Gly Thr Phe Arg Ser Ser Leu Ala
   530 535 540
   Asn Thr Met Thr
   545
<210> 45
   <211> 1710
   <212> DNA
   <213> Oryza sativa
<400> 45
   atgtctcccg ctatgctgca ggcgtcgtcg ctgtgcgtat ccgcggcgct gtcaggcgcc 60
   gcgagccggc cgggccgcct ggccagccag gggcaccagg gcaagcgggc cgtggcgcag 120
   cctctcgcgg ctagcgccgt gacggaggca gcgccgcccg cgccggtcgt cgcgccgccg 180
   gcccgccccg tcgacgcccc gcggcgccgt ggcggacgtg gcggcggcgg aggcggcggc 240
   gagctcgtgg cgtggaagag tgtacggcag gagaggtggg agggtgcgct cgaggtggac 300
   ggagagctgc ctctctggct ggatggcacg tacctgagga acggcccggg actatggaac 360
   ctcggcgact acggcttccg gcacctgttc gacggctacg cgacgctggt gcgcgtctcg 420
   ttccgcggcg gccgcgccgt gggcgcgcac cggcagatcg agtcggaggc gtacaaggcg 480
   gcgcgcgcgc acggcaaggt gtgctaccgc gagttctcgg aggtgcccaa gccggacaac 540
   ttcctgtcct acgtcggcca gctggcgacc ctcttctcgg gctcgtcgct caccgacaac 600
   tccaacaccg gcgtcgtcat gctcggcgac ggccgcgtgc tctgcctcac ggagaccatc 660
   aagggctcca tccaggtcga cccggacacg ctcgacacgg tcggcaagtt ccagtacacg 720
   gacaagctgg gcgggctgat ccactcggcg cacccgatcg tgaccgacac cgagttctgg 780
   acgctgatcc ccgacctgat ccggcccggc tacgtggtgg cgaggatgga cgccggtagc 840
   aacgagaggc agttcgtcgg cagggtggac tgccgcggcg ggccggcgcc agggtgggtg 900
   cactcgttcc ccgtcaccga gcactacgtc gtcgtgccgg agatgccgct ccgctactgc 960
   gccaagaacc tcctccgcgc cgagcccacg ccgctgtaca agttcgagtg gcacctcgag 1020
   tccggcagct acatgcacgt catgtgcaag gccagcggca agattgtggc gagcgtggag 1080
   gtgccgccgt tcgtgacgtt ccacttcatc aacgcgtacg aggagacgga cgaggagggg 1140
   cgcgtgacgg cgatcatcgc cgactgctgc gagcacaacg ccaacaccgc catcctcgac 1200
   aagctccgcc tccacaacct ccgctcctcc agcggccagg acgtcctccc cgacgccagg 1260
   gtggggcggt tcaggatccc cctggacggg agccagttcg gcgagctgga gacggcgctg 1320
   gacccggagg agcacgggcg gggcatggac atgtgcagca tcaacccggc gcacgtcggc 1380
   agggagtacc ggtacgccta cgcctgcggc gcccgccggc cgtgcaactt ccccaacacg 1440
   ctcaccaagg tcgacctggt ggagaggacg gccaagaact ggcacgagga gggctccgtg 1500
   ccgtccgagc ccttcttcgt gccacgcccc ggcgccaccg aggaagacga cggcgtggcg 1560
   atatcgatgg tgagcgccaa ggacgggtcg ggctatgcgc tggtgctgga cggcaagacg 1620
   ttcgaggagg tcgcgcgggc caagttcccg tacgggctgc cctacggctt gcactgctgc 1680
   tgggtgccca ggaaaaggaa cagcaagtaa 1710
<210> 46
   <211> 569
   <212> PRT
   <213> Oryza sativa
<400> 46
   Met Ser Pro Ala Met Leu Gln Ala Ser Ser Leu Cys Val Ser Ala Ala
   1               5                   10                  15
   Leu Ser Gly Ala Ala Ser Arg Pro Gly Arg Leu Ala Ser Gln Gly His
               20                  25                  30
   Gln Gly Lys Arg Ala Val Ala Gln Pro Leu Ala Ala Ser Ala Val Thr
           35                  40                  45
   Glu Ala Ala Pro Pro Ala Pro Val Val Ala Pro Pro Ala Arg Pro Val
       50                  55                  60
   Asp Ala Pro Arg Arg Arg Gly Gly Arg Gly Gly Gly Gly Gly Gly Gly
   65                  70                  75                  80
   Glu Leu Val Ala Trp Lys Ser Val Arg Gln Glu Arg Trp Glu Gly Ala
                   85                  90                  95
   Leu Glu Val Asp Gly Glu Leu Pro Leu Trp Leu Asp Gly Thr Tyr Leu
               100                 105                 110
   Arg Asn Gly Pro Gly Leu Trp Asn Leu Gly Asp Tyr Gly Phe Arg His
           115                 120                 125
   Leu Phe Asp Gly Tyr Ala Thr Leu Val Arg Val Ser Phe Arg Gly Gly
       130                 135                 140
   Arg Ala Val Gly Ala His Arg Gln Ile Glu Ser Glu Ala Tyr Lys Ala
   145                 150                 155                 160
   Ala Arg Ala His Gly Lys Val Cys Tyr Arg Glu Phe Ser Glu Val Pro
                   165                 170                 175
   Lys Pro Asp Asn Phe Leu Ser Tyr Val Gly Gln Leu Ala Thr Leu Phe
               180                 185                 190
   Ser Gly Ser Ser Leu Thr Asp Asn Ser Asn Thr Gly Val Val Met Leu
           195                 200                 205
   Gly Asp Gly Arg Val Leu Cys Leu Thr Glu Thr Ile Lys Gly Ser Ile
       210                 215                 220
   Gln Val Asp Pro Asp Thr Leu Asp Thr Val Gly Lys Phe Gln Tyr Thr
   225                 230                 235                 240
   Asp Lys Leu Gly Gly Leu Ile His Ser Ala His Pro Ile Val Thr Asp
                   245                 250                 255
   Thr Glu Phe Trp Thr Leu Ile Pro Asp Leu Ile Arg Pro Gly Tyr Val
               260                 265                 270
   Val Ala Arg Met Asp Ala Gly Ser Asn Glu Arg Gln Phe Val Gly Arg
           275                 280                 285
   Val Asp Cys Arg Gly Gly Pro Ala Pro Gly Trp Val His Ser Phe Pro
   290 295 300
   Val Thr Glu His Tyr Val Val Val Pro Glu Met Pro Leu Arg Tyr Cys
   305 310 315 320
   Ala Lys Asn Leu Leu Arg Ala Glu Pro Thr Pro Leu Tyr Lys Phe Glu
   325 330 335
   Trp His Leu Glu Ser Gly Ser Tyr Met His Val Met Cys Lys Ala Ser
   340 345 350
   Gly Lys Ile Val Ala Ser Val Glu Val Pro Pro Phe Val Thr Phe His
   355 360 365
   Phe Ile Asn Ala Tyr Glu Glu Thr Asp Glu Glu Gly Arg Val Thr Ala
   370 375 380
   Ile Ile Ala Asp Cys Cys Glu His Asn Ala Asn Thr Ala Ile Leu Asp
   385 390 395 400
   Lys Leu Arg Leu His Asn Leu Arg Ser Ser Ser Gly Gln Asp Val Leu
   405 410 415
   Pro Asp Ala Arg Val Gly Arg Phe Arg Ile Pro Leu Asp Gly Ser Gln
   420 425 430
   Phe Gly Glu Leu Glu Thr Ala Leu Asp Pro Glu Glu His Gly Arg Gly
   435 440 445
   Met Asp Met Cys Ser Ile Asn Pro Ala His Val Gly Arg Glu Tyr Arg
   450 455 460
   Tyr Ala Tyr Ala Cys Gly Ala Arg Arg Pro Cys Asn Phe Pro Asn Thr
   465 470 475 480
   Leu Thr Lys Val Asp Leu Val Glu Arg Thr Ala Lys Asn Trp His Glu
   485 490 495
   Glu Gly Ser Val Pro Ser Glu Pro Phe Phe Val Pro Arg Pro Gly Ala
   500 505 510
   Thr Glu Glu Asp Asp Gly Val Ala Ile Ser Met Val Ser Ala Lys Asp
   515 520 525
   Gly Ser Gly Tyr Ala Leu Val Leu Asp Gly Lys Thr Phe Glu Glu Val
   530 535 540
   Ala Arg Ala Lys Phe Pro Tyr Gly Leu Pro Tyr Gly Leu His Cys Cys
   545 550 555 560
   Trp Val Pro Arg Lys Arg Asn Ser Lys
   565
<210> 47
   <211> 1830
   <212> DNA
   <213> Oryza sativa
<400> 47
   atggcaacac aagcgattgc accgatgcac gccgccgtcg tgcaccgcca ccacgttcta      60
   ccaccccgcc gctgcgtgcg ccgccgtggc gtcttcgtcc gcgcctcggc cgccgccgcc     120
   gccgccgccg ccgagacgga cacgctgtcc gcggccttct gggactacaa cctcctcttc     180
   cggtcgcagc gcgacgagtg cctcgactcc atcccgctcc gcgtcaccga gggcgcgatc     240
   ccgcccgact tcccggccgg cacctactac ctcgccgggc cgggcatctt ctccgacgac     300
   cacggctcca ccgtccaccc cctcgacggc cacggctacc tccgctcctt ccgcttccgg     360
   cccggcgacc gcaccatcca ctactccgcg cggttcgtgg agacggcggc gaagagggag     420
   gagagccggg acggcgcgtc gtggcggttc acgcaccggg ggcccttctc cgtgctgcag     480
   ggcgggaaga aggtgggcaa tgtgaaggtg atgaagaacg tggccaacac cagcgtgctg     540
   cggtggggcg gccggctgct ctgcctctgg gagggcggcc agccgtacga ggttgacccc     600
   cggacgctcg agaccgtcgg cccgttcgac ctgctcggcc tcgccgccgc cgacgacaac     660
   aaggcaacga acgcgtctgc agcacgacgg ccgtggctgc aggaggccgg cctcgacgcc     720
   gccgcgcgcc tgctgcgccc tgttcttagc ggggtgttcg acatgccggg caagaggctg     780
   ctggcgcact acaagatcga cccgcggcgg gggcgtctgc tgatggtcgc ctgcaacgcc     840
   gaggacatgc tcctcccgcg atcccacttc actttctacg agttcgacgc ccacttcgac     900
   ctcgtccaga agcgtgagtt cgtcgtgccg gaccacctca tgatccacga ctgggccttc     960
   accgacaccc actacatcct cctcggcaac aggatcaagc tcgacatccc cggatcgctg    1020
   ctggcattga cgggcactca cccgatgatc gcggcgctgg ccgtggaccc gagaaggcag    1080
   tcgacgccgg tgtacctgct tccgcgctcc ccggagaccg aggcgggcgg ccgcgactgg    1140
   agcgtgccga tcgaggcgcc gtcgcagatg tggtccgtgc acgtcggcaa cgcgttcgag    1200
   gaggcgaacc gccggggcgg cctcgacgtc cggctgcaca tgtcaagctg ctcctaccag    1260
   tggttccatt tccacaggat gtttggttac aattggcacc acaagaagct ggacccgtcg    1320
   ttcatgaacg cggcgaaggg aaaggagtgg ctgcctcgcc tcgttcaggt ggccatcgag    1380
   ctcgacagga cgggagagtg ccggaggtgc tcagtcagga ggctgtccga tcagcacgcc    1440
   aggccggcgg acttcccggc gataaaccca agctacgcca accagaggaa ccggttcgtc    1500
   tacgccggcg ccgcgtccgg ctcccgcaga ttcctcccgt acttcccgtt cgacagcgtg    1560
   gtgaaggtcg acgtctccga tggatcggcg cggtggtggt ctaccgacgg gcgcaagttc    1620
   gtcggcgagc cggtcttcgt cccgaccggc ggcggagagg atggtggcta tgttcttctt    1680
   gtagagtatg cagtctccaa gcacagatgc catctagtgg tgctggatgc aaagaagata    1740
   gggacagaga atgcacttgt ggcaaaacta gaggtgccaa agaacctcac ttttccaatg    1800
   ggattccatg gtttctgggg agatgaatga                                      1830
<210> 48
   <211> 609
   <212> PRT
   <213> Oryza sativa
<400> 48
   Met Ala Thr Gln Ala Ile Ala Pro Met His Ala Ala Val Val His Arg
   1 5 10 15
   His His Val Leu Pro Pro Arg Arg Cys Val Arg Arg Arg Gly Val Phe
   20 25 30
   Val Arg Ala Ser Ala Ala Ala Ala Ala Ala Ala Ala Glu Thr Asp Thr
   35 40 45
   Leu Ser Ala Ala Phe Trp Asp Tyr Asn Leu Leu Phe Arg Ser Gln Arg
   50 55 60
   Asp Glu Cys Leu Asp Ser Ile Pro Leu Arg Val Thr Glu Gly Ala Ile
   65 70 75 80
   Pro Pro Asp Phe Pro Ala Gly Thr Tyr Tyr Leu Ala Gly Pro Gly Ile
   85 90 95
   Phe Ser Asp Asp His Gly Ser Thr Val His Pro Leu Asp Gly His Gly
   100 105 110
   Tyr Leu Arg Ser Phe Arg Phe Arg Pro Gly Asp Arg Thr Ile His Tyr
   115 120 125
   Ser Ala Arg Phe Val Glu Thr Ala Ala Lys Arg Glu Glu Ser Arg Asp
   130 135 140
   Gly Ala Ser Trp Arg Phe Thr His Arg Gly Pro Phe Ser Val Leu Gln
   145 150 155 160
   Gly Gly Lys Lys Val Gly Asn Val Lys Val Met Lys Asn Val Ala Asn
   165 170 175
   Thr Ser Val Leu Arg Trp Gly Gly Arg Leu Leu Cys Leu Trp Glu Gly
   180 185 190
   Gly Gln Pro Tyr Glu Val Asp Pro Arg Thr Leu Glu Thr Val Gly Pro
   195 200 205
   Phe Asp Leu Leu Gly Leu Ala Ala Ala Asp Asp Asn Lys Ala Thr Asn
   210 215 220
   Ala Ser Ala Ala Arg Arg Pro Trp Leu Gln Glu Ala Gly Leu Asp Ala
   225 230 235 240
   Ala Ala Arg Leu Leu Arg Pro Val Leu Ser Gly Val Phe Asp Met Pro
   245 250 255
   Gly Lys Arg Leu Leu Ala His Tyr Lys Ile Asp Pro Arg Arg Gly Arg
   260 265 270
   Leu Leu Met Val Ala Cys Asn Ala Glu Asp Met Leu Leu Pro Arg Ser
   275 280 285
   His Phe Thr Phe Tyr Glu Phe Asp Ala His Phe Asp Leu Val Gln Lys
   290 295 300
   Arg Glu Phe Val Val Pro Asp His Leu Met Ile His Asp Trp Ala Phe
   305 310 315 320
   Thr Asp Thr His Tyr Ile Leu Leu Gly Asn Arg Ile Lys Leu Asp Ile
   325 330 335
   Pro Gly Ser Leu Leu Ala Leu Thr Gly Thr His Pro Met Ile Ala Ala
   340 345 350
   Leu Ala Val Asp Pro Arg Arg Gln Ser Thr Pro Val Tyr Leu Leu Pro
   355 360 365
   Arg Ser Pro Glu Thr Glu Ala Gly Gly Arg Asp Trp Ser Val Pro Ile
   370 375 380
   Glu Ala Pro Ser Gln Met Trp Ser Val His Val Gly Asn Ala Phe Glu
   385 390 395 400
   Glu Ala Asn Arg Arg Gly Gly Leu Asp Val Arg Leu His Met Ser Ser
   405 410 415
   Cys Ser Tyr Gln Trp Phe His Phe His Arg Met Phe Gly Tyr Asn Trp
   420 425 430
   His His Lys Lys Leu Asp Pro Ser Phe Met Asn Ala Ala Lys Gly Lys
   435 440 445
   Glu Trp Leu Pro Arg Leu Val Gln Val Ala Ile Glu Leu Asp Arg Thr
   450 455 460
   Gly Glu Cys Arg Arg Cys Ser Val Arg Arg Leu Ser Asp Gln His Ala
   465 470 475 480
   Arg Pro Ala Asp Phe Pro Ala Ile Asn Pro Ser Tyr Ala Asn Gln Arg
   485 490 495
   Asn Arg Phe Val Tyr Ala Gly Ala Ala Ser Gly Ser Arg Arg Phe Leu
   500 505 510
   Pro Tyr Phe Pro Phe Asp Ser Val Val Lys Val Asp Val Ser Asp Gly
   515 520 525
   Ser Ala Arg Trp Trp Ser Thr Asp Gly Arg Lys Phe Val Gly Glu Pro
   530 535 540
   Val Phe Val Pro Thr Gly Gly Gly Glu Asp Gly Gly Tyr Val Leu Leu
   545 550 555 560
   Val Glu Tyr Ala Val Ser Lys His Arg Cys His Leu Val Val Leu Asp
   565 570 575
   Ala Lys Lys Ile Gly Thr Glu Asn Ala Leu Val Ala Lys Leu Glu Val
   580 585 590
   Pro Lys Asn Leu Thr Phe Pro Met Gly Phe His Gly Phe Trp Gly Asp
   595 600 605
   Glu
<210> 49
   <211> 1644
   <212> DNA
   <213> Oryza sativa
<400> 49
   atgggaggcg gcgatggcga tgaggtgctg ctgctgccgg agccgcgccc tcgcaggggc      60
   ctcgcctcct gggcgctcga tctgctggag cgcgccgccg tccgcctcgg ccacgacgcc     120
   tccaagccgc tctactggct ctccggcaac ttcgcccccg tccaccacga gaccccgccg     180
   gccccggccc tccccgtccg cggccacctc cccgagtgct tgaatggaga atttgtcagg     240
   gtaggaccca atccaaagtt tgtccctgtc gctggctatc attggtttga tggagatgga     300
   atgatccatg cgatgcgtat caaagatggg aaagctacct atgtgtcgag atatgtgaag     360
   acttctcgtc tcaagcaaga agagtatttt ggtggagcaa agtttatgaa gattggagac     420
   ctgaagggat tttatggatt gtttatggtc caaatgcaac aactccggaa aaaactcaaa     480
   gtattggatt ttacatatgg acatgggaca gctaatactg cacttatcta tcaccatggt     540
   aaacttatgg ccctgtcaga ggcagataag ccctgtaagt tctcatccca taatgatgtt     600
   gttaaggtcc ttgaagatgg agacttgcaa actcttggat tgttggatta cgacaaacgg     660
   ttgaaacact ctttcactgc tcatccaaag gtcgatccat ttacagacga aatgttcgct     720
   tttggatatt cgcatgaacc tccttactgt acataccggg tcattaccaa ggatggagcc     780
   atgcttgatc ctgtgccaat aacaattcca gaatctgtaa tgatgcacga ctttgccatt     840
   acagagaatt attctatttt catggacctt cctctgttgt tccgaccaaa ggaaatggtg     900
   aagaatggtg agtttatcta caagtttgat cctacaaaga aagctcgttt tggtatactc     960
   caacgttatg aaaaggatga cacaaacatc agatggtttg aacttcccaa ctgcttcata    1020
   ttccacaatg ctaatgcttg ggaagagggt gacgaagtta tcctaattac ctgccgcttg    1080
   gagaatcctg acttggacaa ggtgaacggt taccaaagcg acaatctcga gaactttggg    1140
   aatgagctgt atgagatgag attcaacatg aaaaccggtg ctgcttcaca aaagcaacta    1200
   tctgtttctg ctgtagattt tcctcgaatt aatgagagct acactggcag aaagcagcgg    1260
   tatgtgtatt gcgctattct aaacagcata gcgaaggtag caggcattat aaaatttgat    1320
   ctacatgctg aaccggagat cagtggcttt aaacaacttg aagtgggggg aaatgtgaga    1380
   ggaatatttg acttgggacc tggtagattc gggtccgagg caatttttgt gcctagggaa    1440
   cccggtgtat ctggagaaga agatgatggt tatttgatat tctttgtcca cgacgagaat    1500
   acagggaaat ctgaagtcaa tgtgattgat gcaaagacaa tgtctgctga tccggtggca    1560
   gttgttgagc taccaagccg agttccctac ggattccatg ctttctttat aaacgaggaa    1620
   caactggcaa aacaatcagc gtga                                           1644
<210> 50
   <211> 547
   <212> PRT
   <213> Oryza sativa
<400> 50
   Met Gly Gly Gly Asp Gly Asp Glu Val Leu Leu Leu Pro Glu Pro Arg
   1 5 10 15
   Pro Arg Arg Gly Leu Ala Ser Trp Ala Leu Asp Leu Leu Glu Arg Ala
   20 25 30
   Ala Val Arg Leu Gly His Asp Ala Ser Lys Pro Leu Tyr Trp Leu Ser
   35 40 45
   Gly Asn Phe Ala Pro Val His His Glu Thr Pro Pro Ala Pro Ala Leu
   50 55 60
   Pro Val Arg Gly His Leu Pro Glu Cys Leu Asn Gly Glu Phe Val Arg
   65 70 75 80
   Val Gly Pro Asn Pro Lys Phe Val Pro Val Ala Gly Tyr His Trp Phe
   85 90 95
   Asp Gly Asp Gly Met Ile His Ala Met Arg Ile Lys Asp Gly Lys Ala
   100 105 110
   Thr Tyr Val Ser Arg Tyr Val Lys Thr Ser Arg Leu Lys Gln Glu Glu
   115 120 125
   Tyr Phe Gly Gly Ala Lys Phe Met Lys Ile Gly Asp Leu Lys Gly Phe
   130 135 140
   Tyr Gly Leu Phe Met Val Gln Met Gln Gln Leu Arg Lys Lys Leu Lys
   145 150 155 160
   Val Leu Asp Phe Thr Tyr Gly His Gly Thr Ala Asn Thr Ala Leu Ile
   165 170 175
   Tyr His His Gly Lys Leu Met Ala Leu Ser Glu Ala Asp Lys Pro Cys
   180 185 190
   Lys Phe Ser Ser His Asn Asp Val Val Lys Val Leu Glu Asp Gly Asp
   195 200 205
   Leu Gln Thr Leu Gly Leu Leu Asp Tyr Asp Lys Arg Leu Lys His Ser
   210 215 220
   Phe Thr Ala His Pro Lys Val Asp Pro Phe Thr Asp Glu Met Phe Ala
   225 230 235 240
   Phe Gly Tyr Ser His Glu Pro Pro Tyr Cys Thr Tyr Arg Val Ile Thr
   245 250 255
   Lys Asp Gly Ala Met Leu Asp Pro Val Pro Ile Thr Ile Pro Glu Ser
   260 265 270
   Val Met Met His Asp Phe Ala Ile Thr Glu Asn Tyr Ser Ile Phe Met
   275 280 285
   Asp Leu Pro Leu Leu Phe Arg Pro Lys Glu Met Val Lys Asn Gly Glu
   290 295 300
   Phe Ile Tyr Lys Phe Asp Pro Thr Lys Lys Ala Arg Phe Gly Ile Leu
   305 310 315 320
   Gln Arg Tyr Glu Lys Asp Asp Thr Asn Ile Arg Trp Phe Glu Leu Pro
   325 330 335
   Asn Cys Phe Ile Phe His Asn Ala Asn Ala Trp Glu Glu Gly Asp Glu
   340 345 350
   Val Ile Leu Ile Thr Cys Arg Leu Glu Asn Pro Asp Leu Asp Lys Val
   355 360 365
   Asn Gly Tyr Gln Ser Asp Asn Leu Glu Asn Phe Gly Asn Glu Leu Tyr
   370 375 380
   Glu Met Arg Phe Asn Met Lys Thr Gly Ala Ala Ser Gln Lys Gln Leu
   385 390 395 400
   Ser Val Ser Ala Val Asp Phe Pro Arg Ile Asn Glu Ser Tyr Thr Gly
   405 410 415
   Arg Lys Gln Arg Tyr Val Tyr Cys Ala Ile Leu Asn Ser Ile Ala Lys
   420 425 430
   Val Ala Gly Ile Ile Lys Phe Asp Leu His Ala Glu Pro Glu Ile Ser
   435 440 445
   Gly Phe Lys Gln Leu Glu Val Gly Gly Asn Val Arg Gly Ile Phe Asp
       450                 455                 460
   Leu Gly Pro Gly Arg Phe Gly Ser Glu Ala Ile Phe Val Pro Arg Glu
   465                 470                 475                 480
   Pro Gly Val Ser Gly Glu Glu Asp Asp Gly Tyr Leu Ile Phe Phe Val
                   485                 490                 495
   His Asp Glu Asn Thr Gly Lys Ser Glu Val Asn Val Ile Asp Ala Lys
               500                 505                 510
   Thr Met Ser Ala Asp Pro Val Ala Val Val Glu Leu Pro Ser Arg Val
           515                 520                 525
   Pro Tyr Gly Phe His Ala Phe Phe Ile Asn Glu Glu Gln Leu Ala Lys
       530                 535                 540
   Gln Ser Ala
   545